(19) Europäisches Patentamt / European Patent Office / Office européen des brevets

(11) EP 2 324 015 B1

(12) **FASCICULE DE BREVET EUROPEEN**

(45) Date de publication et mention de la délivrance du brevet:
**03.07.2013 Bulletin 2013/27**

(21) Numéro de dépôt: **09740422.2**

(22) Date de dépôt: **30.07.2009**

(51) Int Cl.:
*C07D 409/06* (2006.01) *C07D 409/14* (2006.01)
*A61K 31/445* (2006.01) *A61P 29/00* (2006.01)
*A61P 25/00* (2006.01) *A61P 3/00* (2006.01)
*C07D 417/06* (2006.01) *C07D 417/14* (2006.01)
*C07D 333/38* (2006.01) *C07D 211/64* (2006.01)
*C07D 205/04* (2006.01) *C07D 211/38* (2006.01)
*C07D 211/62* (2006.01) *A61K 31/381* (2006.01)
*A61K 31/397* (2006.01) *A61K 31/4535* (2006.01)
*A61K 31/4545* (2006.01) *A61P 1/00* (2006.01)
*A61P 19/00* (2006.01)

(86) Numéro de dépôt international:
**PCT/FR2009/051540**

(87) Numéro de publication internationale:
**WO 2010/012964 (04.02.2010 Gazette 2010/05)**

(54) **DERIVES DE THIOPHENE-2-CARBOXAMIDE, LEUR PREPARATION ET LEUR APPLICATION EN THERAPEUTIQUE**

THIOPHEN-2-CARBONSÄUREAMIDDERIVATE, DEREN HERSTELLUNG UND DEREN THERAPEUTISCHE VERWENDUNG

THIOPHENE-2-CARBOXAMIDE DERIVATIVES, PREPARATION THEREOF AND THERAPEUTIC USE THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **01.08.2008 FR 0804384**

(43) Date de publication de la demande:
**25.05.2011 Bulletin 2011/21**

(73) Titulaire: **SANOFI**
**75008 Paris (FR)**

(72) Inventeurs:
- **BARTH, Francis**
  **F-75013 Paris (FR)**
- **DUCOUX, Jean-Philippe**
  **F-75013 Paris (FR)**
- **GUEULE, Patrick**
  **F-75013 Paris (FR)**
- **RINALDI-CARMONA, Murielle**
  **F-75013 Paris (FR)**
- **ROUQUETTE, Arnaud**
  **F-75013 Paris (FR)**

(74) Mandataire: **Werner, Alain Henri et al**
**Sanofi**
**Département Brevets**
**54, rue La Boétie**
**75008 Paris (FR)**

(56) Documents cités:
**FR-A- 2 860 792**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 324 015 B1

## Description

La présente invention a pour objet des dérivés de 4,5-diarylthiophène-2-carboxamide, leur préparation et leur application en thérapeutique.

Des dérivés de diphénylpyrazole, présentant une affinité pour les récepteurs $CB_1$ des cannabinoïdes, ont été décrits notamment dans les brevets US 5 624 941, EP 0 576 357, EP 0 656 354 et EP 1 150 961.

Des dérivés de 5,6-diphényl-2-pyrazine-carboxamide sont décrits dans la demande internationale WO 03/051 850 comme des antagonistes des récepteurs $CB_1$.

Des dérivés de 1,2-diphényl-4-imidazole-carboxamide sont décrits dans la demande internationale WO 03/027 076 comme des agonistes des récepteurs $CB_1$, des agonistes partiels ou des antagonistes.

Des dérivés de 4,5-diarylthiophène ayant des propriétés analgésiques sont décrits dans la demande internationale WO 91/19 708.

D'autres dérivés de 4,5-diarylthiophène sont décrits dans la demande internationale WO 2005/035 48 ainsi que dans la demande française FR 2860792 comme des antagonistes des récepteurs $CB_1$.

On a maintenant trouvé des nouveaux dérivés de 4,5-diarylthiophène-2-carboxamide portant un substituant particulier sur l'un des groupes aryle qui possèdent des propriétés antagonistes des récepteurs $CB_1$ des cannabinoïdes au niveau central et au niveau périphérique. En particulier, ces nouveaux dérivés ont des propriétés antagonistes des récepteurs CB1 périphériques et présentent une faible pénétration au niveau du cerveau.

Ainsi la présente invention a pour objet des composés répondant à la formule (I) :

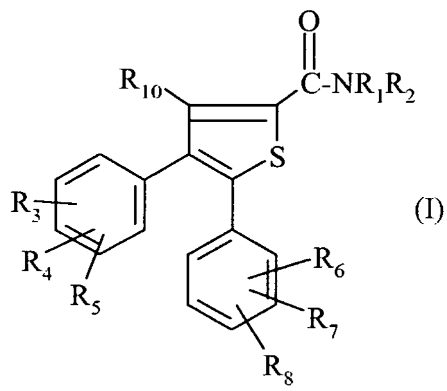

(I)

dans laquelle :

- $R_1$ et $R_2$ ensemble avec l'atome d'azote auquel ils sont liés constituent :

  o soit un radical hétérocyclique saturé de 5 à 7 atomes comprenant deux atomes d'azote, non substitué ou substitué par un groupe phényle, benzyle, benzodioxolyle, benzodioxolylméthyle, tétrahydrofuranylcarbonyle, $-COR_{11}$, et/ou $-CH_2COR_{11}$, le groupe phényle étant lui-même non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  o soit un radical hétérocyclique saturé de 4 à 7 atomes comprenant un atome d'azote, non substitué ou substitué une ou deux fois par un substituant choisi chacun indépendamment parmi :

  • un groupe cyano, $-COR_{11}$, $-CH_2NHR_{12}$, $-(C_3-C_7)$cycloalkyle, $-CH_2COR_{11}$, $-NR_{12}R_{13}$, $-NHCOR_{14}$, $-SO_2R_{14}$, et/ou $-SO_2NR_{12}R_{13}$ ;
  • et/ou un groupe phényle, benzyle, pyridinyle ; lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  • et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou $OCF_3$ ;
  • et/ou un groupe, phénylamino, benzylamino, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  • et/ou un groupe amino$(C_1-C_6)$alkyle non substitué ou substitué une ou plusieurs fois par un substituant

choisi chacun indépendamment parmi un atome d'halogène, un groupe hydroxyle, $(C_1-C_4)$alcoxy, $(C_3-C_7)$ cycloalkyle et/ou cyano ;

- et/ou un groupe amino$(C_3-C_7)$cycloalkyle non substitué ou substitué une ou plusieurs fois par un substituant choisi chacun indépendamment parmi un atome d'halogène, un groupe hydroxyle, $(C_1-C_4)$alkyle,$(C_1-C_4)$ alcoxy et/ou cyano, ledit groupe $(C_1-C_4)$alkyle étant non substitué ou substitué une ou plusieurs fois par un atome de fluor ;

- $R_3$, $R_4$, $R_5$, $R_6$, $R_7$ et $R_8$ représentent chacun indépendamment un atome d'hydrogène, un atome d'halogène, un groupement -CN, $-S(O)_nR_{14}$, $-OSO_2R_{14}$, un groupe $(C_1-C_6)$alkyle et/ou un groupe $(C_1-C_6)$alcoxy, lesdits groupes étant non substitués ou substitués une ou plusieurs fois par un atome de fluor, à la condition que l'un des deux substituants $R_3$, $R_6$ représente un groupe $Y-A-R_9$ ;
- Y représente un atome d'oxygène, un groupe $-S(O)_{n'}-$, ou $-OSO_2$ ;
- A représente un groupe alkylène en $(C_1-C_4)$ non substitué ou substitué une ou plusieurs fois par un groupe $(C_1-C_3)$ alkyle et/ou par un atome de fluor ;
- $R_9$ représente un groupe $-OR_{19}$, -CN, $-CH_3$, $-CF_3$, $-NR_{19}R_{20}$, $-CO_2R_{19}$, - $CONR_{19}R_{20}$, $-NR_{15}COR_{19}$, $-CONHNH_2$, -CONHOH, $-CONHSO_2R_{21}$ $-S(O)_nR_{21}$, $-SO_2NR_{19}R_{20}$, $-NR_{18}SO_2R_{21}$, $-NR_{15}SO_2NR_{19}R_{20}$;
- $R_{10}$ représente un atome d'hydrogène ou un groupe $(C_1-C_4)$alkyle, et de préférence un atome d'hydrogène ;
- $R_{11}$ représente :

  o un groupe $(C_1-C_4)$alkyle, phényle, benzyle, $(C_1-C_4)$alcoxy, ou $(C_1-C_3)$alkylène-O-$(C_1-C_3)$alkyle, lesdits groupes étant non substitués ou substitués par un substituant choisi chacun indépendamment parmi un groupe $(C_1-C_4)$alcoxy et/ou un groupe hydroxy et/ou par un ou plusieurs atomes de fluor ;
  o et/ou un groupement $-NR_{16}R_{17}$ ;

- $R_{12}$ et $R_{13}$ représentent chacun indépendamment un atome d'hydrogène ou un groupe $(C_1-C_6)$alkyle éventuellement substitué une ou plusieurs fois par un substituant choisi chacun indépendamment parmi un atome de fluor, un groupe -OH et/ou un groupe $-OR_{14}$ ;
- ou $R_{12}$ et $R_{13}$ ensemble avec l'atome d'azote auquel ils sont liés constituent un radical hétérocyclique de 4 à 7 chaînons pouvant comporter un second hétéroatome choisi parmi un atome d'azote, d'oxygène ou de soufre ;
- n représente 0, 1 ou 2 ;
- n' représente 0, 1 ou 2 ;
- $R_{14}$ représente un groupe $(C_1-C_4)$alkyle non substitué ou substitué une ou plusieurs fois par un atome de fluor ;
- $R_{15}$ représente un atome d'hydrogène ou un groupe $(C_1-C_4)$alkyle ;
- $R_{16}$ et $R_{17}$ représentent chacun indépendamment :

  o un atome d'hydrogène ;
  o et/ou un groupe benzyle non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$ alcoxy et/ou cyano ;
  o et/ou un groupe $(C_1-C_6)$alkyle éventuellement substitué une ou plusieurs fois par un substituant choisi chacun indépendamment parmi un atome d'halogène, un groupe -OH et/ou $-OR_{14}$ ;

- $R_{18}$ représente un atome d'hydrogène ou un groupe $(C_1-C_4)$alkyle non substitué ou substitué une ou plusieurs fois par un atome de fluor ;
- $R_{19}$ et $R_{20}$ représentent chacun indépendamment un atome d'hydrogène ou un groupe $(C_1-C_6)$alkyle éventuellement substitué une ou plusieurs fois par un substituant choisi chacun indépendamment parmi un atome de fluor, un groupe -OH et/ou un groupe $-OR_{14}$ ;
- ou $R_{19}$ et $R_{20}$ ensemble avec l'atome d'azote auquel ils sont liés constituent un radical hétérocyclique de 4 à 7 chaînons pouvant comporter un second hétéroatome choisi parmi un atome d'azote, d'oxygène ou de soufre ;
- $R_{21}$ représente un groupe $(C_1-C_4)$alkyle non substitué ou substitué une ou plusieurs fois par un atome de fluor ;

à l'état de bases (= correspondent aux formes libres des composés) ainsi que leurs sels acceptables pharmaceutiquement ou acceptables pour la purification et/ou l'isolement desdits composés de formule (I).

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques font partie de l'invention.

Les composés de formule (I) peuvent exister à l'état de bases (c'est-à-dire tels quels sous leurs formes libres), de sels d'addition à des acides ou de sels d'addition à des bases. Ces sels sont avantageusement préparés avec des

sels pharmaceutiquement acceptables ; les sels d'autres acides utiles, par exemple, pour la purification ou l'isolement des composés de formule (I) font également partie de l'invention.

Par groupe alkyle, on entend un radical carboné linéaire ou ramifié, tel qu'en particulier : méthyle, éthyle, propyle, isopropyle, butyle, isobutyle, *tert*-butyle, n-pentyle, isopentyle, n-hexyle, isohexyle. Le groupe méthyle est préféré pour un ($C_1$-$C_4$)alkyle et pour un ($C_1$-$C_6$)alkyle.

Par groupe aminoalkyle, on entend un groupe amino lié à un radical carboné linéaire ou ramifié, tel que par exemple : méthyle, éthyle, propyle, isopropyle, butyle, isobutyle, *tert*-butyle, n-pentyle, isopentyle, n-hexyle, isohexyle, heptyle, isoheptyle. Par groupe alkylène, on entend un radical carboné bivalent linéaire tel que $-(CH_2)-$, $-(CH_2)_2-$, $-(CH_2)_{3-}$, $-(CH_2)_4-$, $-(CH_2)_5-$.

Par ($C_1$-$C_4$)alcoxy et ($C_1$-$C_6$)alcoxy , on entend respectivement un atome d'oxygène lié à un radical carboné linéaire ou ramifié de un à quatre atomes de carbones et de un à six atomes de carbones, tel que le radical méthoxy, éthoxy, propoxy, isopropoxy, butoxy, sec-butoxy, *tert*-butoxy, pentoxy, hexyloxy, le groupe méthoxy étant préféré.

Par atome d'halogène, on entend un atome de fluor, de chlore, de brome ou d'iode, les atomes de fluor, chlore ou brome étant préférés.

Par groupe cycloalkyle, on entend un radical carboné cyclopropyle, cyclobutyle, cyclopentyle, cyclohexyle, cycloheptyle.

Par groupe aminocycloalkyle, on entend un groupe amino lié à un radical carboné cyclique, tel que par exemple : cyclopropyle, cyclobutyle, cyclopentyle, cyclohexyle, cycloheptyle.

Par radical hétérocyclique, saturé de 4 à 7 chaînons, contenant 1 ou 2 atomes d'azote, on entend notamment des radicaux tels que azétidin-1-yle, pyrrolidin-1-yle, pipéridin-1-yle, homopipéridin-1-yle, imidazolin-1-yle, pipérazin-1-yle et 1,4-diazépan-1-yle

Selon la présente invention, on distingue :

- les composés de formule (IA) dans laquelle Y représente un atome d'oxygène ;
- les composés de formule (IB) dans laquelle Y représente un groupe -S(O)n'- ;
- les composés de formule (IC) dans laquelle Y représente un groupe $-O(SO_2)-$;
- les autres substituants étant tels que définis pour les composés de formule (I).

Au sein des composés de formules (I), (IA), (IB) et (IC), on distingue en particulier :

- les composés dans lesquels le substituant $R_3$ représente un groupe Y-A-$R_9$ et le substituant $R_6$ représente un atome d'hydrogène, un atome d'halogène, un groupement -CN, $-S(O)_nR_{14}$, $-OSO_2R_{14}$, un groupe ($C_1$-$C_6$)alkyle ou un groupe ($C_1$-$C_6$) alcoxy, lesdits groupes étant non substitués ou substitués par un ou plusieurs atomes de fluor ;
- et les composés dans lesquels le substituant $R_6$ représente un groupe Y-A-$R_9$ et le substituant $R_3$ représente un atome d'hydrogène, un atome d'halogène, un groupement -CN, $-S(O)_nR_{14}$, $-OSO_2R_{14}$, un groupe ($C_1$-$C_6$)alkyle ou un groupe ($C_1$-$C_6$) alcoxy, lesdits groupes étant non substitués ou substitués par un ou plusieurs atomes de fluor ;
- les autres substituants étant tels que définis ci-avant pour les composés de formule (1).

De préférence pour les composés dans lesquels le substituant $R_3$ représente un groupe Y-A-$R_9$, $R_4$, $R_5$, $R_6$, $R_7$ et $R_8$ représentent chacun indépendamment un atome d'hydrogène, un atome d'halogène, un groupement -CN et/ou un groupe ($C_1$-$C_6$)alkyle, ledit groupe étant non substitué ou substitué une ou plusieurs fois par un atome de fluor.

De préférence pour les composés dans lesquels le substituant $R_6$ représente un groupe Y-A-$R_9$, $R_3$, $R_4$, $R_5$, $R_7$ et $R_8$ représentent chacun indépendamment un atome d'hydrogène, un atome d'halogène, un groupement -CN et/ou un groupe ($C_1$-$C_6$)alkyle, ledit groupe étant non substitué ou substitué une ou plusieurs fois par un atome de fluor.

Pour ces deux modes préférentiels, les autres substituants sont tels que définis ci-avant pour les composés de formule (I).

Pour les composés dans lesquels le substituant $R_3$ représente un groupe Y-A-$R_9$, le substituant $R_4$ et/ou le substituant $R_5$ correspondent de préférence à un atome d'hydrogène. Plus préférentiellement, $R_3$ est en position 4 sur le phényle. Pour cette variante des composés de formule (I) selon l'invention, on préfère de plus $R_6$ correspondant à un atome d'hydrogène ou d'halogène tel que le chlore ou le fluor, $R_7$ correspondant à un atome d'halogène, de préférence un atome de chlore et $R_8$ correspondant à un atome d'hydrogène.

Selon la présente invention, on préfère les composés de formule (I) dans lesquels :

- A représente un groupe alkylène en ($C_1$-$C_4$) non substitué ;
- $R_9$ représente un groupe $-OR_{19}$, $-CH_3$, $-CF_3$, $-NR_{19}R_{20}$, $-CONR_{19}R_{20}$, - $NR_{15}COR_{19}$, $-S(O)_nR_{21}$, ou $-NR_{18}SO_2R_{21}$ ;
- et les autres substituants étant tels que définis ci-avant pour les composés de formule (I).

En particulier pour Y, on préfère un atome d'oxygène ou un atome de soufre.

En particulier pour $R_9$, on préfère un groupe $-OR_{19}$, $-NR_{19}R_{20}$, $-CONR_{19}R_{20}$, $-S(O)nR_{21}$, ou $-NR_{18}SO_2R_{21}$.

Selon la présente invention, on préfère les composés de formule (I) dans lesquels :

- $R_1$ et $R_2$ ensemble avec l'atome d'azote auquel ils sont liés constituent un radical homopipéridin-1-yle, pipéridin-1-yle, pyrrolidin-1-yle ou azétidin-1-yle, lesdits radicaux étant substitués une ou deux fois par un substituant choisi chacun indépendamment parmi :

  o un groupe cyano, $-COR_{11}$, $-NR_{12}R_{13}$, $-NHCOR_{14}$, $-CH_2COR_{11}$, $-SO_2R_{14}$, et/ou $-SO_2NR_{12}R_{13}$;
  o et/ou un groupe phényle, benzyle, pyridinyle ; lesdits groupes étant non substitués ou substitués une ou plusieurs fois par un substituant choisi chacun indépendamment parmi un atome d'halogène, un groupe $(C_1-C_4)$ alkyle, trifluorométhyle, hydroxyle, $(C_1-C_4)$alcoxy et/ou cyano ;
  o et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle,$(C_1-C_4)$alcoxy, hydroxy, trifluorométhyle et/ou $OCF_3$ ;
  o et/ou un groupe phénylamino, benzylamino, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  o et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
  o et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$alkyle,$(C_1-C_4)$alcoxy et/ou cyano, ledit groupe $(C_1-C_4)$alkyle étant non substitué ou substitué une ou plusieurs fois par un atome de fluor ;

- les autres substituants étant tels que définis pour les composés de formule (I).

En particulier, on préfère les composés de formule (I) dans lesquels :

- $R_1$ et $R_2$ ensemble avec l'atome d'azote auquel ils sont liés constituent un radical homopipéridin-1-yle, pipéridin-1-yle, pyrrolidin-1-yle ou azétidin-1-yle, ledit radical étant gem-disubstitué :

  o le premier substituant dudit radical étant choisi parmi un groupe cyano, $-COR_{11}$, $-NHCOR_{14}$, ou $-SO_2R_{14}$ ;
  o le second substituant dudit radical étant choisi parmi :

    • $NR_{12}R_{13}$;
    • et/ou un groupe phényle, ledit groupe étant non substitué ou substitué une ou plusieurs fois par un substituant choisi chacun indépendamment parmi un atome d'halogène, un groupe $(C_1-C_4)$alkyle,trifluorométhyle, hydroxyle, $(C_1-C_4)$alcoxy et/ou cyano ;
    • et/ou un groupe pipéridin-1-yle, ledit groupe étant non substitué ou substitué une ou plusieurs fois par un substituant choisi chacun indépendamment parmi un atome de fluor, un groupe $(C_1-C_4)$alkyle,$(C_1-C_4)$alcoxy, hydroxy, trifluorométhyle et/ou $OCF_3$ ;
    • et/ou un groupe benzylamino, ledit groupe étant non substitué ou substitué une ou plusieurs fois par un substituant choisi chacun indépendamment parmi un atome d'halogène, un groupe $(C_1-C_4)$alkyle,trifluorométhyle, hydroxyle, $(C_1-C_4)$alcoxy et/ou cyano ;

- les autres substituants étant tels que définis pour les composés de formule (I).

Plus particulièrement, on préfère les composés de formule (I) dans lesquels $R_1$ et $R_2$ ensemble avec l'atome d'azote auquel ils sont liés constituent un radical pipéridin-1-yle ou azétidin-1-yle. Dans ce cas :

- le premier substituant du radical pipéridin-1-yle ou azétidin-1-yle est de préférence $-COR_{11}$ ;
- et le second substituant du radical pipéridin-1-yle ou azétidin-1-yle est de préférence choisi parmi $-NR_{12}R_{13}$, un groupe phényle, un groupe benzylamino ou un groupe pipéridin-1-yle, ledit groupe pipéridin-1-yle étant non substitué ou substitué une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou OCF3 ;
- les autres substituants étant tels que définis pour les composés de formule (I).

Parmi les composés selon l'invention, on peut notamment citer les composés ci-après, tels quels ainsi que

leurs sels :

| Nom IUPAC | Structure Chimique |
|---|---|
| 1-({4-(2,4-dichlorophényl)-5-[4-(3-hydroxypropoxy)phényl]-2-thiényl}carbonyl)-4-phénylpipéridine-4-carboxamide | 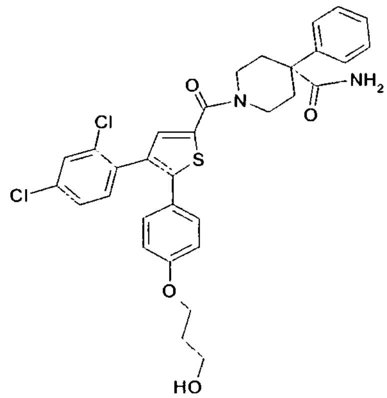 |
| 1-({5-[4-(3-aminopropoxy)phényl]-4-(2,4-dichlorophényl)-2-thiényl}carbonyl)-4-phénylpipéridine-4-carboxamide | 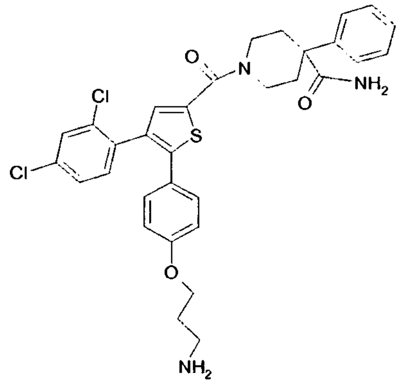 |
| 1- {[4-(2,4-dichlorophényl)-5-(4-{3-[(méthylsulfonyl)amino] propoxy}phényl)-2-thiényl]carbonyl}-4-phénylpipéridine-4-carboxamide | 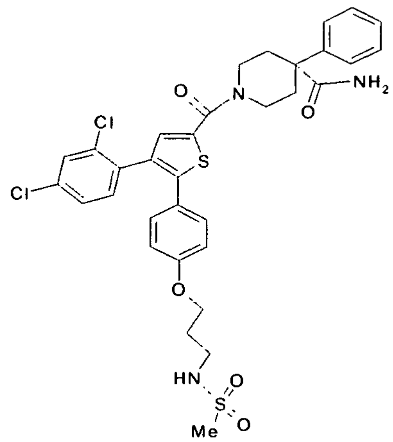 |
| 1- {[4-(2,4-dichlorophényl)-5-{4-[3-(méthylthio)propoxy] phényl}-2-thiényl]carbonyl}-4-phénylpipéridine-4-carboxamide | 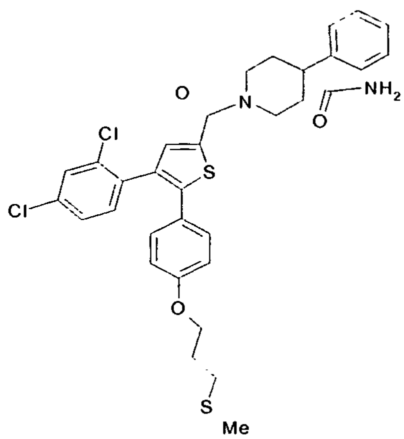 |

(suite)

| Nom IUPAC | Structure Chimique |
|---|---|
| 1-{[4-(2,4-dichlorophényl)-5-{4-[3-(méthylsulfonyl)propoxy] phényl}-2-thiényl]carbonyl}-4-phénylpipéridine-4-carboxamide | 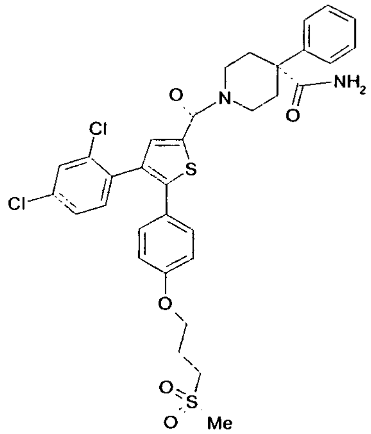 |
| 1-({4-(2,4-dichlorophényl)-5-[4-(2-hydroxyethoxy)phényl]-2-thiényl}carbonyl)-4-phénylpipéridine-4-carboxamide | 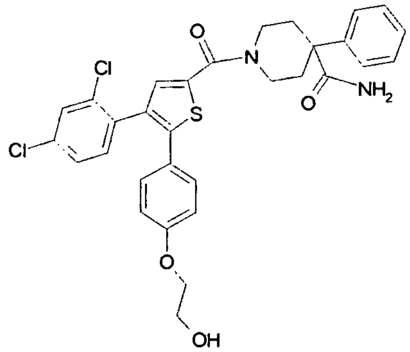 |
| 1'-({4-(2,4-dichlorophényl)-5-[4-(3-hydroxypropoxy)phényl]-2-thienyl}carbonyl)-4,4-difluoro-1,4'-bipipéridine-4'-carboxamide | 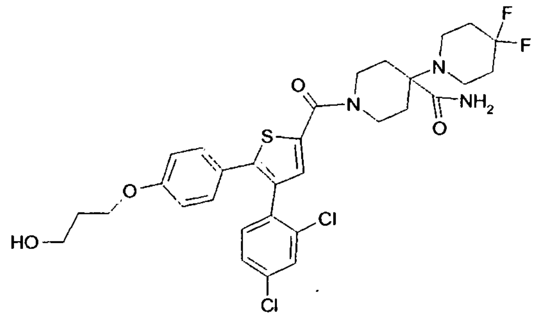 |
| 1-({5-(2,4-dichlorophényl)-4-[4-(3-hydroxypropoxy)phényl]-2-thiényl}carbonyl)-4-phénylpipéridine-4-carboxamide | 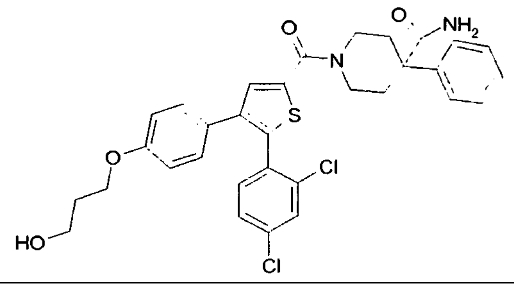 |
| 1-{[5-(2,4-dichlorophényl)-4-(4-{3-[(méthylsulfonyl)amino] propoxylphényl)-2-thiényl]carbonyl}-4-phénylpipéridine-4-carboxamide | 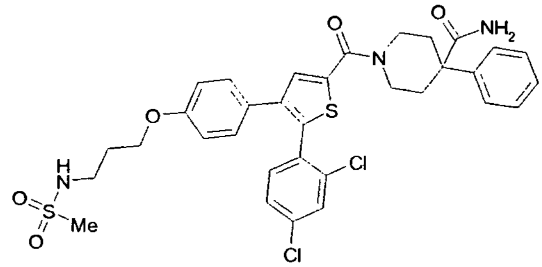 |

(suite)

| Nom IUPAC | Structure Chimique |
|---|---|
| 1-({5-(2,4-dichlorophényl)-4-[4-(3-pyrrolidin-1-ylpropoxy) phényl]-2-thiényl}carbonyl)-4-phénylpipéridine-4-carboxamide | 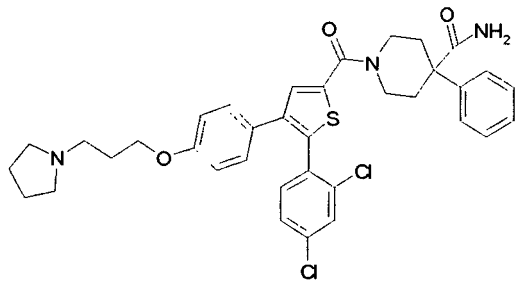 |
| 1-{[5-(2,4-dichlorophényl)-4-{4-[(4-hydroxybutyl)thio] phényl}-2-thiényl]carbonyl}-4-phénylpipéridine-4-carboxamide | 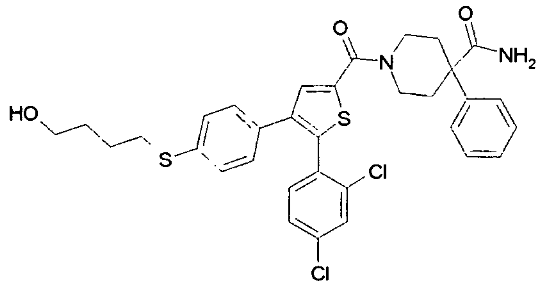 |
| 1-{[5-(2,4-dichlorophényl)-4-{4-[(4-hydroxybutyl)sulfinyl] phényl}-2-thiényl]carbonyl}-4-phénylpipéridine-4-carboxamide | 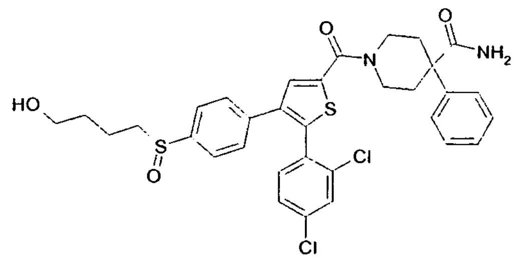 |
| 1- {[5-(2,4-dichlorophényl)-4-{4-[(4-hydroxybutyl)sulfonyl] phényl}-2-thiényl]carbonyl}-4-phénylpipéridine-4-carboxamide | 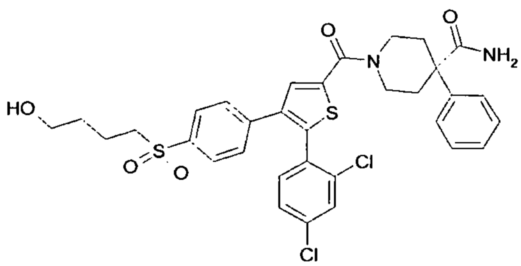 |
| propane-1-sulfonate de 4-{5-[(4-carbamoyl -4-phénylpipéridin-1-yl) carbonyl]-2-(2-chlorophényl)thién-3-yl} phényle | 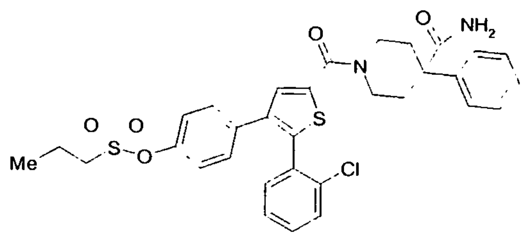 |
| 3,3,3-trifluoropropane-1-sulfonate de 4-{5-[(4-carbamoyl-4-phénylpipéridin-1-yl) carbonyl]-2-(2-chlorophényl)thién-3-yl} phényle | 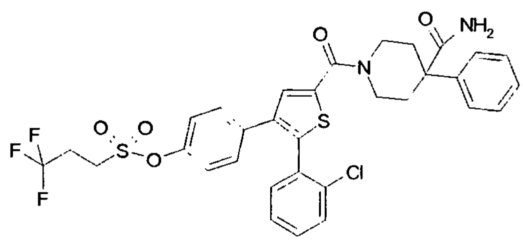 |

(suite)

| Nom IUPAC | Structure Chimique |
|---|---|
| 1-{[5-(2,4-dichlorophényl)-4-{4-[(4,4,4-trifluorobutyl)thio] phényl}-2-thiényl]carbonyl}-4-phénylpipéridine-4-carboxamide | 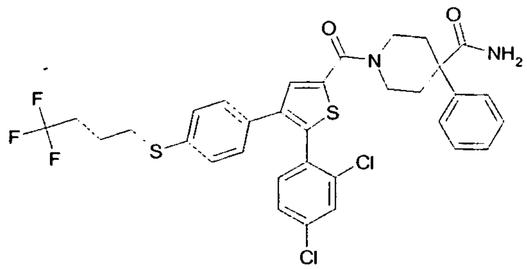 |
| 1-{[5-(2,4-dichlorophényl)-4-{4-[(4,4,4-trifluorobutyl) sulfonyl]phényl -2-thiényl]carbonyl}-4-phénylpipéridine-4-carboxamide | 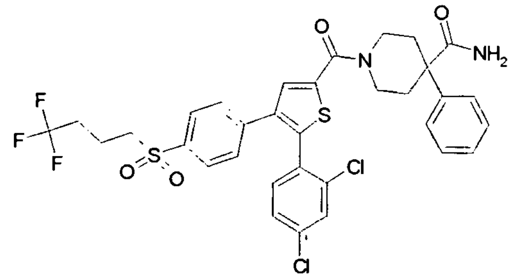 |
| 1-({5-(2-chloro-4-fluorophényl)-4-[4-(3-hydroxypropoxy) phényl]-2-thiényl}carbonyl)-4-phénylpipéridine-4-carboxamide | 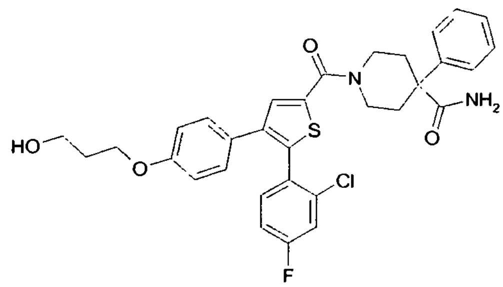 |
| 1'-({5-(2-chloro-4-fluorophényl)-4-[4-(3-hydroxypropoxy) phényl]-2-thiényl{carbonyl)-4,4-difluoro-1,4'-bipipéridine-4'-carboxamide | 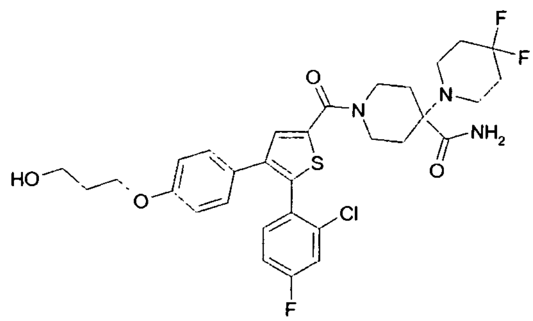 |
| 1-({5-(2-chloro-4-fluorophényl)-4-[4-(3-hydroxypropoxy) phényl]-2-thiényl}carbonyl)-4-[(3,3,3-trifluoropropyl)amino] pipéridine-4-carboxamide | 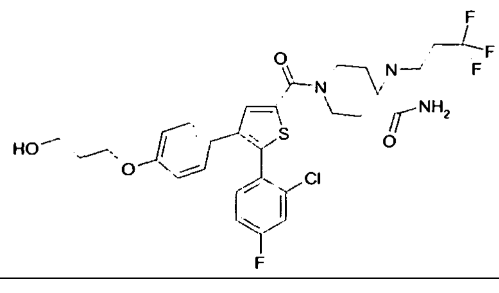 |

(suite)

| Nom IUPAC | Structure Chimique |
|---|---|
| 1- {[5-(2-chloro-4-fluorophényl)-4-{4-[3-(méthylthio)propoxy] phényl}-2-thiényl]carbonyl}-4-phénylpipéridine-4-carboxamide | 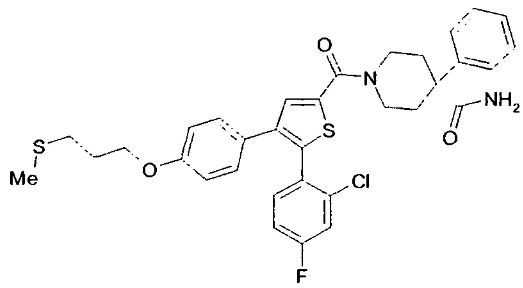 |
| 1-{[5-(2-chloro-4-fluorophényl)-4-{4-[3-(méthylsulfonyl) propoxy]phényl]-2-thiényl]carbonyl}-4-phénylpipéridine-4-carboxamide | 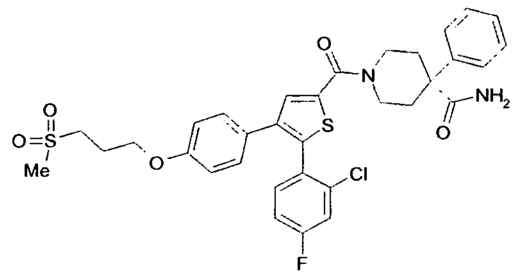 |
| 1'-{[5-(2-chloro-4-fluorophényl)-4-{4-[3-(méthylthio) propoxy]phényl}-2-thiényl]carbonyl}-4,4-difluoro-1,4'-bipipéridine-4'-carboxamide | 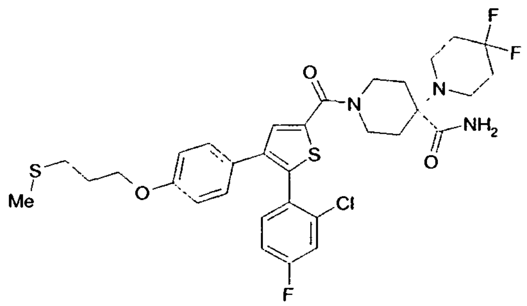 |
| 1'-{[5-(2-chloro-4-fluorophényl)-4-{4-[3-(méthylsulfonyl) propoxy]phényl}-2-thiényl]carbonyl}-4,4-difluoro-1,4'-bipipéridine-4'-carboxamide | 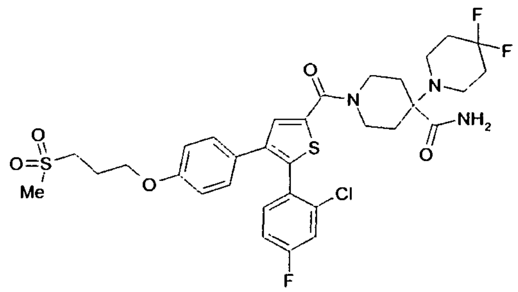 |
| 1-{[4-(4-[(2-aminoethyl)thio]phényl}-5-(2,4-dichlorophényl)-2-thiényl]carbonyl}-4-phénylpipéridine-4-carboxamide | 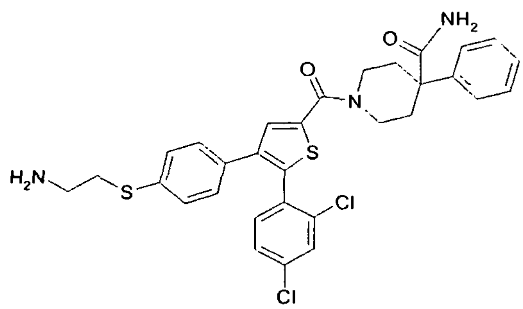 |

(suite)

| Nom IUPAC | Structure Chimique |
|---|---|
| 1-({5-(2,4-dichlorophényl)-4-[4-({2-[(méthylsulfonyl)amino] ethyl}thio)phényl] -2-thiényl}carbonyl)-4-phénylpipéridine-4-carboxamide | 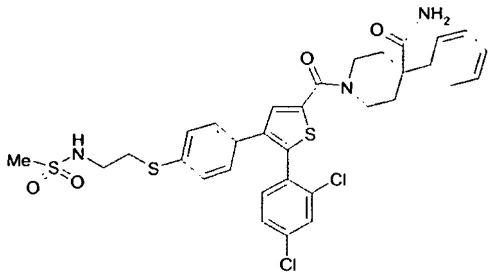 |
| 1-({5-(2,4-dichlorophényl)-4-[4-(2-hydroxyethoxy)phényl]-2-thiényl}carbonyl)-4-phénylpipéridine-4-carboxamide | 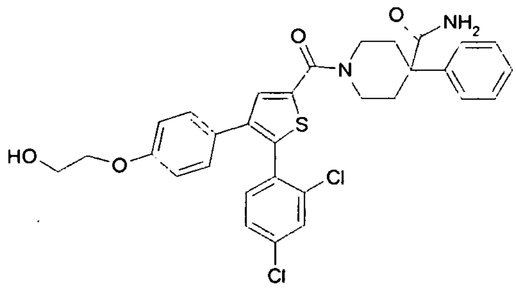 |
| 1'-({5-(2,4-dichlorophényl)-4-[4-(3-hydroxypropoxy)phényl]-2-thiényl}carbonyl)-4,4-difluoro-1,4'-bipipéridine-4'-carboxamide | 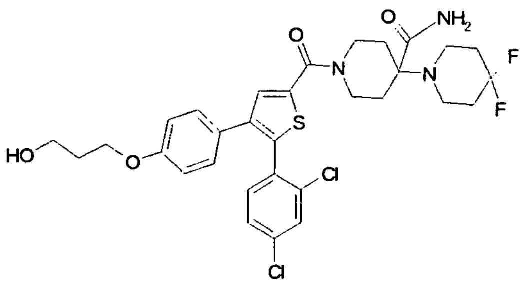 |
| 1-({5-(2,4-dichlorophényl)-4-[4-(3-hydroxypropoxy)phényl]-2-thiényl}carbonyl)-4-[(2,2,2-trifluoroethyl)amino]pipéridine-4-carboxamide | 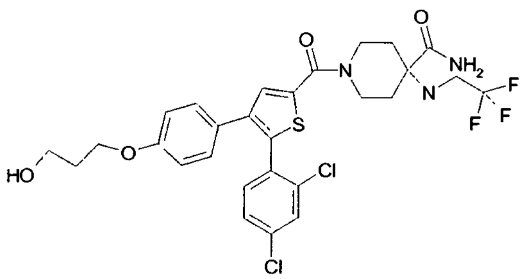 |
| 1'-({5-(2,4-dichlorophényl)-4-[4-(2-hydroxyethoxy)phényl]-2-thiényl}carbonyl)-4,4-difluoro-1,4'-bipipéridine-4'-carboxamide | 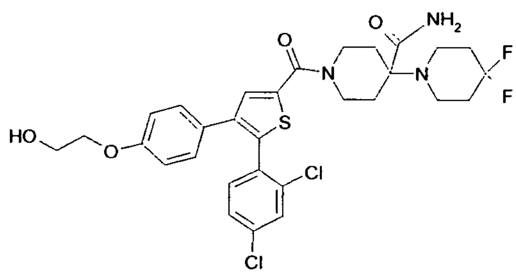 |

(suite)

| Nom IUPAC | Structure Chimique |
|---|---|
| propane-1-sulfonate de 4-{5-[(4-carbamoyl-4-phénylpipéridin-1-yl)carbonyl]-2-(2,4-dichlorophényl)thién-3-yl}phényle | 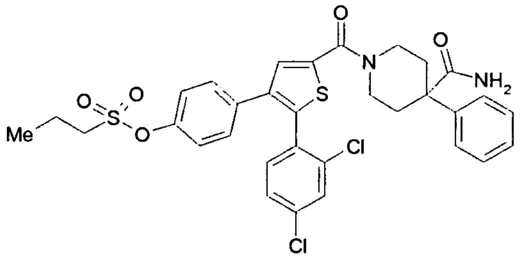 |
| 1-{[5-(2,4-dichlorophényl)-4-{4-[(3-hydroxypropyl)sulfonyl] phényl}-2-thiényl]carbonyl}-4-phénylpipéridine-4-carboxamide | 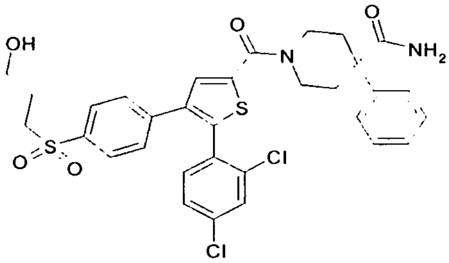 |
| 1-{[5-(2,4-dichlorophényl)-4-{4-[(3-hydroxypropyl)thio] phényl}-2-thiényl]carbonyl}-4-phénylpipéridine-4-carboxamide | 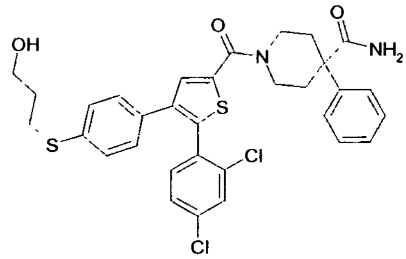 |
| 1-({4-[4-(3-amino-3-oxopropoxy)phényl]-5-(2-chlorophényl)-2-thiényl}carbonyl)-4-phénylpipéridine-4-carboxamide | 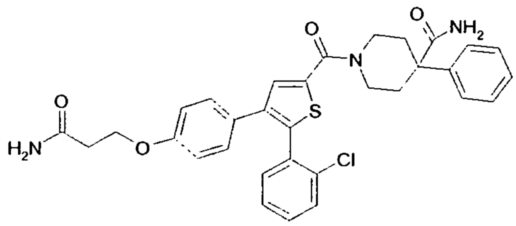 |
| 1-{[5-(2,4-dichlorophényl)-4-{4-[3-(méthylthio)propoxy] phényl}-2-thiényl]carbonyl}-4-phénylpipéridine-4-carboxamide | 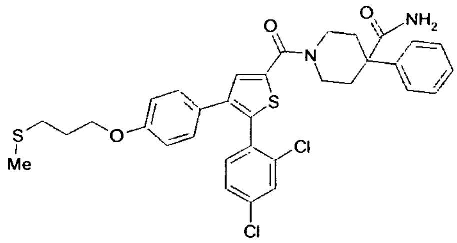 |
| 1-{[5-(2,4-dichlorophényl)-4-{4-[3-(méthylsulfonyl)propoxy] phényl}-2-thiényl]carbonyl}-4-phénylpipéridine-4-carboxamide | 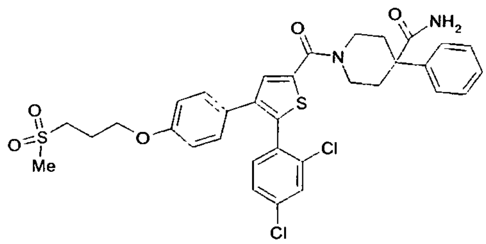 |

(suite)

| Nom IUPAC | Structure Chimique |
|---|---|
| 1- {[5-(2,4-dichlorophényl)-4-{4-[(4-hydroxybutyl)thio] phényl}-2-thiényl]carbonyl}-4-[(3,3,3-trifluoropropyl)amino] pipéridine-4-carboxamide | 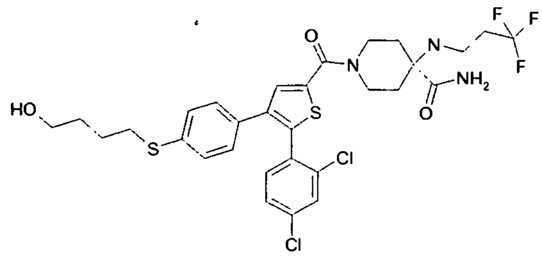 |
| 1-({5-(2,4-dichlorophényl)-4-[4-(2-hydroxy éthoxy)phényl] thién-2-yl}carbonyl)-4-[(3,3,3-trifluoropropyl)amino] pipéridine-4-carboxamide | 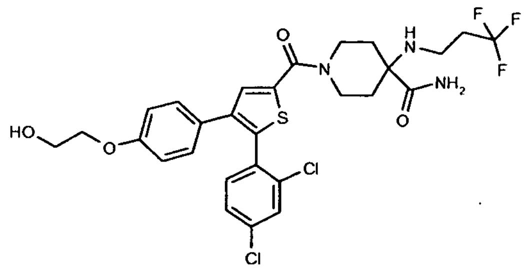 |
| 1-[4-[4-(butane-1-sulfonyl)-phényl]-5-(2,4-dichlorophényl)-thiophène-2-carbonyl]-4-phénylpipéridine-4-carboxamide | 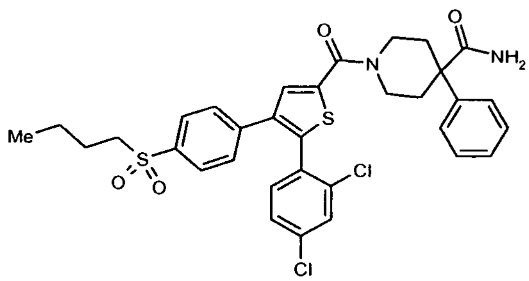 |
| 1'-{5-(2,4-dichloro-phényl)-4-[4-(4-hydroxy-butylsulfanyl)-phényl]-thiophène-2-carbonyl}-4,4-difluoro-[1,4']bipipéridinyl-4'-carboxamide | 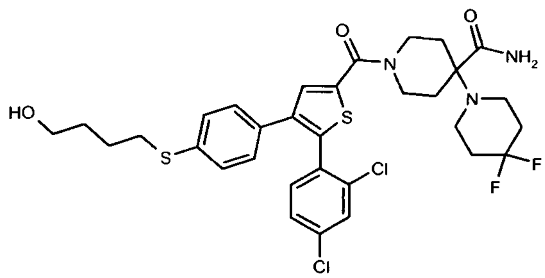 |
| 1-({5-(2-chloro-4-fluorophényl)-4-[4-(3-hydroxypropoxy) phényl]thién-2-yl}carbonyl)-3-phénylazétidine-3-carboxamide | 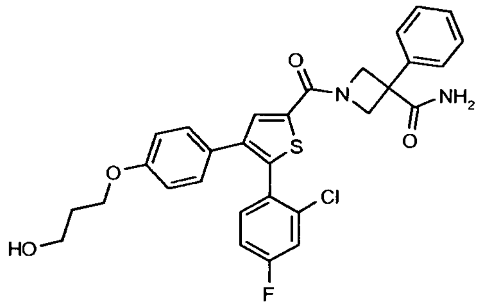 |

(suite)

| Nom IUPAC | Structure Chimique |
|---|---|
| 1'-{5-(2,4-dichloro-phényl)-4-[4-(3-hydroxy-propylsulfanyl)-phényl]-thiophène-2-carbonyl}-4,4-difluoro-[1,4']bipipéridinyl-4'-carboxamide | 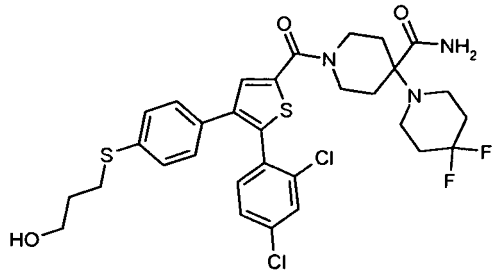 |
| 1-{5-(2,4-dichloro-phényl)-4-[4-(2-hydroxy-ethoxy)-phényl]-thiophène-2-carbonyl}-3-phénylazetidine-3-carboxamide | 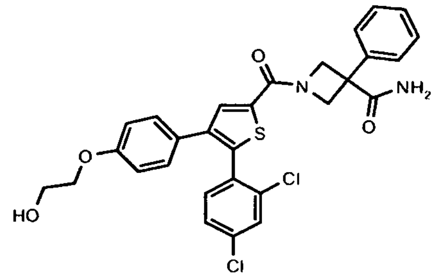 |
| 1-{5-(2,4-Dichloro-phényl)-4-[4-(3-hydroxy-propoxy)-phényl]-thiophène-2-carbonyl}-3-phénylazetidine-3-carboxamide | 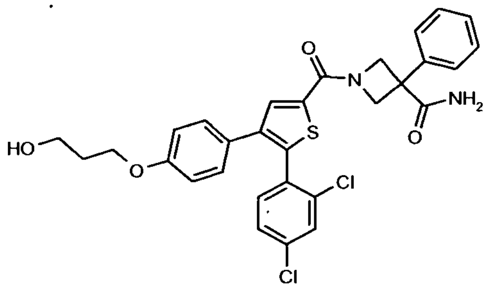 |
| 1-({5-(2-chlorophényl)-4-[4-(3-hydroxypropoxy)phényl] thién-2-yl}carbonyl)-4-phénylpipéridine-4-carboxamide | 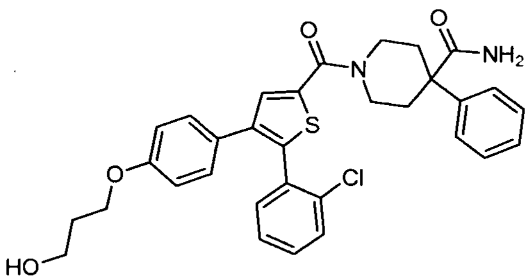 |
| 1-({5-(2-chlorophényl)-4-[4-(3-hydroxypropoxy)phényl] thién-2-yl}carbonyl)-4-[(4-fluorobenzyl)amino]pipéridine-4-carboxamide | 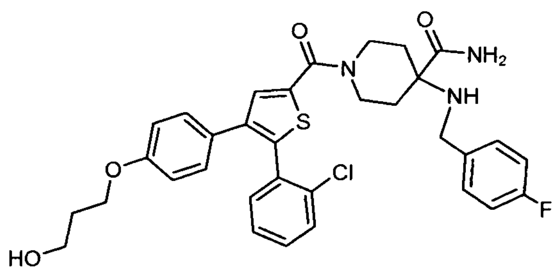 |

(suite)

| Nom IUPAC | Structure Chimique |
|---|---|
| 1-({5-(2-chlorophényl)-4-[4-(2-hydroxyéthoxy)phényl]thién-2-yl}carbonyl)-4-phénylpipéridine-4-carboxamide | 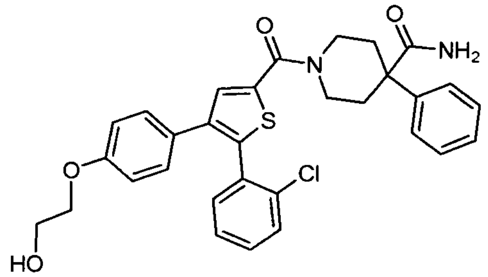 |
| 1-({5-(2-chlorophényl)-4-[4-(2-hydroxyéthoxy)phényl]thién-2-yl}carbonyl)-4-[(4-fluorobenzyl)amino]pipéridine-4-carboxamide | 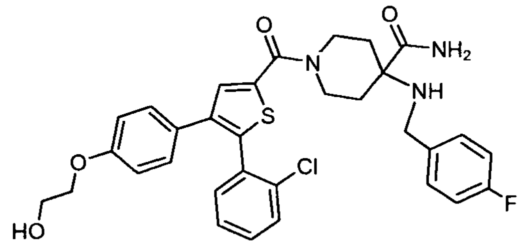 |

La présente invention a également pour objet un procédé de préparation des composés selon l'invention.

Ce procédé est caractérisé en ce que l'on traite avec une amine de formule (III) $HNR_1R_2$ dans laquelle $R_1$ et $R_2$ sont tels que définis pour (I) l'acide de formule (II) ou un dérivé fonctionnel de cet acide de formule (II) :

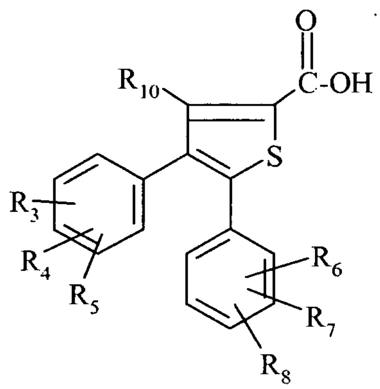

**(II)**

dans laquelle $R_3$, $R_4$, $R_5$, $R_6$, $R_7$, $R_8$ et $R_{10}$ sont tels que définis pour (I) et le groupe Y-A-$R_9$ est remplacé par un groupe Z précurseur chimique du groupe Y-A-$R_9$.

Par groupe Z, on entend des groupes chimiques qui conduisent après une ou plusieurs étapes réactionnelles connues de l'homme de l'art aux groupes Y-A-$R_9$. Par exemple, le groupe précurseur Z correspond à Y-H, un atome d'halogène, un groupe Y-A-OH, Y-A-Cl, Y-A-$CO_2$Alk, Y-A-S-Alk, Y-Alk, Y-A-OPr, Y-A-NHPg. Le radical Alk correspond à un radical carboné linéaire ou ramifié comprenant notamment 1 à 6 atomes de carbone, et de préférence correspond à un radical méthyle. Le groupe Pg correspond à un groupe protecteur de la fonction amine, par exemple le *tert*-butyloxycarbonyl. D'autres exemples de groupes protecteurs Pg sont donnés dans «Protective Group in Organic Synthesis», Green et al, 4e edition , John Wiley & Sons, Inc., New York, 2007. Le groupe Pr correspond à un groupe protecteur de la fonction alcool ; par exemple, le tétrahydropyranyle (THP), le tétrabutyldiméthylsilyle (TBDMS) et le triméthylsilyle (TMS).

Les composés de formule (I) obtenus par les différents modes opératoires peuvent être ultérieurement séparés du milieu réactionnel et purifiés selon les méthodes classiques, par exemple par cristallisation ou chromatographie.

Eventuellement, on transforme le composé de formule (I) ainsi obtenu en un de ses sels.

Comme dérivé fonctionnel de l'acide (II) on peut utiliser le chlorure d'acide, l'anhydride, un anhydride mixte, un ester alkylique en $C_1$-$C_4$ dans lequel l'alkyle est droit ou ramifié, un ester benzylique, un ester activé, par exemple l'ester de p-nitrophényle, ou l'acide libre opportunément activé, par exemple avec un agent de couplage tel que le N,N-dicyclohexylcarbodiimide, l'hexafluorophosphate de benzotriazol-1-yloxotris(diméthylamino)-phosphonium (BOP),

l'hexafluorophosphate de benzotriazol-1-yloxotris-(pyrrolidino)phosphonium (PyBOP), l'hexafluorophosphate de 2-(1-H-benzotriazol-1-yl)-1, 1, 3, 3-tétraméthyl-uronium) (HBTU) ou le O-benzotriazol 1 yl N, N, N', N', tetraméthylurronium tetrafluoroborate (TBTU).

Ainsi dans le procédé selon l'invention, on peut faire réagir le chlorure de l'acide de formule (II), obtenu par réaction du chlorure de thionyle ou du 1-chloro-N,N-2-triméthyl-1-propen-1-amine effectué selon Chem. Comm., 1988, 475-477, à 0°C sur l'acide de formule (II), avec une amine $HNR_1R_2$, dans un solvant inerte, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer l'anhydride mixte de l'acide de formule (II) par réaction du chloroformiate d'éthyle avec l'acide de formule (II), en présence d'une base telle que la triéthylamine, et à le faire réagir avec une amine $HNR_1R_2$, dans un solvant tel que le dichlorométhane, sous une atmosphère inerte, à la température ambiante, en présence d'une base telle que la triéthylamine.

Les composés de formule (II) peuvent être préparés selon le schéma 1 ci-après.

Pour ce mode de préparation, le groupe précurseur Z est :

- soit présent au sein du composé (IV) ; dans ce cas $R_3$ représente le groupe Z.
- soit présent au sein du composé (V); dans ce cas $R_6$ représente le groupe Z.

SCHEMA 1

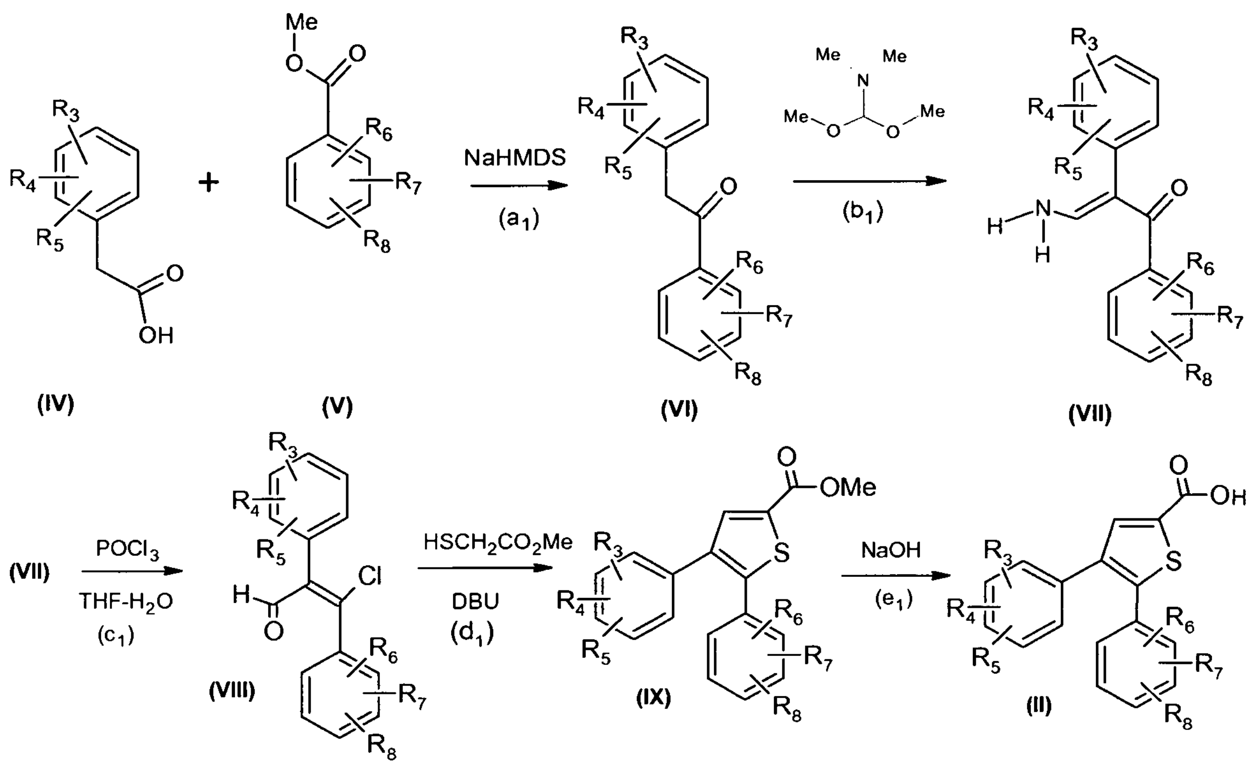


(IV) (V) (VI) (VII) (VIII) (IX) (II)

La préparation des composés de formule (VI) s'effectue à partir des composés de formule (IV) et (V) en présence d'une base forte telle que par exemple l'hexaméthyldisilazane de sodium (NaHMDS), l'hexaméthyldisilazane de lithium (LiHMDS), le dicyclohexylamide de sodium ($LiNCy_2$) ou le diisopropylamide de lithium (LDA).

Après réaction à l'étape (b1) avec la 1,1-diméthoxy-N,N- diméthylméthanamine, on fait réagir les composés (VII) avec le trichlorure de phosphoryle suivi d'une hydrolyse en présence d'un mélange solvant-eau tel que par exemple le tétrahydofurane-eau ou le dioxane-eau.

La cyclisation par le mercaptoacétate de méthyle à l'étape (d1) est effectuée en présence d'une base tel que par exemple une amine encombrée : le 1,8 diazabicyclo[5,4,0] undec-7-ène (DBU) ou 1,3-diazabicyclo[5.4.0]undécane (DBN). Les composés de formule (IX) obtenus sont ensuite traités avec de l'hydroxyde de sodium pour conduire aux composés de formule (II).

Les composés de formule (II) et dérivés fonctionnels de l'acide (II) peuvent également être préparés selon les modes de préparations décrits dans la demande internationale WO 2005/035 488 (voir notamment page 4 ligne 31 à page 9 ligne 32).

Ces composés de formule (II) et dérivés fonctionnels de l'acides (II) conduisent ensuite en une ou plusieurs étapes aux composés de formule (I) selon l'invention.

Ainsi, selon le schéma 2 ci-après, on part de l'acide de formule (IIa) dans laquelle $R_6$ est remplacé par le groupe Z. Cet acide est traité avec une amine de formule (III) $HNR_1R_2$, $R_1$ et $R_2$ étant tels que définis pour (I). On obtient un amide de formule (Xa).

Puis le groupe Z du composé de formule (Xa) obtenu est transformé en une ou plusieurs étapes en groupe Y-A-$R_9$ par une des méthodes connues de l'homme de l'art pour conduire aux composés de formule (I).

SCHEMA 2

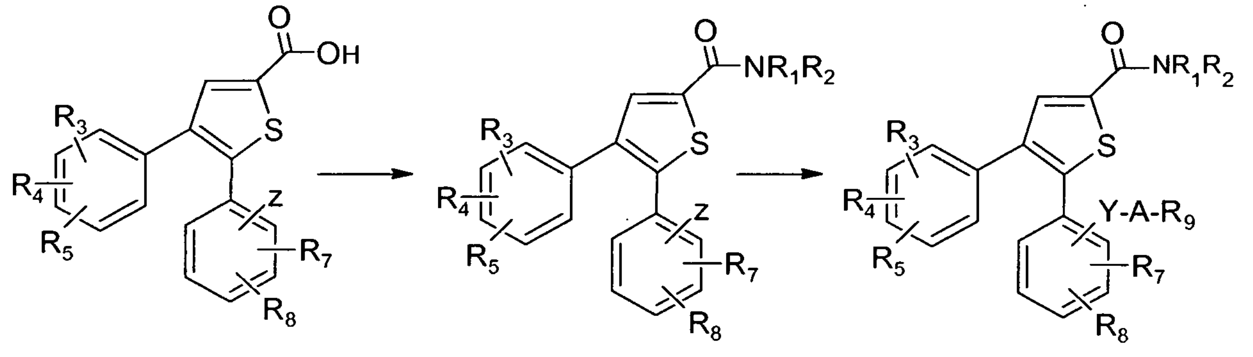

(IIa) (Xa) (I)

Ou bien selon le schéma 2' ci-après, on part de l'acide de formule (IIb) dans laquelle $R_3$ est remplacé par le groupe Z. Cet acide est traité avec une amine de formule (III) $HNR_1R_2$, $R_1$ et $R_2$ étant tels que définis pour (I). On obtient un amide de formule (Xb).

Puis le groupe Z du composé de formule (Xb) obtenu est transformé en une ou plusieurs étapes en groupe Y-A-$R_9$ par une des méthodes connues de l'homme de l'art pour conduire aux composés de formule (I).

SCHEMA 2'

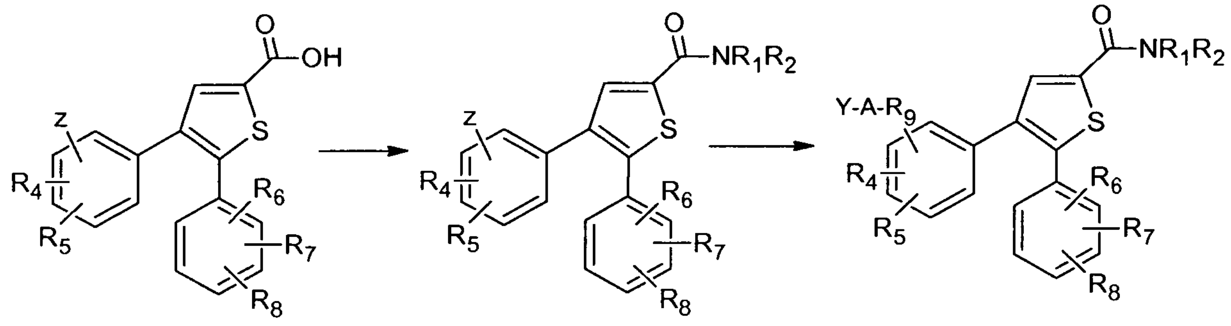

(IIb) (Xb) (I)

A titre d'exemples, les composés de formule (I) dans laquelle Y correspond à un atome d'oxygène et $R_9$ représente un groupe $OR_{19}$, $CO_2R_{19}$, $CONR_{19}R_{20}$, $NR_{19}R_{20}$, $NR_{18}SO_2R_{21}$, $S(O)_nR_{21}$ peuvent être obtenus à partir de composés de formule (IIb) dans laquelle $R_3$ est remplacé par Z, et Z correspond à Y-Alk, Alk étant en radical carboné linéaire ou ramifié, de préférence un radical méthyle.

Selon le schéma 3, le composé (XI) obtenu par réaction du composé (IIb) et de l'amine $HNR_1R_2$ est traité à l'étape (b3) avec du tribromure de bore puis le composé (XII) est traité à l'étape (c3) avec un chloroalcanol de formule Cl-A-OH ou bromoalcanol de formule Br-A-OH en milieu alcalin (ex: carbonate de potasium ou de césuim) pour mener au composé de formule (I) dans laquelle $R_9$ correspondant à un radical -OH.

## SCHEMA 3

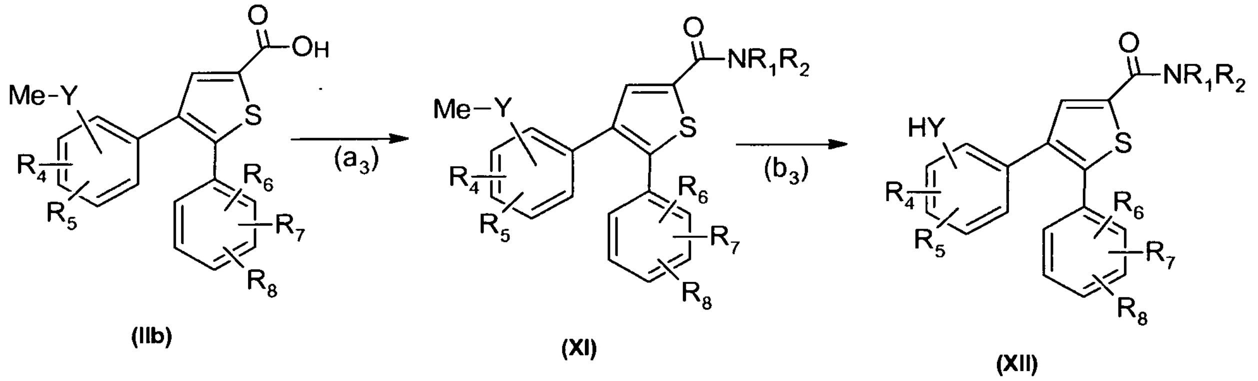


(IIb) (XI) (XII)

(XII)

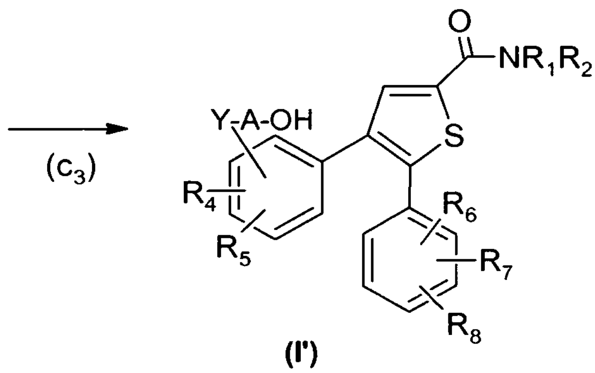


(I')

Les composés de formule (I) obtenus à l'issu du schéma réactionnel 3, référencés (I'), peuvent conduire à d'autres composés de formule (I).

Notamment selon le schéma 4, les composés (I') permettent de préparer les composés de formule (1) dans laquelle Y-A-$R_9$ correspond à Y-A-S-$R_{21}$. A l'étape (a4), le composé de formule (I') est traité avec du chlorure de mésyle en présence d'une amine tel que par exemple la triéthylamine. Puis, le composé obtenu est traité avec un alkylthiolate de sodium de formule -$R_{21}$SNa pour mener au composé de formule (I'') dans laquelle $R_9$ correspond à un radical -S-$R_{21}$.

## SCHEMA 4

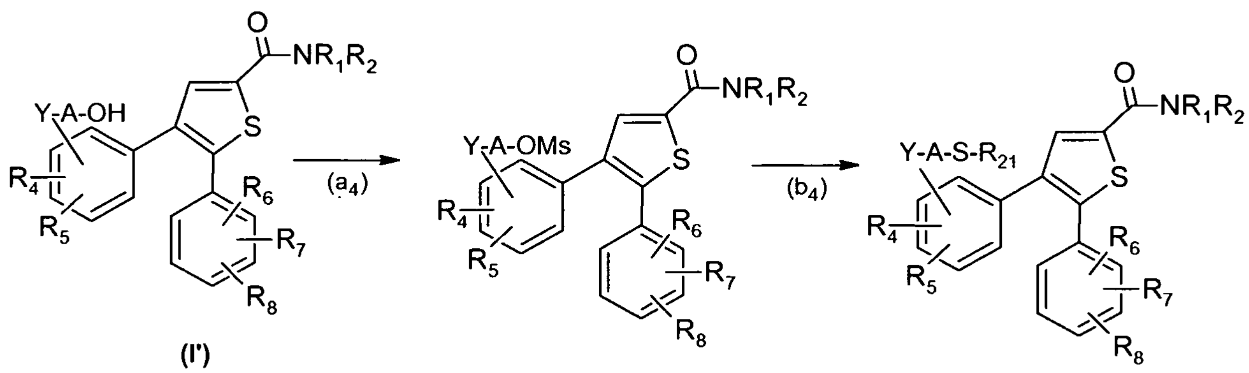


(I') (I'')

En accord avec le schéma 5, les composés de formule (I) obtenus à l'issu du schéma réactionnel 4, référencés (I''), peuvent ensuite être traités avec de l'acide 3-chloroperoxybenzoique (MCPBA) pour conduire aux composés de formule (I''') dans laquelle $R_9$ correspond à un radical -S(O)$_{n'}$-$R_{21}$ avec n' égal à 1 ou 2.

SCHEMA 5

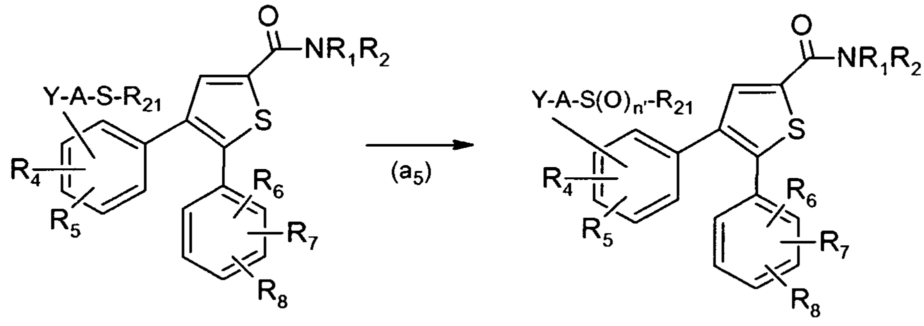


(I'') (I''')

Les composés de formule (XII) peuvent conduire à d'autres composés de formule (I). Par exemple, on ajoute dans une première étape à un composé de formule (XII) du *tert*-butyl (3-bromopropyl)carbamate puis dans une deuxième étape du TFA pour obtenir un composé de formule (I) dans laquelle $-R_9$ correspond à un radical $-NH_2$.

Les composés de formule (II) permettent également de conduire à des composés de formule (I) dans laquelle Y correspond au radical divalent $-S(O)_{n'}-$ et $-R_9$ représente un radical $-OR_{19}$, $-CH_3$ ou $-CF_3$.

Par exemple, on part d'un composé de formule (IIb) dans laquelle Z correspond à un atome de Br.

Selon le schéma 6, le composé de formule (IIb) avec Z correspondant à Br est mis en contact avec un réactif de formule $HS\text{-}A\text{-}OR_{19}$ et de l'hydrure de sodium. On ajoute ensuite un catalyseur tel que $Pd_2(dba)_3$ et un ligand tel que le composé organophosphoré Xantphos pour arriver à un composé de formule (XIII).Ce composé de formule (XIII) correspond à un composé de formule (I) dans lequel-Y-A-$R_9$ est -S-A-$OR_{19}$ .

SCHEMA 6

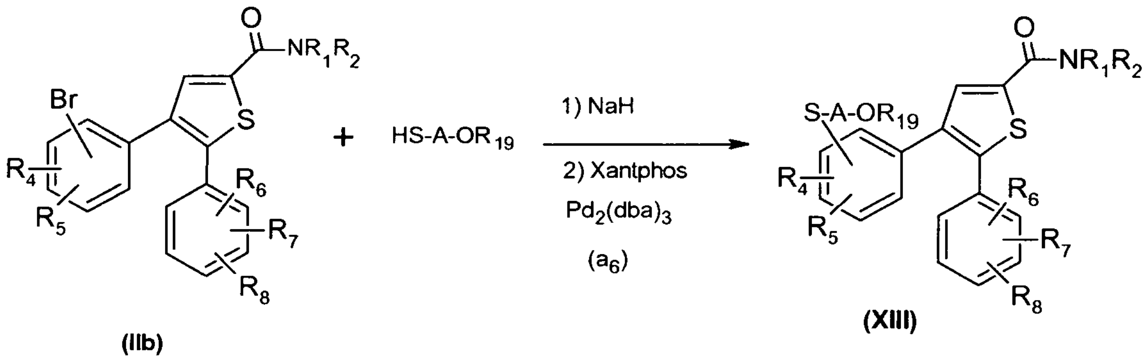


(IIb) (XIII)

Selon le schéma 7, le composé obtenu (XIII) via le mode opératoire du schéma 6 peut être ensuite traité avec du MCPBA pour oxyder le soufre présent sur la chaine latérale du phényle et mener à des composés de formule (I) dans laquelle -Y-A-R9 est -(SO)-A-O $R_{19}$ (ci-après le composé de formule (XIV) ou $-SO_2$-A-$OR_{19}$ (ci-après le composé de formule (XV).

SCHEMA 7

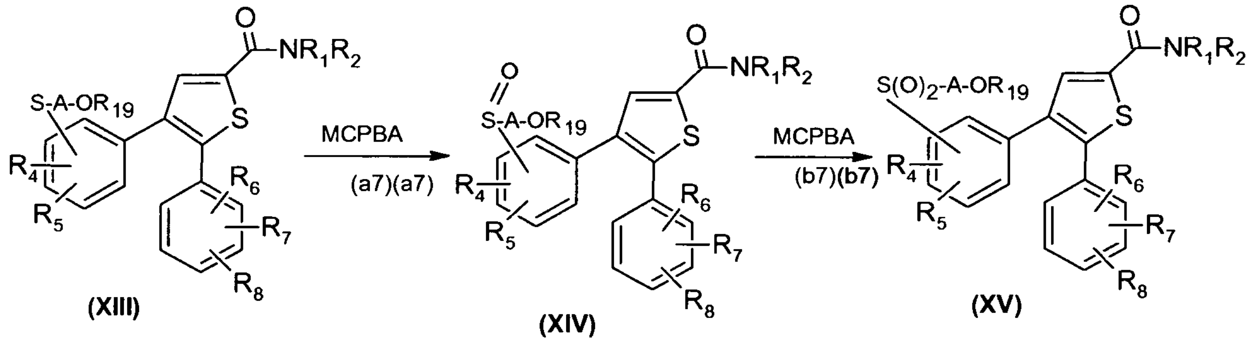


(XIII) (XIV) (XV)

En remplaçant dans la séquence réactionnelle du schéma 6 le réactif HS-A-$OR_{19}$ par un réactif de formule HS-A-$CH_3$ ou HS-A-$CF_3$, les composés de formules (XIII') et (XIII") avec n" égal à 0, 1 ou 2 sont préparés :

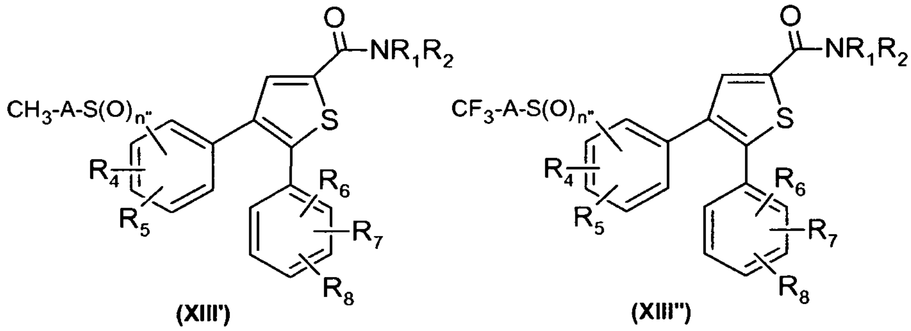

(XIII') (XIII")

Les composés de formule (IX) où $R_3$ ou $R_6$ correspond à Z et Z est OAlk permettent également de conduire à des composés de formule (I) où Y-A-$R_9$ correspond à O-A-OH ou O-A-$NHR_{19}$ selon le schéma 8 réactionnel ci-après.

Ainsi selon le schéma 8, on part du composé de formule (IX) où $R_3$ correspond à Z et Z est OAlk, référencé ci-après (XVI).

Alternativement, on peut partir du composé de formule (IX) où $R_6$ correspond à Z et Z est OAlk. Dans ce cas, le radical fonctionnalisé Y-A- $R_9$ du composé final est présent sur le phényle en position 5 du thiophène.

SCHEMA 8

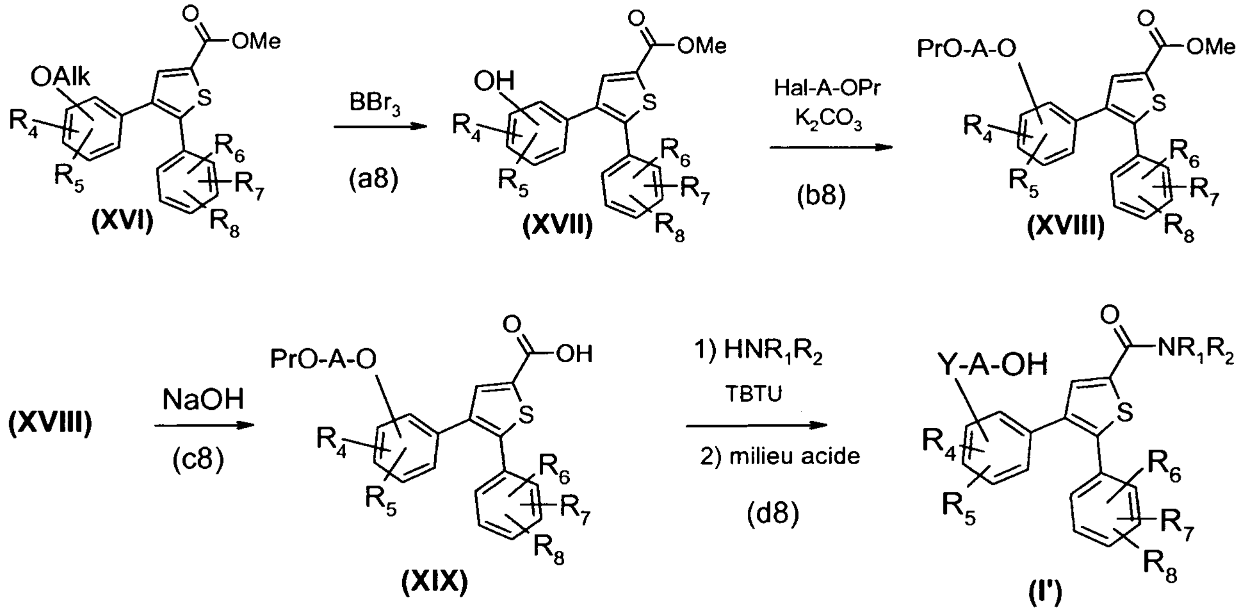


(XVI) (XVII) (XVIII) (XVIII) (XIX) (I')

Selon le schéma 8, le composé de formule (XVI) est traité avec $BBr_3$, Le composé obtenu de formule (XVII) est alkylé par un dérivé halogéné de formule Hal-A-OPr avec Hal égal à Br ou Cl pour obtenir un composé intermédiaire de formule (XVIII) dans laquelle Z est devenu O-A-OPr.

Après saponification avec par exemple NaOH, l'acide de formule (XIX) est traité à l'étape (d8) avec une amine de formule $HNR_1R_2$, en présence d'un agent de couplage tel que par exemple TBTU. Puis, le composé obtenu est traité en milieu acide pour conduire au composé de formule (I').

A l'étape (b8), on peut remplacer Hal-A-OPr par Hal-A-$NR_{19}$Pg . Dans ce cas, on obtient après saponification un composé intermédiaire de formule (XXI) qui conduit ensuite aux composés de formule (I) où Y-A-$R_9$ correspond à O-A-$NHR_{19}$.

SCHEMA 9

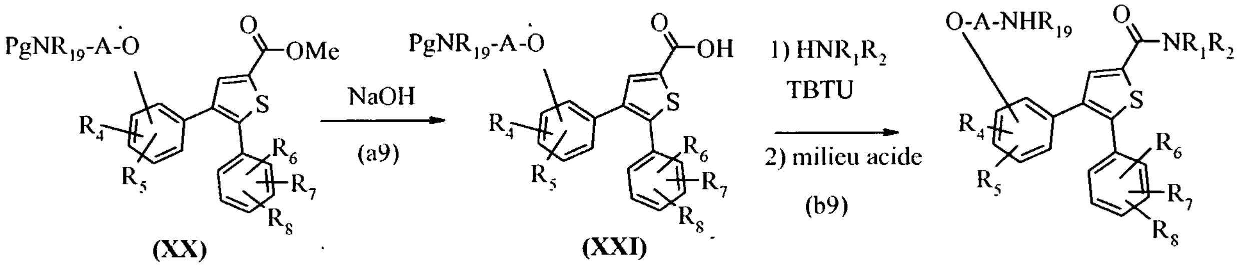


(XX) (XXI)

A l'étape (b8), on peut également remplacer Hal-A-OPr par Hal-A-$NR_{19}R_{20}$ avec $R_{19}$ et $R_{20}$ différents d'un atome d'hydrogène. Dans ce cas, on obtient après saponification un composé intermédiaire de formule (XXIII) qui conduit ensuite aux composés de formule (I) où Y-A-$R_9$ correspond à O-A-$NR_{19}R_{20}$.

SCHEMA 10

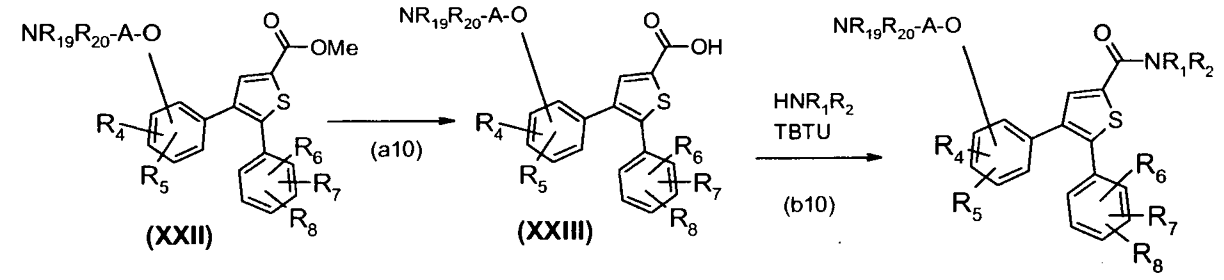


A partir des composés de formule (XVIII), on peut de plus préparer en suivant le schéma réactionnel 11 des composés de formule (I) où Y-A-$R_9$ correspond à O-A-$SR_{21}$.

A l'issue de l'étape (b11), les composés intermédiaires de formule (XXV) peuvent ensuite être traités avec de l'acide 3-chloroperoxybenzoique (MCPBA) pour conduire à des composés intermédiaires (XXV') dans laquelle -$R_9$ correspond à un radical - $S(O)_{n'}$-$R_{21}$ avec n' égal à 1 ou 2. Ensuite, ces composés (XXV') suivent les étapes

(c11) et (d11) pour mener à des composés de formule (I''') où Y-A-$R_9$ correspond à - O-A-$S(O)_{n'}R_{21}$ .

SCHEMA 11

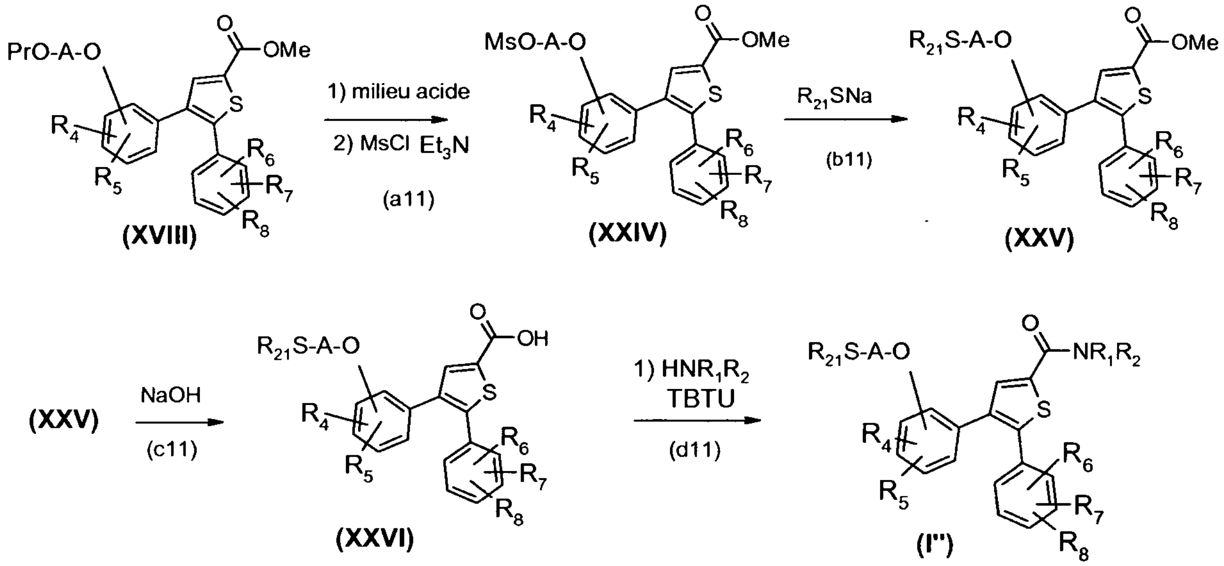


A partir de composés de formule (XXVII) en suivant le schéma réactionnel 12, on peut préparer des composés de formule (I) où Y-A-$R_9$ correspond à S-A-$OR_{19}$.L'étape (a12) est réalisée dans des conditions similaires à celles du schéma réactionnel 6 et l'étape (b12) de saponification puis celle de couplage (c12) sont effectuées dans des conditions similaires à celles des étapes (c8) et (d8) du schéma réactionnel 8.

SCHEMA 12

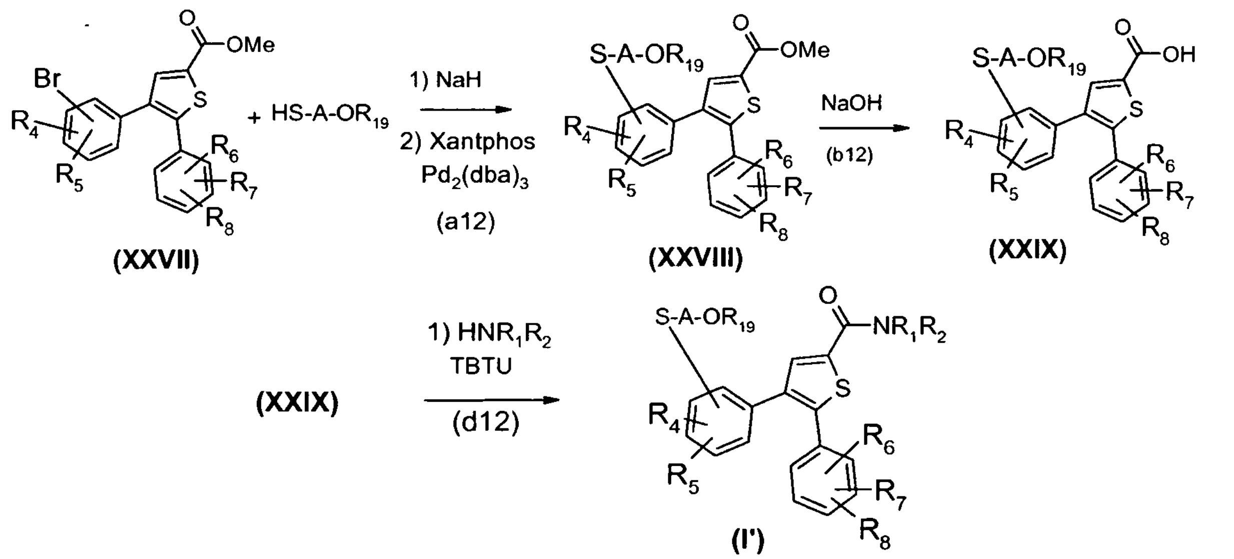


(XXVII) (XXVIII) (XXIX)

(XXIX) → (I')

Les amines de formule (III) $HNR_1R_2$ sont connues ou préparées par des méthodes connues, par exemple celle décrite dans J. Med. Chem., 7, 1964, 619-622.

Les composés intermédiaires de formules (XXII), (XXIII), (XXV), (XXVI), (XXVIII) et (XXIX) sont nouveaux et sont utilisés pour la préparation des composés de formule (I).

Les composés intermédiaires de formule (XXVII) correspondent aux composés de formule (IX) dans lequel $R_3$ correspond à un atome de brome. Ces composés de formule (XXVII) sont préparés selon le schéma réactionnel 1.

La présente invention a également pour objet les composés de formules (XXX) (XXXI) et (XXXII), incluant notamment les composés de formules (XXII), (XXIII), (XXV), (XXVI), (XXVIII), et (XXIX). Ces composés sont utilisés pour la préparation des composés de formule (I) et correspondent aux formules suivantes :

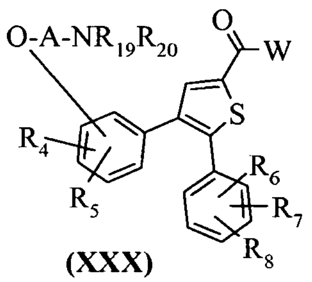

(XXX)

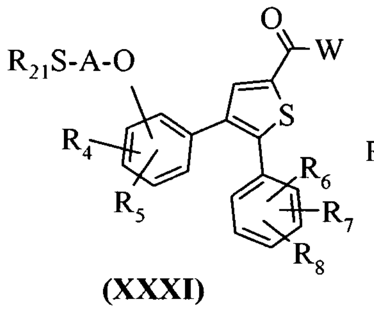

(XXXI)

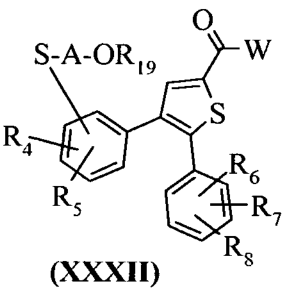

(XXXII)

dans lesquelles :

- W représente un groupe ($C_1$-$C_4$)alcoxy, de préférence méthoxy, un halogène, de préférence un atome de chlore ou un radical hydroxyle,
- et les autres substituants sont tels que définis pour les composés de formule (I).

Lorsque W représente un groupe ($C_1$-$C_4$)alcoxy, on traite les composés de formule (XXX), (XXXI) ou (XXXII) avec un agent de saponification tel que par exemple NaOH puis avec une amine de formule (III) $HNR_1R_2$ dans laquelle $R_1$ et $R_2$ sont tels que définis pour (I) pour préparer les composés de formule (I).

Lorsque W représente un radical hydroxyle ou un atome de chlore, on traite les composés de formule (XXX), (XXXI) ou (XXXII) avec une amine de formule (III) $HNR_1R_2$ dans laquelle $R_1$ et $R_2$ sont tels que définis pour (I) pour préparer les composés de formule (I).

Les exemples suivants décrivent la préparation de certains composés conformes à l'invention. Ces exemples ne sont pas limitatifs et ne font qu'illustrer la présente invention.

Dans les exemples, on utilise les abréviations suivantes :

AcOEt : acétate d'éthyle
$AcONH_4$ : acétate d'ammonium
$BBr_3$ : tribromure de bore
$CH_2Cl_2$ : dichlorométhane
DBU : 1,8 diazabicyclo[ 5,4,0 ]undèc-7-ene
DIPEA : diisopropyléthylamine
DCM : dichlorométhane
DMF : N,N-diméthylformamide.
$Et_3N$ : triéthylamine
HPLC : chromatographie en phase liquide sous haute pression
LiHMDS : hexaméthyldisilazane de lithium
MeOH : méthanol
MCPBA : acide 3-chloroperoxybenzoique
MsCl : chlorure de mésyle
NaHMDS : hexaméthyldisilylane de sodium
$NH_3$ : ammoniac
$NH_4OH$ : ammoniaque
$Pd(dba)_2$ : bis(dibenzyllideneacétone)palladium
$Pd_2(dba)_3$ : tris(dibenzyllideneacétone)dipalladium
$POCl_3$ : trichlorure de phosphoryle
$P(tBu)_3$ : tri*ter*-butylphosphine
TA : température ambiante
TBTU : O-benzotriazol-1-yl-N, N, N', N'- tétraméthylurronium tetrafluoroborate
Tf : point de fusion
TFA : acide trifluoroacétique
THF : tétrahydrofurane
UPLC : chromatographie en phase liquide 'Ultra Performance'
Xantphos :

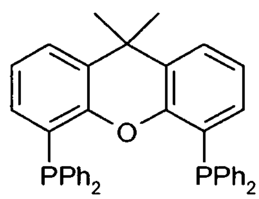

Les spectres de résonance magnétique nucléaire sont enregistrés à 250 MHz ou 400 MHz dans le DMSO-d6. Pour l'interprétation des spectres, on utilise les abréviations suivantes : s : singulet, d : doublet, t : triplet, q : quadruplet, qui : quintuplet, m : massif, sl : singulet large, dd : doublet de doublet.
Les composés selon l'invention sont analysés par couplage LC/UV/MS (chromatographie liquide/détection UV/spectrométrie de masse). On mesure le pic moléculaire caractéristique ($MH^+$) et le temps de rétention (tr) en minutes (min).
Les composés sont analysés par couplage HPLC-UV-MS ou bien UPLC-UV-MS (chromatographie liquide-détection UV et détection masse).
Les conditions analytiques sont les suivantes :

<u>Conditions A (HPLC) :</u>

On utilise une colonne: Symmetry C18 (50 x 2,1 mm ; 3,5 $\mu$m)
Eluant A : 0,005 % d'acide trifluoroacétique (TFA) dans l'eau à environ pH 3,1
Eluant B : 0,005% de TFA 0.005% dans l'acétonitrile.

Gradient:

| Temps (min) | % A | % B |
|---|---|---|
| 0 | 100 | 0 |
| 10 | 10 | 90 |
| 15 | 10 | 90 |
| 16 | 100 | 0 |
| 20 | 100 | 0 |
| Température colonne : 30°C ; débit : 0,4 ml/minute. Détection : λ = 210 nm - 220 nm | | |

Conditions B (HPLC) :

On utilise une colonne XTerra MS C18 ( 50 x 2,1 mm ; 3,5 μm)
Eluant A : $AcONH_4$ 10mM à environ pH 7
Eluant B : acétonitrile

Gradient :

| Temps (min) | % A | % B |
|---|---|---|
| 0 | 100 | 0 |
| 10 | 10 | 90 |
| 15 | 10 | 90 |
| 16 | 100 | 0 |
| 20 | 100 | 0 |
| Température colonne : 30°C ; débit : 0,4 ml/minute. Détection : λ = 220 nm | | |

Conditions C (UPLC) :

On utilise une colonne Acquity BEH C18 ( 50 x 2,1 mm ; 1,7 μm)
Eluant A : 0,005 % de TFA dans l'eau à environ pH 3,1 / acétonitrile (97 / 3)
Eluant B : 0,035% de TFA dans l'acétonitrile.

Gradient:

| Temps (min) | % A | % B |
|---|---|---|
| 0 | 100 | 0 |
| 2.3 | 5 | 95 |
| 2.9 | 5 | 95 |
| 3 | 100 | 0 |

(suite)

| Temps (min) | % A | % B |
|---|---|---|
| 3.5 | 100 | 0 |
| Température colonne : 40°C ; débit : 1 ml/minute. Détection : λ = 220 nm | | |

Conditions de spectrométrie de masse

L'enregistrement des spectres de masse est effectué en mode electrospray (ESI) positif, afin d'observer les ions issus de la protonation de composés analysés ($MH^+$), ou de la formation d'adduits avec d'autres cations tels que $Na^+$, $K^+$, etc.

PREPARATIONS

Préparation 1 : 4-[(3,3,3-trifluoropropyl)amino]pipéridine-4-carboxamide.

(i) 4-Carbamoyl-4-[(3,3,3-trifluoropropyl)amino]pipéridine-1-carboxylate de *tert*-butyle.

Dans 250 ml de $CH_2Cl_2$ on ajoute à TA, 10 g de 4-amino-4-carbamoylpipéridine-1-carboxylate de tert-butyle, 4,42 g de 3,3,3-trifluoro-propionaldéhyde, 17,61 g de triacétoxyborohydrure de sodium, 4,51 ml d'acide acétique puis on agite le mélange pendant 3 heures. On ajoute de l'eau et extrait le composé attendu au $CH_2Cl_2$, lave la phase organique avec une solution saturée de $NaHCO_3$, puis avec de l'eau. Après séchage de la phase organique, filtration et évaporation à sec, on obtient 13 g de composé attendu.

(ii) 4-[(3,3,3-trifluoropropyl)amino]pipéridine-4-carboxamide

On agite 13 g de composé obtenu en (i) dans 25 ml de MeOH, puis on ajoute 30 ml d'éther chlorhydrique (2M) et on laisse sous agitation pendant une nuit. Après filtration et séchage on obtient 9, 57 g de composé attendu.

Préparation 2 : Chlorhydrate de 4-phénylpipéridine-4-carboxamide.

(i) Chlorhydrate de 1-benzyl-4-phénylpipéridine-4-carboxamide.

Dans 77 ml d'acide sulfurique concentré, on ajoute 10 g de 1-benzyl-4-phényl-pipéridine-4-carbonitrile puis chauffe à 100°C pendant une heure. Le mélange réactionnel est ensuite ajouté sur de la glace puis basifié avec $NH_4OH$. Le produit est extrait au $CH_2Cl_2$, séché, évaporé. Après cristallisation du chlorhydrate dans l'éther, on obtient 5,7 g de produit attendu.

(ii) Chlorhydrate de 4-phénylpipéridine-4-carboxamide.

Dans 70 ml de MeOH, on ajoute 5,6 g de produit obtenu en (i), 6,7 g de cyclohexadiène, 0,5 g de palladium à 10% sur charbon et on chauffe le mélange réactionnel à reflux pendant 6 heures. On filtre le catalyseur sur de la célite et évapore à sec. Après cristallisation dans un mélange éther-éther isopropylique, on obtient 3 g de produit attendu.

Préparation 3 : Formiate de 3-phényl-azétidine-3-carboxamide.

(i) 1-Benzyl-3-méthyl-1,3-azétidinedicarboxylate.

Dans 150 ml de $CH_2Cl_2$, on ajoute 10 g de chlorhydrate d'ester méthylique d'acide azétidine-3-carboxylique puis 23 ml de triéthylamine. On refroidit et ajoute 13,5 g de benzyl chloroformate. Après une nuit à TA, on lave à l'eau puis à HCl (N). Après séchage, concentration à sec et purification par chromatographie sur silice (éluant : Heptane-AcOEt (gradient de 0% à 50%)), on obtient 13, 1 g de composé attendu.

(ii) 1-Benzyl-3-méthyl-3-phényl-1,3-azétidinedicarboxylate

Dans 40 ml de toluène, on ajoute 5,11 ml de bromo-benzène puis 0,5 g de $Pd(dba)_2$, 2,15 ml de solution à 10% dans l'hexane de tri*ter*butylphosphine ($P(tBu)_3$) puis 8,4 g de LiHMDS. On ajoute 11 g du composé obtenu à l'étape précédente (i) dans 10 ml de toluène en maintenant la température entre 15 et 20°C. Après une nuit, on verse sur une solution saturée de $NH_4Cl$, extrait à l'éther, sèche sur $MgSO_4$ et concentre à sec. Après purification par chromatographie sur silice (éluant : Heptane-AcOEt (gradient de 0% à 30%)), on obtient 3,25 g de composé attendu.

(iii) Ester benzylique d'acide 3-phényl-azétidine-1,3-dicarboxylique.

Dans 40 ml de méthanol, on ajoute 3,7 g de composé obtenu à l'étape précédente (ii) puis 3 ml de soude à 30%. On chauffe à reflux pendant 1 heure puis on concentre à sec. On reprend dans l'eau, lave à l'éther, puis acidifie avec HCl concentré. Après extraction à l'éther, séchage, concentration à sec et cristallisation dans le pentane, on obtient 2,87 g de composé attendu.

(iv) Ester benzylique d'acide 3-carbamoyl-3-phényl-azétidine-1-carboxylique.

Dans 60 ml de $CH_2Cl_2$, on ajoute 2,87 g de composé obtenu à l'étape précédente (iii), 2,4 ml de DIPEA puis 3,3 g de TBTU. On fait barboter du $NH_3$ dans le milieu en maintenant la température à 25°C. On agite à TA pendant une nuit. On concentre à sec, reprend dans l'acétate d'éthyle, lave à l'eau puis à l'HCl (N) et finalement avec une solution aqueuse à 10% de $NaHCO_3$.

Après séchage, concentration à sec puis cristallisation dans l'éther, on obtient 1,51 g de composé attendu.

(v) Formiate de 3-phényl-azétidine-3-carboxamide.

Dans 30 ml d'éthanol, on ajoute 1,51 g de composé obtenu à l'étape précédente (iv), 1,57 g de formiate d'ammonium, puis 0,15 g de palladium sur charbon à 10% .On agite à TA pendant 3 heures. On filtre le catalyseur et concentre à sec. Après cristallisation dans l'acétone, on obtient 0,985 g de composé attendu.

EXEMPLES

Exemple 1 : 1-({5-(2,4-dichlorophényl)-4-[4-(3-hydroxypropoxy)phényl]thién-2-yl}carbonyl)-4-phénylpipéridine-4-carboxamide.

1A) 1-(2,4-dichlorophényl)-2-(4-méthoxyphényl)éthanone.

Dans 250 ml de THF, on ajoute 367 ml de NaHMDS (2M dans le THF) et refroidit le mélange à -70°C. On ajoute alors 54 g d'acide (4- méthoxyphényl) acétique ; on laisse sous agitation pendant 2 heures puis ajoute 49 g de 2,4-dichlorobenzoate de méthyle. On laisse le mélange réactionnel revenir à 10°C, verse sur 2 litres d' HCl (2N) glacé et on extrait le produit à l'éther. Après lavage avec une solution saturée de bicarbonate de sodium, puis une solution saturée de NaCl, séchage de la phase organique et évaporation, le produit cristallise dans l'heptane ; on obtient 32 g du composé attendu.

1B) (2E)1-(2,4-dichlorophényl)-3-(diméthylamino)-2-(4-méthoxyphényl)prop-2-én-1-one.

Dans 100 ml de THF, on ajoute 15 g de composé obtenu en 1A) et 33,76 ml de 1,1-diméthoxy-*N,N*-diméthylméthanamine et on chauffe à 80°C pendant une nuit. Après évaporation à sec du mélange réactionnel, on ajoute de l'eau et extrait à l'éther. On sèche et on évapore. Après purification par chromatographie sur silice (éluant : Heptane-AcOEt (gradient de 0% à 30%)), on obtient 18 g de composé attendu.

1C) (2*E*)-3-chloro-3-(2,4-dichlorophényl)-2-(4-méthoxyphényl)acrylaldéhyde.

Dans 100 ml de $CH_2Cl_2$, on ajoute 18 g de composé obtenu en 1B), 12 ml de $POCl_3$ et chauffe à 45 °C pendant 3 heures. Après une nuit à TA, on évapore à sec, reprend dans du THF puis ajoute 50 ml d'eau. On extrait à l'éther, lave à l'eau puis avec une solution aqueuse saturée en bicarbonate de sodium. Après séchage sur $MgSO_4$ puis concentration à sec, on obtient 15,5 g de composé attendu.

1D) 5-(2,4-dichlorophényl)-4-(4-méthoxyphènyl)thiophène-2-carboxylate de méthyle.

Dans 80 ml d'acétonitrile, on mélange 11 g de composé obtenu en 1C) et 7,2 ml de mercaptoacétate de méthyle. On chauffe à 60°C et on ajoute du DBU. On maintient la température du milieu réactionnel à 60°C et on revient à TA pendant une nuit. On évapore à sec, ajoute une solution d'HCl 1N et extrait le composé à l'acétate d'éthyle. On sèche la phase organique et évapore. Après purification par chromatographie sur silice (éluant : Heptane-AcOEt (gradient de 0% à 30%)), on obtient 6,3 g du composé attendu.

1E) Acide 5-(2,4-dichlorophényl)-4-(4-méthoxyphényl)thiophène-2-carboxylique.

Dans un mélange 50-50 Dioxane-Méthanol, on ajoute 7,2 g du composé obtenu à l'étape 1D) et 1,83 g de NaOH. On chauffe le mélange réactionnel à 50°C pendant une nuit. On évapore à sec, ajoute de l'eau distillée et extrait les résidus organiques avec de l'éther. On acidifie la phase aqueuse avec HCl concentré puis on extrait l'acide avec de l'éther. La phase organique est séchée puis évaporée. On obtient 5,6 g du composé attendu.

1F) 1-{[5-(2,4-dichlorophényl)-4-(4-méthoxyphényl)thién-2-yl]carbonyl}-4-phénylpipéridine-4-carboxamide Dans 40 ml de $CH_2Cl_2$, on ajoute à 2 g du composé obtenu à l'étape 1E), 1,27 g de 4-phénylpipéridine-4-carboxamide (préparation 2), 2,22 ml de Et3N et 1,86 g de TBTU. Le mélange est mis sous agitation à TA pendant 3 heures. On évapore à sec, ajoute de l'eau et extrait à l'acétate d'éthyle. La phase organique est séchée puis évaporée. Après purification par chromatographie sur silice (éluant : Heptane-AcOEt (gradient de 0% à 30%)), on obtient 2,8 g du composé attendu.

1G) 1-{[5-(2,4-dichlorophényl)-4-(4-hydroxyphényl)thién-2-yl]carbonyl}-4-phénylpipéridine-4-carboxamide.

Dans 70 ml de $CH_2Cl_2$ à la température de la glace fondante, on ajoute à 2,8 g de composé obtenu à l'étape 1F), 19,8 ml de solution 1M de $BBr_3$ dans $CH_2Cl_2$. On laisse remonter la température à TA tout en continuant d'agiter pendant 3 heures.On verse ensuite le milieu réactionnel sur de l'eau et on récupère la phase organique qui est ensuite séchée puis évaporée. Après cristallisation dans un mélange éther - acétate d'éthyle, on obtient 1,8 g du composé attendu.

1H) 1-({5-(2,4-dichlorophényl)-4-[4-(3-hydroxypropoxy)phényl]thién-2-yl}carbonyl)-4-phénylpipéridine-4-carboxamide.

Dans 10 ml de DMF, on ajoute à 1,65 g du composé obtenu à l'étape 1G), 0,57 g de $K_2CO_3$ et 0,83 g de 3-chloropropan-1-ol. Le milieu réactionnel est chauffé à 90°C pendant 3 heures. On verse alors le milieu réactionnel sur de l'eau distillée et extrait le composé attendu à l'acétate d'éthyle. On sèche et on évapore la phase organique. Après cristallisation dans l'acétate d'éthyle, on obtient 1, 2 g du composé attendu.

Exemple 2 : 1-{[5-(2,4-dichlorophényl)-4-(4-{3-[(méthylsulfonyl)amino]propoxy} phényl)-thién-2-yl]carbonyl}-4-phénylpipéridine-4-carboxamide.

2A) Méthanesulfonate de 3-(4-{5-[(4-carbamoyl-4-phénylpipéridin-1-yl)carbonyl]-2-(2,4-dichlorophényl)thién-3-yl}phénoxy)propyle.

A 1,5 g de composé obtenu à l'étape 1H) mis dans 10 ml de $CH_2Cl_2$, on ajoute 0,07 ml de MsCl puis 0,02 ml de $Et_3N$. On laisse sous agitation pendant 1 heure. On évapore à sec, ajoute une solution d'HCl 0,1N et on extrait le composé attendu à l'acétate d'éthyle. Après séchage et évaporation, on obtient 0,15 g du composé attendu.

2B) 1-{[5-(2,4-dichlorophényl)-4-(4-{3-[(méthylsulfonyl)amino]propoxy}phényl) thién- 2-yl]carbonyl} -4-phénylpipéridine-4-carboxamide.

Dans 5 ml de DMF, on ajoute 0,029 g de méthanesulfonamide et 0,016 g d'hydrure de sodium puis on agite pendant 5 minutes. On ajoute alors 0,14 g du composé obtenu à l'étape 2A) dissout dans 2 ml de DMF et on agite 2 heures. On verse le milieu réactionnel sur de l'eau et on extrait à l'acétate d'éthyle. On sèche et on évapore. Après purification par chromatographie sur silice (éluant : $CH_2Cl_2$-MeOH (gradient de 0% à 3%), on obtient 0,035 g de composé attendu.

Exemple 3 : 1-({5-(2,4-dichlorophényl)-4-[4-(3-pyrrolidin-1-ylpropoxy)phényl]thién-2-yl}carbonyl)-4-phénylpipéridine-4-carboxamide.

Dans 5 ml de DMF, on ajoute 0,12 ml de pyrrolidine à 0,2 g du composé obtenu à l'étape 2A), puis on chauffe

à 70°C pendant 2 heures. On verse le milieu réactionnel sur de l'eau distillée et on extrait le composé à l'acétate d'éthyle. On sèche et on évapore. Après lavage à l'éther et filtration, on obtient 0,1 g du composé attendu.

Exemple 4 : 1-({4-(2,4-dichlorophényl)-5-[4-(3-hydroxypropoxy)phényl]thién-2-yl}carbonyl)-4-phénylpipéridine-4-carboxamide.

4A) 1-{[4-(2,4-dichlorophényl)-5-(4-méthoxyphényl)thién-2-yl]carbonyl}-4-phénylpipéridine-4-carboxamide.

L'acide 4-(2,4-dichlorophényl)-5-(4-méthoxyphényl)thiophène-2-carboxylique est préparé suivant le mode opératoire utilisé dans les étapes 1A - 1E à partir d'acide (2,4-dichlorophényl)acétique et de 4-méthoxybenzoate de méthyle . Dans 100 ml de $CH_2Cl_2$, on ajoute à TA 3,8 g d'acide 4-(2,4-dichlorophényl)-5-(4-méthoxyphényl) thiophène-2-carboxylique, 2,53 g de 4-phénylpipéridine-4-carboxamide (préparation 2), 0,72 ml de $Et_3N$ et 3,53 g de TBTU. Le mélange est mis sous agitation pendant une nuit. On évapore à sec, on ajoute de l'eau et on extrait le composé obtenu à l'acétate d'éthyle. On sèche la phase organique et évapore à sec. Après cristallisation, lavage avec de l'éther éthylique, filtration puis séchage, on obtient 5,1 g du composé attendu.

4B) 1-{[4-(2,4-dichlorophényl)-5-(4-hydroxyphényl)thién-2-yl]carbonyl}-4-phénylpipéridine-4-carboxamide.

Dans 100 ml de $CH_2Cl_2$ à la température de la glace fondante, on ajoute 5 g du composé issu de l'étape 4A) puis 35,37 ml de solution de 1M de $BBr_3$ dans $CH_2Cl_2$. On laisse la température du milieu réactionnel revenir à TA pendant la nuit. On verse ensuite le milieu réactionnel sur de l'eau distillée et extrait le composé obtenu au $CH_2Cl_2$. On sèche et on évapore la phase organique. Après cristallisation dans l'éther éthylique, on obtient 3,1 g du composé attendu.

4C) 1-({4-(2,4-dichlorophényl)-5-[4-(3-hydroxypropoxy)phényl]thién-2-yl}carbonyl)-4-phénylpipéridine-4-carboxamide.

Dans 6 ml de DMF, on ajoute 1 g du composé obtenu à l'étape 4B), 0, 376 g de carbonate de potassium et 0,223g de 3-chloropropan-1-ol. On chauffe le milieu réactionnel à 110°C pendant 3 heures. On verse ensuite le milieu réactionnel sur de l'eau distillée et on extrait le composé obtenu à l'acétate d'éthyle. On sèche et on évapore la phase organique. Après purification par chromatographie sur silice (éluant : $CH_2Cl_2$-MeOH (gradient de 0 % à 4 %)), on obtient 0,6 g de composé attendu.

Exemple 5 : Chlorhydrate de 1-({5-[4-(3-aminopropoxy)phényl]-4-(2,4-dichlorophényl)thién-2-yl}carbonyl)-4-phénylpipéridine-4-carboxamide.

5A) [3-(4-{5-[(4-Carbamoyl-4-phénylpipéridin-1-yl)carbonyl]-3-(2,4-dichloro phényl)thién-2-yl}phénoxy)propyl]carbamate de tert-butyle.

Dans 60 ml d'acétone, on ajoute 1 g du composé obtenu à l'étape 4B), 0,504 g de carbonate de potassium et 0,868 g de (3-bromopropyl)carbamate de *tert*-butyle puis on chauffe le milieu réactionnel à reflux pendant 4 heures. On filtre les insolubles et évapore à sec. Après purification par chromatographie sur silice (éluant : $CH_2Cl_2$ - MeOH (gradient de 0 % à 7 %)). On obtient 0,6 g de composé attendu.

5B) Chlorhydrate de 1-({5-[4-(3-aminopropoxy)phényl]-4-(2,4-dichlorophényl)thién-2-yl}carbonyl)-4-phénylpipéridine-4-carboxamide.

Dans 5 ml de $CH_2Cl_2$, on ajoute 0,4g de composé obtenu en 5A), 1,28 g de TFA et on laisse sous agitation à TA pendant 1 heure. On évapore à sec, ajoute une solution de soude 1N et extrait le composé au $CH_2Cl_2$. La phase organique est séchée avant d'ajouter de l'éther chlorhydrique pour former le sel. Après évaporation à sec et séchage sous vide, on obtient 0,28 g de composé attendu.

Exemple 6 : 1-{[4-(2,4-dichlorophényl)-5-(4-{3-[(méthylsulfonyl)amino] propoxy}phényl)thién-2-yl]carbonyl}-4-phénylpipéridine-4-carboxamide.

Dans 10 ml de $CH_2Cl_2$, on ajoute 0,19 g de composé obtenu en 5B), 0,063 ml de $Et_3N$ puis 0,03 ml de MsCl et on laisse sous agitation pendant 2 heures. On évapore à sec, ajoute une solution de soude puis extrait le composé au $CH_2Cl_2$. On sèche et on évapore. Après purification par chromatographie sur silice (éluant : $CH_2Cl_2$ - MeOH (gradient de 0% à 5%)), on obtient 0,085 g de composé attendu.

Exemple 7 : 1-{[4-(2,4-dichlorophényl)-5-{4-[3-(méthylthio)propoxy]phényl}thién-2-yl] carbonyl}-4-phénylpipéridine-4-carboxamide.

7A) Méthanesulfonate de 3-(4-{5-[(4-carbamoyl-4-phénylpipéridin-1-yl)carbonyl]-3-(2,4-dichlorophényl)thién-2-yl}phénoxy)propyle.

Dans 20 ml de $CH_2Cl_2$ à TA, on ajoute 0,8 g de composé obtenu en 4C), 0,37 ml de $Et_3N$, 0,12 ml de MsCl puis on laisse sous agitation pendant 1 heure. On évapore à sec, ajoute une solution d'HCl 0,1N et extrait le composé à l'acétate d'éthyle. Après séchage et évaporation, on obtient 0,79 g de composé attendu.

7B) 1-{[4-(2,4-dichlorophényl)-5-{4-[3-(méthylthio)propoxy]phényl}thién-2-yl] carbonyl}-4-phénylpipéridine-4-carboxamide.

Dans 4 ml de DMF, on ajoute 0,79 g de composé obtenu en 7A), 0,097 g de méthanethiolate de sodium puis on laisse sous agitation pendant 4 heures à TA. On verse ensuite le mélange réactionnel sur de l'eau distillée et extrait le composé à l'acétate d'éthyle. On sèche et on évapore. Après purification par chromatographie sur silice (éluant : $CH_2Cl_2$ - MeOH (gradient de 0 % à 5 %)), on obtient 0,55 g de composé attendu.

Exemple 8 : 1-{[4-(2,4-dichlorophényl)-5-{4-[3-(méthylsulfonyl)propoxy] phényl}thién-2-yl] carbonyl}-4-phénylpipéridine-4-carboxamide.

Dans 20 ml de $CH_2Cl_2$ à TA, on ajoute 0,27 g de composé obtenu en 7B), 0,26 g de MCPBA à 70% puis on laisse sous agitation pendant une nuit. On ajoute alors une solution de $Na_2CO_3$ à 10 % et agite vigoureusement pendant 30 minutes. On sépare la phase organique, la sèche et évapore. Après purification par chromatographie sur silice (éluant : $CH_2Cl_2$ - MeOH (gradient de 0 % à 4 %)), on obtient 0,07 g de composé attendu.

Exemple 9 : 1-{[5-(2,4-dichlorophényl)-4-{4-[(4-hydroxybutyl)thio]phényl}thién-2-yl]carbonyl}-4-phénylpipéridine-4-carboxamide.

9A) 1-{[4-(4-bromophényl)-5-(2,4-dichlorophényl)thién-2-yl]carbonyl}-4-phénylpipéridine-4-carboxamide.

L'acide 4-(4-bromophényl)-5-(2,4-dichlorophényl)-thiophène-2-carboxylique est préparé suivant le mode opératoire utilisé dans les étapes étapes 1A - 1E à partir de l'acide (4-bromophényl)acétique et de 2,4-dichlorobenzoate de méthyle . Dans 100 ml de $CH_2Cl_2$, on ajoute à TA 7 g d'acide 4-(4-bromophényl)-5-(2,4-dichlorophényl)-thiophène-2-carboxylique, 3,94 g de chlorhydrate de 4-phénylpipéridine-4-carboxamide (préparation 2), 6,83 ml de $Et_3N$, 5,77 g de TBTU puis on agite le mélange pendant une nuit. On évapore à sec, ajoute de l'eau et extrait le composé attendu à l'acétate d'éthyle. On sèche la phase organique avec $MgSO_4$, filtre et évapore à sec. Après cristallisation, lavage avec de l'éther éthylique, filtration et séchage, on obtient 8,41 g de composé attendu.

9B) 1-{[5-(2,4-dichlorophényl)-4-{4-[(4-hydroxybutyl)thio]phényl}thién-2-yl] carbonyl}-4-phénylpipéridine-4-carboxamide.

Dans 25 ml de xylène à 0°C et préalablement dégazé 15 minutes à l'argon, on ajoute 0,455 g de 4-mercaptobutan-1-ol et 0,072 g d'hydrure de sodium puis on laisse agiter 1 heure en laissant remonter à TA. On ajoute alors 1 g de composé obtenu en 9A), 0,119 g de $Pd_2(dba)_3$ et 0,087 g de xantphos. Le milieu réactionnel est ensuite chauffé à 150°C pendant la nuit. On évapore le milieu réactionnel à sec, ajoute de l'eau et de l'acétate d'éthyle, filtre l'insoluble, lave la phase organique avec de l'eau, sèche la phase organique, filtre et concentre. Après purification par chromatographie sur silice (éluant : $CH_2Cl_2$ - MeOH (gradient de 0% à 3%)), on obtient 0,35 g de composé attendu.

Exemple 10 : 1-{[5-(2,4-dichlorophényl)-4-{4-[(4-hydroxybutyl)sulfinyl]phényl} thién-2-yl]carbonyl}-4-phénylpipéridine-4-carboxamide.

Dans 20 ml de $CH_2Cl_2$ à TA, on ajoute 0,23 g de composé obtenu en 9B), 0,109 g de MCPBA à 70 % puis on laisse sous agitation pendant une nuit. On ajoute alors une solution de $Na_2CO_3$ à 10% et agite ensuite pendant 15 minutes. La phase organique est séparée, séchée et évaporée. Après purification par chromatographie sur silice (éluant : $CH_2Cl_2$ - MeOH (gradient de 0 % à 4 %)), on obtient 0,14 g de composé attendu.

Exemple 11 : 1-{[5-(2,4-dichlorophényl)-4-{4-[(4-hydroxybutyl)sulfonyl]phényl}thién-2-yl]carbonyl}-4-phénylpipéridine-4-carboxamide.

Dans 20 ml de $CH_2Cl_2$ à TA, on ajoute 0,16 g de composé obtenu à l'exemple 10 et 0,121 g de MCPBA à 70 % puis on laisse sous agitation pendant une nuit. On ajoute alors une solution de $Na_2CO_3$ à 10 % et agite ensuite pendant 15 minutes. On sépare la phase organique, la sèche et évapore. Après purification par chromatographie sur silice (éluant : $CH_2Cl_2$ - MeOH (gradient de 0 % à 4 %)), on obtient 0,05 g de composé attendu.

Exemple 12 : Propane-1-sulfonate de 4-{5-[(4-carbamoyl-4-phénylpipéridin-1-yl)carbonyl]-2-(2-chlorophényl)thién-3-yl} phényle.

12A) Acide 5-(2-chlorophényl)-4-(4-méthoxyphényl)thiophène-2-carboxylique.

L'acide 5-(2-chlorophényl)-4-(4-méthoxyphényl)-thiophène-2-carboxylique est préparée suivant le mode opératoire utilisé dans les étapes 1A - 1E à partir de l'acide (4-méthoxyphényl) acétique et de 2-chlorobenzoate de méthyle. A la dernière étape de ce mode opératoire, on ajoute 5 g de méthyl 5-(2-chlorophényl)-4-(4-méthoxyphényl)-2-thiophènecarboxylate et 1,11 g de soude en pastille dans 30 ml de méthanol et 3 ml de dioxane. On chauffe le milieu réactionnel à 50-60°C pendant 5 heures. On évapore à sec, ajoute de l'eau et extrait les impuretés à l'éther. On acidifie la phase aqueuse et extrait le composé attendu à l'éther. Après séchage et évaporation, on obtient 3,5 g de composé attendu.

12B) 1-{[5-(2-chlorophényl)-4-(4-méthoxyphényl)thién-2-yl]carbonyl}-4-phénylpipéridine-4-carboxamide.

Dans 100 ml de $CH_2Cl_2$, on ajoute à TA 1,5 g de composé obtenu en 12A), 1,047 g de chlorhydrate de 4-phénylpipéridine-4-carboxamide, 1,83 ml de $Et_3N$, 1,56 g de TBTU puis on agite le mélange pendant une nuit. On évapore à sec, ajoute de l'eau et extrait le composé attendu à l'acétate d'éthyle. On sèche là phase organique, filtre et évapore à sec. Le composé qui a cristallisé est lavé avec de l'éther éthylique puis filtré et séché. On obtient 2 g de composé attendu.

12C) 1-{[5-(2-chlorophényl)-4-(4-hydroxyphényl)thién-2-yl]carbonyl}-4-phénylpipéridine-4-carboxamide.

Dans 50 ml de $CH_2Cl_2$ à la température de la glace fondante, on ajoute 1,7 g de composé obtenu à l'étape 12B) puis 12,8 ml de solution 1M de $BBr_3$ dans $CH_2Cl_2$ et on laisse revenir la température du milieu réactionnel à TA pendant la nuit. On verse ensuite le milieu réactionnel sur de l'eau distillée et extrait le composé attendu au $CH_2Cl_2$. On sèche et on évapore la phase organique. Le composé cristallise dans l'éther éthylique. On obtient 1,2 g de composé attendu.

12D) Propane-1-sulfonate de 4-{5-[(4-carbamoyl-4-phénylpipéridin-1-yl)carbonyl] -2-(2-chlorophényl)thién-3-yl}phényle.

Dans 30 ml de $CH_2Cl_2$, on ajoute 0,3 g de composé obtenu en 12C), 0,29 ml de $Et_3N$ puis 0,207 g de propane-1-sulfonyl chloride à TA. Après 3 heures, on verse le milieu réactionnel sur de l'eau distillée et extrait le composé. Après séchage, évaporation puis lavage avec de l'éther, on obtient 0,13 g de composé attendu.

Exemple 13 : 3,3,3-trifluoropropane-1-sulfonate de 4-{5-[(4-carbamoyl-4-phénylpipéridin-1-yl)carbonyl]-2-(2-chlorophényl)thién-3-yl phényle.

Dans 30 ml de $CH_2Cl_2$, on ajoute 0,3 g de composé obtenu à l'étape 12C), 0,29 ml de $Et_3N$, puis 0,28 g de chlorure de 3,3,3-trifluoropropane-1-sulfonyle à TA. Après 3 heures, on évapore à sec. On ajoute de l'eau et de l'acétate d'éthyle puis on agite l'ensemble. Après filtration, rinçage à l'éther puis séchage, on obtient 0,13 g de composé attendu.

Exemple 14 : 1-{[5-(2,4-dichlorophényl)-4-{4-[(4,4,4-trifluorobutyl)thio]phényl thién-2-yl]carbonyl}-4-phénylpipéridine-4-carboxamide.

Dans 25 ml de xylène à 0°C et préalablement dégazé 15 minutes à l'Argon, on ajoute 0,455 g de 4,4,4-trifluorobutane-1-thiol et 0,072 g d'hydrure de sodium puis on laisse agiter 1 heure en laissant remonter à TA. On ajoute alors 1 g de composé obtenu en 9A), 0,119 g de $Pd_2(dba)_3$ et 0,087 g de xantphos. Le milieu réactionnel est ensuite chauffé à 150°C pendant la nuit. On évapore le milieu réactionnel à sec, ajoute de l'eau et de l'acétate d'éthyle, filtre l'insoluble, lave la phase organique avec de l'eau, sèche la phase organique, filtre et concentre. Après purification par

chromatographie sur silice (éluant : $CH_2Cl_2$-MeOH (gradient de 0% à 4 %)), on obtient 0,16 g de composé attendu.

Exemple 15 : 1-{[5-(2,4-dichlorophényl)-4-{4-[(4,4,4-trifluorobutyl)sulfonyl] phényl]thién-2-yl]carbonyl}-4-phénylpipéridine-4-carboxamide.

Dans 20 ml de $CH_2Cl_2$ à TA, on ajoute 0,36 g de composé obtenu à l'exemple 14), 0,385g de MCPBA à 70 % puis on laisse sous agitation pendant une nuit. On ajoute alors une solution de $Na_2CO_3$ à 10 % et agite vigoureusement pendant 30 minutes. On sépare la phase organique, la sèche et l'évapore. Après purification par chromatographie sur silice (éluant : $CH_2Cl_2$-MeOH (gradient de 0 % à 4 %)), on obtient 0,19 g de composé attendu.

Exemple 16 : 1-({5-(2-Chloro-4-fluorophényl)-4-[4-(3-hydroxypropoxy)phényl] thién-2-yl}carbonyl)-4-phénylpipéridine-4-carboxamide.

16A) 1-(2-chloro-4-fluorophényl)-2-(4-méthoxyphényl)éthanone.

Dans 250 ml de THF, on introduit sous azote 230 ml de solution 2M de NaHMDS dans le THF. On refroidit à -60°C puis ajoute à cette température 30,5 g d'acide (4-méthoxyphényl)acétique dans 120 ml de THF. Après 1h 30 mn à -60°C, on additionne 33 g de 2-chloro-4-fluorobenzoate de méthyle, agite à -60°C pendant 45 mn puis on laisse revenir à 0°C. On verse le milieu réactionnel sur 500 ml d' HCl 2N glacé, extrait à l'éther, lave à l'eau puis avec une solution aqueuse saturée en chlorure de sodium. Après séchage, concentration à sec puis cristallisation dans le pentane, on obtient 27, 4 g de composé attendu.

16B) (2E)-1-(2-chloro-4-fluorophényl)-3-(diméthylamino)-2-(4-méthoxyphényl) prop-2-én-1-one

Dans 100 ml de THF, on ajoute 27,4 g de composé obtenu en 16A), 35 g de 1,1-diméthoxy-*N,N*-diméthylméthanamine et on porte à reflux pendant 3 heures. Après concentration à sec puis cristallisation dans l'éther isopropylique, on obtient 24,7 g de composé attendu.

16C) (2E)-3-chloro-3-(2-chloro-4-fluorophényl)-2-(4-méthoxyphényl)acrylal-déhyde.

Dans 200 ml de $CH_2Cl_2$, on ajoute 24,7 g de composé obtenu à l'étape 16B) puis 17,3 g de $POCl_3$. On chauffe à 45°C pendant une nuit puis on concentre à sec. On reprend dans du THF puis ajoute 50 ml d'eau. On extrait à l'éther, lave à l'eau puis avec une solution aqueuse saturée en chlorure de sodium. Après séchage, concentration à sec, on obtient 24 g de composé attendu.

16D) 5-(2-chloro-4-fluorophényl)-4-(4-méthoxyphényl)thiophène-2-carboxylate de méthyle.

Dans 240 ml d'acétonitrile, on ajoute 24 g de composé obtenu en 16C) puis 16,5 ml de mercaptoacétate de méthyle. On chauffe à 60°C et ajoute 12 ml de DBU. On maintient à 60°C pendant 2 heures puis à TA pendant une nuit. On concentre à sec, reprend dans l'acétate d'éthyle, lave à l' HCl 1N puis à l'eau. Après séchage, concentration à sec puis cristallisation dans le méthanol, on obtient 20,6 g de composé attendu.

16E) 5-(2-chloro-4-fluorophényl)-4-(4-hydroxyphényl)thiophène-2-carboxylate de méthyle.

Dans 100 ml de $CH_2Cl_2$, on ajoute le composé obtenu à l'étape 16D) et on refroidit à -50°C, ajoute 40 ml de solution 1M de $BBr_3$ dans $CH_2Cl_2$. On laisse revenir la température à 20°C puis on ajoute 20 ml de méthanol. On concentre à sec, reprend dans du $CH_2Cl_2$, lave à l'eau, sèche sur $MgSO_4$, concentre à sec. Après purification par chromatographie sur silice (heptane jusqu'à heptane / AcOEt 75/25). On obtient 4,8 g de composé attendu.

16F) 5-(2-chloro-4-fluorophényl)-4-{4-[3-(tétrahydro-2H-pyran-2-yloxy)propoxy] phényl}thiophène-2-carboxylate de méthyle.

Dans 30 ml de DMF, on ajoute 4,8 g de composé obtenu à l'étape 16E), 3,6 g de 2-(3-bromopropoxy)tétrahydro-2*H*-pyran, 2,2 g de carbonate de potassium puis on chauffe à 60°C pendant 7 heures. On verse sur eau glacée, extrait à l'éther, sèche et concentre à sec. Après purification par chromatographie sur silice (heptane puis heptane / AcOEt : 90 / 10), on obtient 6,2 g de composé attendu.

16G) Acide 5-(2-chloro-4-fluorophényl)-4-{4-[3-(tétrahydro-2H-pyran-2-yloxy)propoxy]phényl}thiophène-2-carboxylique.

Dans 30 ml de méthanol et 1 ml d'eau, on ajoute 3,1 g de composé obtenu à l'étape 16F) puis 800 mg de soude en pastille. On porte le mélange réactionnel à reflux pendant 2 heures. On concentre à sec, reprend dans 500 ml de solution tampon à pH 2, extrait à l'éther, sèche et concentre à sec. On obtient 3 g de composé attendu, cristallisé dans le pentane.

16H) 1-{[5-(2-chloro-4-fluorophényl)-4-{4-[3-(tétrahydro-2H-pyran-2-yloxy) propoxy]phényl}thién-2-yl]carbony}-4-phénylpipéridine-4-carboxamide.

Dans 10 ml de $CH_2Cl_2$, on ajoute 0,5 g de composé obtenu à l'étape 16G), 0,25 g de chlorhydrate de 4-phénylpipéridine-4-carboxamide, 0,43 ml de $Et_3N$ et 0,36 g de TBTU puis on laisse la réaction 2 heures à TA. Après concentration à sec, reprise dans un mélange eau / éther, et filtration, on obtient 0,6 g de composé attendu.

16I) 1-({5-(2-chloro-4-fluorophényl)-4-[4-(3-hydroxypropoxy)phényl]thién-2-yl}carbonyl)-4-phénylpipéridine-4-carboxamide.

Dans 15 ml de méthanol, on ajoute 0,6 g de composé obtenu à l'étape 16H), 2 ml d'éther chlorhydrique 2N. Après 2 heures à TA, on filtre le composé attendu. On obtient 0,385 g de composé attendu.

Exemple 17 : Chlorhydrate de 1'-({5-(2-Chloro-4-fluorophényl)-4-[4-(3-hydroxypropoxy)phényl]-2-thiényl}carbonyl)-4,4-difluoro-1,4'-bipipéridine-4'-carboxamide.

17A) 1'-{[5-(2-chloro-4-fluorophényl)-4-{4-[3-(tétrahydro-2*H*-pyran-2-yloxy) propoxy]phényl}thién-2-yl]carbonyl}-4,4-difluoro-1,4'-bipipéridine-4'-carboxamide.

Dans 10 ml de $CH_2Cl_2$, on ajoute 0,5 g de composé obtenu à l'étape 16G), 0,33 g de 4,4-difluoro-[1,4']bipipéridinyl-4'-carboxamide (décrit à la préparation 5 page 23 de la demande WO 2008/068 423), 0,57 ml de $Et_3N$ et 0,36 g de TBTU. Après 2 heures à TA, concentration à sec, reprise dans un mélange eau/éther et filtration, on obtient 0,63 g de composé attendu.

17B) Chlorhydrate de 1'-({5-(2-chloro-4-fluorophényl)-4-[4-(3-hydroxypropoxy) phényl]thién-2-yl}carbonyl)-4,4-difluoro-1,4'-bipipéridine-4'-carboxamide.

Dans 15 ml de méthanol, on ajoute 0,63 g du composé obtenu à l'étape 17A) et 2 ml d'éther chlorhydrique 2N. Après 1 heure à TA, concentration à sec puis cristallisation dans l'AcOEt, on obtient 0, 612 g de composé attendu.

Exemple 18 : Chlorydrate de 1-({5-(2-chloro-4-fluorophényl)-4-[4-(3-hydroxypropoxy)phényl]thién-2-yl}carbonyl)-4-[(3,3,3-trifluoropropyl)amino] pipéridine-4-carboxamide.

18A) 1-{[5-(2-chloro-4-fluorophényl)-4-{4-[3-(tétrahydro-2*H*-pyran-2-yloxy) propoxy]phényl}thién-2-yl]carbonyl}-4-[(3,3,3-trifluoropropyl)amino]pipéridine-4-carboxamide.

Dans 10 ml de $CH_2Cl_2$, on ajoute 0,5 g de composé obtenu à l'étape 16G), 0,41 g de 4-(3,3,3-trifluoro-propylamino)-pipéridine-4-carboxamide (préparation 1), 0,71 ml de $Et_3N$ et 0,36 g de TBTU. Après 3 heures à TA, on concentre à sec, reprend dans l'éther, lave à l'eau, sèche et concentre à sec. Après purification par chromatographie sur silice (gradient d'élution : heptane/ AcOEt : 90/10 jusqu'à AcOEt pur). On obtient 0,7 g de composé attendu.

18B) Chlorydrate de 1-({5-(2-chloro-4-fluorophényl)-4-[4-(3-hydroxypropoxy) phényl]thién-2-yl}carbonyl)-4-[(3,3,3-trifluoropropyl)amino]pipéridine-4-carboxamide.

Dans 15 ml de méthanol, on ajoute 0,7g de composé obtenu à l'étape 18A) et 3 ml d'éther chlorhydrique 2N. Après 2 heures à TA, concentration à sec, reprise dans l'éther puis filtration on obtient 0,57 g de composé attendu.

Exemple 19 : 1-{[5-(2-chloro-4-fluorophényl)-4-{4-[3-(méthylthio)propoxy] phényl}thién-2-yl]carbonyl} -4-phénylpipéridine-4-carboxamide.

19A) 5-(2-chloro-4-fluorophényl)-4-[4-(3-hydroxypropoxy)phényl]thiophène-2-carboxylate de méthyle.

Dans 40 ml de méthanol, on ajoute 3,1 g de composé obtenu à l'étape 16F) et 1 ml de résine Amberlyst 15. On porte à reflux pendant 2 heures, filtre la résine et concentre à sec. On reprend dans l'éther, lave à l'eau, sèche puis concentre à sec. Après purification par chromatographie sur silice (gradient d'élution : heptane jusqu'à heptane / AcOEt ; 60/40), on obtient 2,0 g de composé attendu.

19B) 5-(2-chloro-4-fluorophényl)-4-(4-{3-[(méthylsulfonyl)oxy]propoxy}phényl) thiophène-2-carboxylate de méthyle.

Dans 40 ml de $CH_2Cl_2$, on ajoute 2 g de composé obtenu en 19A) puis 0,79 ml de $Et_3N$. On refroidit à 0°C puis ajoute 0,44 ml de MsCl. Après 15 minutes, on lave à l'eau, sèche et concentre à sec. On obtient 2,38 g de composé attendu.

19C) 5-(2-chloro-4-fluorophényl)-4-{4-[3-(méthylthio)propoxy]phényl}thiophène-2-carboxylate de méthyle.

Dans 12 ml de DMF, on ajoute 2,38 g de composé obtenu en 19B), 0,83 g de méthanethiolate de sodium et agite à TA pendant 2 heures. On verse sur de l'eau, extrait à l'éther, lave à l'HCl, dilue à l'eau, sèche et concentre à sec. Après purification par chromatographie sur silice (gradient d'élution : heptane jusqu'à heptane / AcOEt 93/7), on obtient 1,68 g de composé attendu.

19D) Acide 5-(2-chloro-4-fluorophényl)-4-{4-[3-(méthylthio)propoxy]phényl} thiophène-2-carboxylique.

Dans 20 ml de MeOH et 1 ml d'eau, on ajoute 1,68 g de composé obtenu en 19C) puis 0,45g de NaOH. On porte à reflux pendant 2 heures, puis concentre à sec. On reprend dans l'eau, acidifie avec de HCl 2N, extrait à l'éther, sèche et concentre à sec. On obtient 1,51 g de composé attendu.

19E) 1-{[5-(2-Chloro-4-fluorophényl)-4-{4-[3-(méthylthio)propoxy]phényl}thién-2-yl] carbonyl}-4-phénylpipéridine-4-carboxamide.

Dans 10 ml de $CH_2Cl_2$, on ajoute 0,5 g de composé obtenu en 19D), 0,28 g de chlorhydrate de 4-phénylpipéridine-4-carboxamide (préparation 2), 0,48 ml de $Et_3N$ et 0,405 g de TBTU. Après 2 heures à TA, on concentre à sec, reprend dans un mélange eau / éther et filtre. On obtient 0,635 g de composé attendu.

Exemple 20 : 1-{[5-(2-Chloro-4-fluorophényl)-4-{4-[3-(méthylsulfonyl)propoxy]phényl}thién-2-yl]carbonyl]-4-phénylpipéridine-4-carboxamide.

Dans 10 ml de $CH_2Cl_2$, on ajoute 0,43 g de composé obtenu à l'étape 19E), 0,425 g de MCPBA et on agite à TA pendant 3 heures. On lave avec une solution aqueuse de bicarbonate de sodium, sèche et concentre à sec. Après purification par chromatographie sur silice (gradient d'élution : $CH_2Cl_2$ jusqu'à $CH_2Cl_2$ / MeOH 98/2), on obtient 0,216 g de composé attendu.

Exemple 21 : Chlorhydrate de 1'-{[5-(2-chloro-4-fluorophényl)-4-{4-[3-(méthylthio)propoxy]phényl}thién-2-yl]carbony)}-4,4-difluoro-1,4'-bipipéridine-4'-carboxamide.

Dans 10 ml de $CH_2Cl_2$, on ajoute 0,5 g de composé obtenu à l'étape 19D), 0,51 g de 4,4-difluoro-[1,4']bipipéridinyl-4'-carboxamide (décrit à la préparation 5 page 23 de la demande WO 2008/068 423), 0,8 ml de $Et_3N$ et 0,405 g de TBTU. Après 3 heures à TA, on concentre à sec, reprend dans l'éther, lave à l'eau, sèche et concentre à sec. Après purification par chromatographie sur silice (gradient d'élution : $CH_2Cl_2$ jusqu'à $CH_2Cl_2$/MeOH 98/2), on dissout le composé obtenu dans $CH_2Cl_2$, ajoute de l'éther chlorhydrique jusqu' à pH 1, puis concentre à sec. Après reprise dans l'éther et filtration, on obtient 0,723 g de composé attendu.

Exemple 22 : Chlorhydrate de 1'-{[5-(2-chloro-4-fluorophényl)-4-{4-[3-(méthylsulfonyl)propoxy]phényl}thién-2-yl]carbonyl}-4,4-difluoro-1,4'-bipipéridine-4'-carboxamide.

22A) Acide 5-(2-chloro-4-fluorophényl)-4-{4-[3-(méthylsulfonyl)propoxy]phényl} thiophène-2-carboxylique.

Dans 15 ml de $CH_2Cl_2$, on ajoute 0,5 g de composé obtenu à l'étape 19D), 0,62 g de MCPBA puis on agite à TA pendant 4 heures. Après concentration, filtration et rinçage à l'éther isopropylique, on obtient 0,585 g de composé attendu.

22B) Chlorhydrate de 1'-{[5-(2-chloro-4-fluorophényl)-4-{4-[3-(méthylsulfonyl) propoxy]phényl}thién-2-yl]carbonyl}-4,4-difluoro-1,4'-bipipéridine-4'-carboxamide.

Dans 10 ml de $CH_2Cl_2$, on ajoute dans l'ordre 0,3 g de composé obtenu à l'étape 22A), 0,3 g de 4,4-difluoro-[1,4'] bipipéridinyl-4'-carboxamide (décrit à la préparation 5 page 23 de la demande WO 2008/068 423) , 0,45 ml de $Et_3N$, et 0,25 g de TBTU.

Après 3 heures à TA, on concentre à sec, reprend dans l'AcOEt, lave à l'eau, sèche et concentre à sec. Après purification par chromatographie sur silice (gradient d'élution : $CH_2Cl_2$ jusqu'à $CH_2Cl_2$/MeOH ; 98/2), on dissout le composé obtenu dans du $CH_2Cl_2$, ajoute de l'éther chlorhydrique jusqu' à pH 1 puis concentre à sec. On obtient 0,28 g de composé attendu.

Exemple 23 : 1-({5-(2-chlorophenyl)-4-[4-(3-hydroxypropoxy)phényl]thién-2-yl}carbonyl)-4-phénylpipéridine-4-carboxamide.

23A) 1-(2-chlorophényl)-2-(4-méthoxyphényl)éthanone.

Dans 250 ml de THF, on introduit sous azote 230 ml de solution 2M de NaHMDS dans le THF. On refroidit à -60°C puis ajoute à cette température 30,5g d'acide (4-méthoxyphényl)acétique dans 120 ml de THF. Après 1h 30 mn à -60°C, on additionne 29,8 g de 2-chlorobenzoate de méthyle, agite à -60°C pendant 45 mn puis on laisse revenir à 0°C. On verse le milieu réactionnel sur 500 ml d' HCl 2N glacé, extrait à l'éther, lave à l'eau puis avec une solution aqueuse saturée en chlorure de sodium. Après séchage, concentration à sec, on purifie par chromatographie sur silice (gradient : heptane jusqu'à heptane / AcOEt : 80 / 20). On obtient 7, 3 g de composé attendu.

23B) (2*E*)-1-(2-chlorophényl)-3-(diméthylamino)-2-(4-méthoxyphényl)prop-2-én-1-one.

Dans 25 ml de THF, on ajoute 7,3 g de composé obtenu en 23A), 9,5 g de 1,1-diméthoxy-*N,N*-diméthylméthanamine et on porte à reflux pendant 3 heures. Après concentration à sec, on purifie par chromatographie sur silice (gradient : $CH_2Cl_2$ jusqu'à $CH_2Cl_2$ / AcOEt : 80 / 20). On obtient 9,2 g de composé attendu.

23C) (2*E*)-3-chloro-3-(2-chlorophényl)-2-(4-méthoxyphényl)acrylaldéhyde.

Dans 75 ml de $CH_2Cl_2$, on ajoute 9,2 g de composé obtenu à l'étape 23B) puis 6,8g de $POCl_3$. On chauffe à 45°C pendant une nuit puis on concentre à sec. On reprend dans du THF puis ajoute 20 ml d'eau. On extrait à l'éther, lave à l'eau puis avec une solution aqueuse saturée en chlorure de sodium. Après séchage, concentration à sec, on obtient 9,1 g de composé attendu.

23D) 5-(2-chlorophényl)-4-(4-méthoxyphényl)thiophène-2-carboxylate de méthyle.

Dans 90 ml d'acétonitrile, on ajoute 9,1 g de composé obtenu en 23C) puis 6,62 ml de mercaptoacétate de méthyle. On chauffe à 60°C et ajoute 4,83 ml de DBU. On maintient à 60°C pendant 2 heures puis à TA pendant une nuit. On concentre à sec, reprend dans l'acétate d'éthyle, lave à l' HCl 1N puis à l'eau. Après séchage, concentration à sec puis cristallisation dans le méthanol, on obtient 7,8 g de composé attendu.

23E) 5-(2-chlorophényl)-4-(4-hydroxyphényl)thiophène-2-carboxylate de méthyle.

Dans 100 ml de $CH_2Cl_2$, on ajoute 5g de composé obtenu à l'étape 23D) et on refroidit à -50°C, ajoute 41,8 ml de solution 1M de $BBr_3$ dans $CH_2Cl_2$. On laisse revenir la température à 20°C puis on ajoute 20 ml de méthanol. On concentre à sec, reprend dans du $CH_2Cl_2$, lave à l'eau, sèche sur $MgSO_4$, concentre à sec. Après purification par chromatographie sur silice (heptane jusqu'à heptane / AcOEt 75/25), on obtient 4,57 g de composé attendu.

23F) 5-(2-chlorophényl)-4-{4-[3-(tétrahydro-*2H*-pyran-2-yloxy)-propoxy]-phényl}thiophène-2-carboxylate de méthyle.

Dans 15 ml de DMF, on ajoute 2,3 g de composé obtenu à l'étape 23E), 1,95 g de 2-(3-bromopropoxy)tétrahydro-*2H*-pyran, 1,2 g de carbonate de potassium puis on chauffe à 60°C pendant 7 heures. On verse sur eau glacée, extrait à l'éther, sèche et concentre à sec. Après purification par chromatographie sur silice (heptane puis heptane / AcOEt : 80 / 20), on obtient 3,1 g de composé attendu.

23G) Acide 5-(2-chlorophényl)-4-{4-[3-(tétrahydro-*2H*-pyran-2-yloxy)-propoxy]-phényl}thiophène-2-carboxylique.

Dans 30 ml de méthanol et 1 ml d'eau, on ajoute 3,1 g de composé obtenu à l'étape 23F) puis 800 mg de soude en pastille. On porte le mélange réactionnel à reflux pendant 2 heures. On concentre à sec, reprend dans 500 ml de solution tampon à pH 2, extrait à l'éther, sèche et concentre à sec. On obtient 2,8 g de composé attendu, cristallisé dans le mélange éther isopropylique/ pentane.

23H) 1-{[5-(2-chlorophényl)-4-{4-[3-(tétrahydro-*2H*-pyran-2-yloxy)propoxy] phényl}thién-2-yl]carbonyl}-4-phénylpipéridine-4-carboxamide.

Dans 15 ml de $CH_2Cl_2$, on ajoute 0,5 g de composé obtenu à l'étape 23G), 0,26 g de chlorhydrate de 4-phénylpipéridine-4-carboxamide, 0,44 ml de $Et_3N$ et 0,38 g de TBTU puis on laisse la réaction 2 heures à TA. Après concentration à sec, reprise dans un mélange eau / éther, et filtration, on obtient 0,45 g de composé attendu.

23I) 1-({5-(2-chlorophényl)-4-[4-(3-hydroxypropoxy)phényl]thién-2-yl}carbonyl)-4-phénylpipéridine-4-carboxamide.

Dans 10 ml de méthanol, on ajoute 0,45 g de composé obtenu à l'étape 23H), 2 ml d'éther chlorhydrique 2N. Après 2 heures à TA, on filtre le composé attendu. On obtient 0,26 g de composé attendu.Les tableaux 1 et 2 indiquent les structures chimiques de quelques composés selon l'invention ainsi que leurs propriétés physiques (analyse par couplage LC/UV/MS : chromatographie liquide/détection UV/spectrométrie de masse). Ces composés sont exemplifiés ci-avant ou préparés selon des modes opératoires similaires à ceux des composés exemplifiés (exemples 1 à 23).

Dans les tableaux 1 et 2, Me représente un groupe méthyle.

**TABLEAU 1**

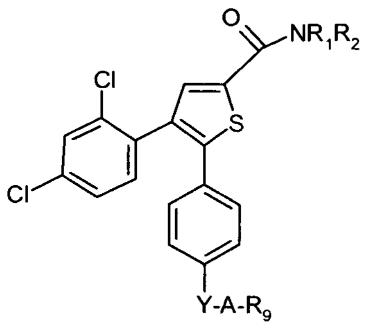

| Composé | $-NR_1R_2$ | -Y-A-R9 | Caractérisation LC/MS (condition) |
|---|---|---|---|
| 1 | 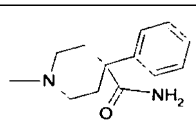 | $-O-(CH_2)_3-OH$ | $MH^+$ = 609 tr = 9,33 (A) |
| 2 | 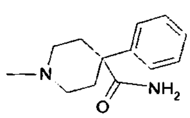 | $-O-(CH_2)_3-NH_2$ | $MH^+$ = 608 tr = 7,08 (A) |
| 3 | 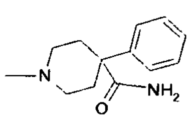 | $-O-(CH_2)_3-NH-SO_2-Me$ | $MH^+$ = 686 tr = 9,41 (A) |

(suite)

| Composé | $-NR_1R_2$ | -Y-A-R9 | Caractérisation LC/MS (condition) |
|---|---|---|---|
| 4 | 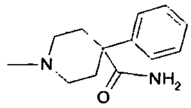 | $-O-(CH_2)_3-S-Me$ | $MH^+$ = 639 tr = 11,29 (A) |
| 5 | 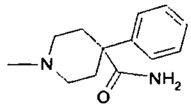 | $-O-(CH_2)_3-SO_2-Me$ | $MH^+$ = 671 tr = 9,39 (A) |
| 6 | 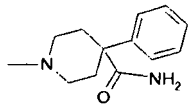 | $-O-(CH_2)_2-OH$ | $MH^+$ = 595 tr = 9,04 (A) |
| 7 | 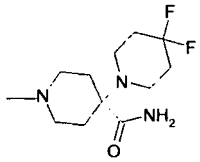 | $-O-(CH_2)_3-OH$ | $MH^+$ = 652 tr = 1,49 (C) |

**TABLEAU 2**

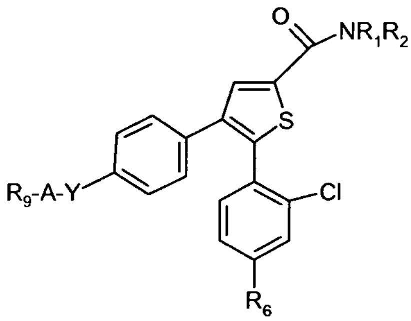

| Composé | $-NR_1R_2$ | $-Y-A-R_9$ | $R_6$ | Caractérisation LC/MS (condition) |
|---|---|---|---|---|
| 8 | 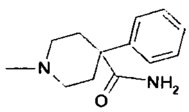 | $-O-(CH_2)_3-OH$ | Cl | $MH^+$ = 609 tr = 9,55 (A) |
| 9 | 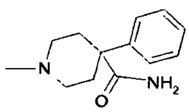 | $-O-(CH_2)_3-NH-SO_2-Me$ | Cl | $MH^+$ = 686 tr = 9,68 (A) |
| 10 | 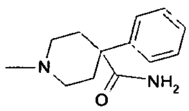 | 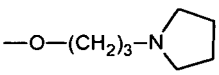 | Cl | $MH^+$ = 662 tr = 7,48 (A) |
| 11 | 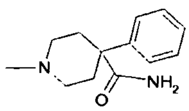 | $-S-(CH_2)_4-OH$ | Cl | $MH^+$ = 639 tr = 1,79 (C) |
| 12 | 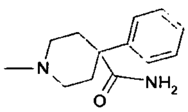 | $-SO-(CH_2)_4-OH$ | Cl | $MH^+$ = 655 tr = 8,15 (B) |

(suite)

| Composé | $-NR_1R_2$ | -Y-A-$R_9$ | $R_6$ | Caractérisation LC/MS (condition) |
|---|---|---|---|---|
| 13 | 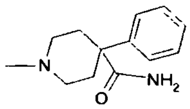 | $-SO_2-(CH_2)_4-OH$ | Cl | $MH^+$ = 671 tr = 1,53 (C) |
| 14 | 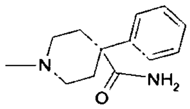 | $-O-SO_2-(CH_2)_2-CH_3$ | H | $MH^+$ = 623 tr = 9,93 (A) |
| 15 | 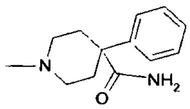 | $-O-SO_2-(CH_2)_2-CF_3$ | H | $MH^+$ = 677 tr = 10,13 (A) |
| 16 | 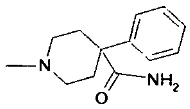 | $-S-(CH_2)_3-CF_3$ | Cl | $MH^+$ = 677 tr = 11,25 (B) |
| 17 | 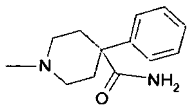 | $-SO_2-(CH_2)_3-CF_3$ | Cl | $MH^+$ = 709 tr = 1,83 (C) |
| 18 | 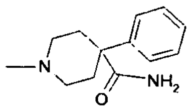 | $-O-(CH_2)_3-OH$ | F | $MH^+$ = 593 tr = 1,55 (C) |
| 19 | 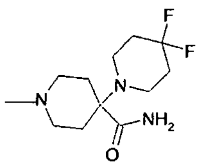 | $-O-(CH_2)_3-OH$ | F | $MH^+$ = 636 tr = 1,42 (C) |
| 20 | 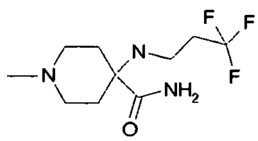 | $-O-(CH_2)_3-OH$ | F | $MH^+$ = 628 tr = 1,25 (C) |
| 21 | 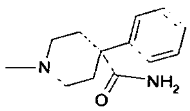 | $-O-(CH_2)_3-S-CH_3$ | F | $MH^+$ = 623 tr = 1,95 (C) |
| 22 | 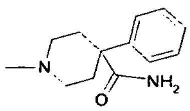 | $-O-(CH_2)_3-SO_2-CH_3$ | F | $MH^+$ = 655 tr = 1,56 (C) |
| 23 | 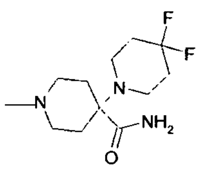 | $-O-(CH_2)_3-S-CH_3$ | F | $MH^+$ = 666 tr = 1,82 (C) |
| 24 | 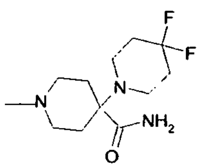 | $-O-(CH_2)_3-SO_2-CH_3$ | F | $MH^+$ = 698 tr = 1,42 (C) |

(suite)

| Composé | $-NR_1R_2$ | -Y-A-$R_9$ | $R_6$ | Caractérisation LC/MS (condition) |
|---|---|---|---|---|
| 25 | 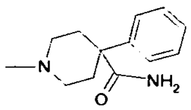 | $-S-(CH_2)_2-NH_2$ | Cl | $MH^+$ = 611 tr = 7,34 (A) |
| 26 | 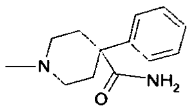 | $-S-(CH_2)_2-NH-SO_2-CH_3$ | Cl | $MH^+$ = 688 tr = 9,79 (A) |
| 27 | 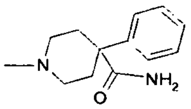 | $-O-(CH_2)_2-OH$ | Cl | $MH^+$ = 595 tr = 9,26 (A) |
| 28 | 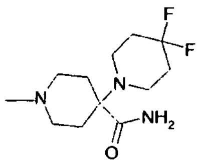 | $-O-(CH_2)_3-OH$ | Cl | $MH^+$ = 652 tr = 1,53 (C) |
| 29 | 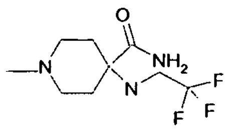 | $-O-(CH_2)_3-OH$ | Cl | $MH^+$ = 630 tr = 1,6 (C) |
| 30 | 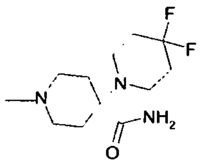 | $-O-(CH_2)_2-OH$ | Cl | $MH^+$ = 638 tr = 1,45 (C) |
| 31 | 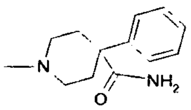 | $-O-SO_2-(CH_2)_2-CH_3$ | Cl | $MH^+$ = 657 tr = 10,62 (A) |
| 32 | 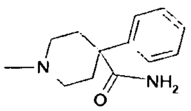 | $-SO_2-(CH_2)_3-OH$ | Cl | $MH^+$ = 657 tr = 1,52 (C) |
| 33 | 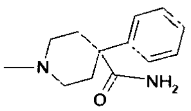 | $-S-(CH_2)_3-OH$ | Cl | $MH^+$ = 625 tr = 1,74 (B) |
| 34 | 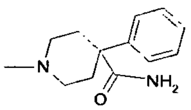 | $-O-(CH_2)_2-CO-NH_2$ | H | $MH^+$ = 588 tr = 8,32 (A) |
| 35 | 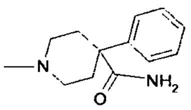 | $-O-(CH_2)_3-S-CH_3$ | Cl | $MH^+$ = 639 tr = 11,43 (A) |
| 36 | 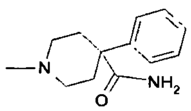 | $-O-(CH_2)_3-SO_2-CH_3$ | Cl | $MH^+$ = 671 tr = 9,64 (A) |

(suite)

| Composé | $-NR_1R_2$ | -Y-A-$R_9$ | $R_6$ | Caractérisation LC/MS (condition) |
|---|---|---|---|---|
| 37 | 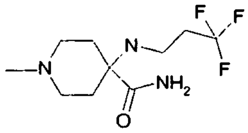 | $-S-(CH_2)_4-OH$ | Cl | $MH^+$ = 674 tr = 9,62 (B) |
| 38 | 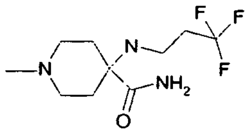 | $-O-(CH_2)_2-OH$ | Cl | $MH^+$ = 629 tr =8,71 (B) |
| 39 | 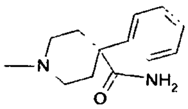 | $-SO_2-(CH_2)_3-CH,$ | Cl | $MH^+$ = 655 tr = 1,84 (C) |
| 40 | 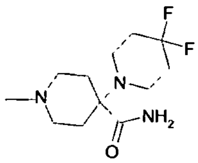 | $-S-(CH_2)_4-OH$ | Cl | $MH^+$ = 682 tr = 1,64 |
| 41 | 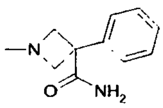 | $-O-(CH_2)_3-OH$ | F | $MH^+$ = 565 tr = 1,52 (C) |
| 42 | 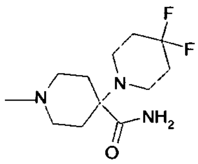 | $-S-(CH_2)_3-OH$ | Cl | $MH^+$ =668 tr = 1,63 (C) |
| 43 | 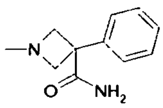 | $-O-(CH_2)_2-OH$ | Cl | $MH^+$ = 567 tr = 1,52 (C) |
| 44 | 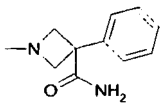 | $-O-(CH_2)_3-OH$ | Cl | $MH^+$ = 581 tr = 1,68 (C) |
| 45 | 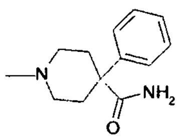 | $-O-(CH_2)_3-OH$ | H | $MH^+$ = 575 tr = 1,49 (C) |
| 46 | 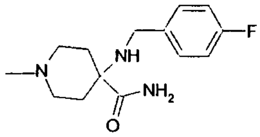 | $-O-(CH_2)_3-OH$ | H | $MH^+$ = 622 tr = 1,20 (C) |
| 47 | 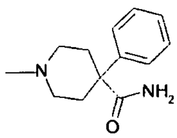 | $-O-(CH_2)_2-OH$ | H | $MH^+$ = 561 tr = 1,43 (C) |

(suite)

| Composé | $-NR_1R_2$ | $-Y-A-R_9$ | $R_6$ | Caractérisation LC/MS (condition) |
|---|---|---|---|---|
| 48 | 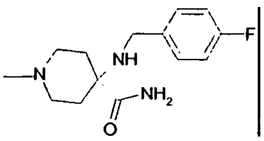 | $-O-(CH_2)_2-OH$ | H | $MH^+$ = 608 tr = 1,15 (C) |

Les analyses effectuées par RMN pour les composés 6, 8, 13, 27, 31 et 45 sont données ci-après :

Composé 6 : RMN $^1H$ : DMSO-$d_6$ (250 MHz) : $\delta$ (ppm) : 1.67- 1.90 : m : 2 H ; 2.39 - 2.58 : m : 2 H ; 3.13 - 3.38 : m : 2 H ; 3.63 : q : 2 H ; 3.91 : t : 2 H ; 4.03 - 4.22 : m : 2 H ; 4.81 : t : 1 H ; 6.86 : d : 2 H ; 7.00 - 7.12 : m : 3 H ; 7.15 - 7.47 : m : 9 H ; 7.68 : d : 1 H.

Composé 8 : RMN $^1H$ : DMSO-$d_6$ (250 MHz) : $\delta$ (ppm) : 1.60 - 2.04 : m : 4 H ; 2.37 - 2.62 : m : 2 H ; 3.15 - 3.41 : m : 2 H ; 3.48 : q : 2 H ; 3.94 : t : 2 H ; 4.02 - 4.21 : m : 2 H ; 4.46 : t : 1 H ; 6.80 : d : 2 H ; 6.97 - 7.13 : m : 3 H ; 7.14 - 7.25 : m : 2 H ; 7.27 - 7.41 : m : 4 H ; 7.44 : s : 2 H ; 7.55 : s : 1 H ; 7.68 : s : 1 H.

Composé 13 : RMN $^1H$ : DMSO-$d_6$ (250 MHz) : $\delta$ (ppm) : 1.31 - 1.66 : m : 4 H ; 1.76 - 1.97: m : 2 H ; 2.48 - 2.63 : m : 2 H ; 3.19 - 3.50 : m : 6 H ; 4.10 - 4.25 : m : 2 H ; 4.42 : t : 1 H ; 7.12 : s : 1 H ; 7.20 - 7.60 : m : 10 H ; 7.75 : d : 2 H ; 7.82 : d : 2 H.

Composé 27 : RMN $^1H$ : DMSO-$d_6$ (250 MHz) : $\delta$ (ppm) : 1.77 - 2.02 : m : 2 H ; 2.46 - 2.62 : m : 2 H ; 3.20 - 3.53 : m : 2 H ; 3.68 : q : 2 H ; 3.95 : t : 2 H ; 4.10 - 4.25 : m : 2 H ; 4.83 : t : 1 H ; 6.85 : d : 2 H ; 7.07 - 7.17 : m : 3 H ; 7.22 - 7.30 : m : 2 H : 7.32 - 7.47 : m : 4 H ; 7.49 : d : 2 H ; 7.61 : s : 1 H ; 7.73 : t : 1 H.

Composé 31 : RMN $^1H$ : DMSO-$d_6$ (400 MHz) : $\delta$ (ppm) : 1.00 : t : 3 H ; 1.71-1.85 : m : 2 H ; 1.84 - 1.95 : m : 2 H ; 2.53 : d : 2 H ; 3.32 - 3.44 : m : 2 H ; 3.47 : t : 2 H ; 4.15 : d : 2 H ; 7.09 : s : 1 H ; 7.20-7.32 : m : 6 H ; 7.34 : t : 2 H ; 7.41 : d : 2 H ; 7.48 : dd : 1 H ; 7.52 : d : 1 H ; 7.67 : s : 1 H ; 7.73 : d : 1 H.

Composé 45 : RMN $^1H$ : DMSO-$d_6$ (250 MHz) : $\delta$ (ppm) : 1,70 - 1,99 : m : 4 H ; 2,45 - 2,63 : m : 2 H ; 3,31 : br : s : 2H ; 3,53 : q : 2 H ; 3,98 : t : 2 H ; 4,17 : dt : 2 H ; 4,51 : t : 1H ; 6,82 : d : 2 H ; 7,06 - 7,18 ; m ; 3 H ; 7,20 - 7,65 ; m 11 H. Les composés de formule (I) possèdent une très bonne affinité *in vitro* ($IC_{50} \leq 5.10^{-7}$M) pour les récepteurs aux cannabinoïdes $CB_1$, dans les conditions expérimentales décrites par M. Rinaldi-Carmona et al. (FEBS Letters, 1994, 350, 240-244).

La nature antagoniste des composés de formule (I) a été démontrée *in vitro* par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

La faible pénétration des composés de formule (I) au niveau de la barrière hémato-encéphalique (BHE) a été évaluée *in vivo* par :

- Mesure (1) : la quantification des composés de formule (1) (inchangé) dans des échantillons de cerveau de souris après une administration par voie intraveineuse (iv, 3 mg/kg) ou orale, à l'aide de technique analytique (LC-MS/MS).

$$\text{Le ratio } \frac{\text{quantité présente dans le cerveau}}{\text{quantité présente dans le plasma}} \text{ inférieur à 0,2 traduit une faible}$$

pénétration du composé au niveau du cerveau.

- Mesure (2) : la mesure de l'interaction des composés de formule (I) avec les récepteurs $CB_1$ présents dans le cerveau chez la souris à l'aide d'un test de binding *ex vivo* du [3H]-CP55940 (agoniste $CB_1$) après une administration par voie intraveineuse (10 mg/kg) comme décrit dans M. Rinaldi-Carmona et al., FEBS Letters, 1994, 350, 240-244

et M. Rinaldi-Carmona et al., Life Sciences, 1995, 56, 1941-1947, M. Rinaldi-Carmona et al., J. Pharmacol. Exp. Ther., 2004, 310, 905-914.

Un pourcentage d'inhibition de la liaison du [3H]-CP55940 au niveau du cerveau inférieur à 50% à 10 mg/kg traduit une faible pénétration au niveau du cerveau. De préférence, ce pourcentage est inférieur à 40% et plus préférentiellement inférieur à 30%.

- Mesure (3) : la mesure du blocage par les composés de formule (I) de l'effet hypothermique induit par un agoniste des récepteurs $CB_1$ (CP55940), après une administration par voie intraveineuse (10 mg/kg), comme décrit dans Rinaldi-Carmona M. et al., JPET 2004, 310, 905-914).

Un pourcentage de réversion de l'effet hypothermique du CP55940 inférieur ou égal à 60 % à 10 mg/kg traduit une faible pénétration au niveau du cerveau. De préférence, ce pourcentage est inférieur à 40% et plus préférentiellement inférieur à 30%.

L'interaction d'un composé de formule (I) selon l'invention avec les récepteurs CB1 présents à la périphérie a été démontrée chez la souris par la mesure du blocage de l'effet inhibiteur induit par le CP55940 sur le transit gastrointestinal (TGI), après une administration orale (po, 10 mg/kg), comme décrit dans M. Rinaldi-Carmona et al., J. Pharmacol. Exp. Ther., 2004, 310, 905-914.

Un pourcentage de réversion de l'effet hypothermique du CP55940 supérieur à 50 % à 10 mg/kg traduit un pouvoir antagoniste significatif au niveau des récepteurs $CB_1$ présent à la périphérie. De préférence, le pourcentage de réversion est compris entre 70% et 100%.

A titre d'exemples, les mesures suivantes ont été faites pour les composés n° 8 et 45 du Tableau 3

Tableau 3

| | Quantification des composés de formule (I) Ratio : quantité présente dans le cerveau / quantité présente dans le plasma [voie iv à 3 mg/kg , selon mesure (1)] | % d'inhibition de la liaison du [3H]-CP55940 au niveau des récepteurs $CB_1$ présents dans le cerveau, [voie iv à 10 mg/kg, selon mesure (2)] | % de réversion de l'effet hypothermique du CP55940 au niveau des récepteurs $CB_1$ présents dans le cerveau [voie iv à 10 mg/kg, selon mesure (3)] | % de reversion de l'effet inhibiteur du CP55940 au niveau des récepteurs $CB_1$ présents à la périphérie (TGI) [voie po à 10 mg/kg] |
|---|---|---|---|---|
| Témoin : rimonabant | 1.8 | 100% | 100% [dose efficace 50 ($DE_{50}$) = 0,3 mg/kg ] | 100% |
| Composé n°8 | 0.04 | 13% | 17.5% | 75.5% |
| Composé n°45 | nd | 32% | 35.5% | 64.5% |

Les composés de formule (I) sont compatibles avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide ou une base pharmaceutiquement acceptable du composé de formule (I).

Ainsi les composés selon l'invention peuvent être utilisés chez l'homme ou chez l'animal (notamment chez les mammifères incluant de façon non limitative les chiens, les chats, les chevaux, les bovins, les moutons) dans le traitement et/ou la prévention de maladies impliquant les récepteurs aux cannabinoïdes $CB_1$.

Par exemple et de manière non limitative, les composés de formule (I) sont utiles comme médicaments psychotropes, notamment pour le traitement et/ou la prévention des désordres psychiatriques incluant l'anxiété, la dépression, les troubles de l'humeur, l'insomnie, les troubles délirants, les troubles obsessionnels, les psychoses en général, la schizophrénie, les troubles du déficit de l'attention et de l'hyperactivité (TDAH) chez les enfants hyperkinétiques ainsi que pour le traitement et/ou la prévention des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement et/ou la prévention de la migraine, du stress, des maladies d'origine psychosomatique, des crises d'attaques de panique,

de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement et/ou la prévention des troubles mnésiques, des troubles cognitifs, en particulier dans le traitement et/ou la prévention des démences séniles, de la maladie d'Alzheimer, ainsi que dans le traitement et/ou la prévention des troubles de l'attention ou de la vigilance.

De plus, les composés de formule (I) peuvent être utiles comme neuroprotecteurs, dans le traitement de l'ischémie, des traumatismes crâniens et le traitement et/ou la prévention des maladies neurodégénératives aiguës ou chroniques : incluant la chorée, la chorée de Huntington, le syndrome de Tourrette.

Les composés de formule (I) selon l'invention peuvent être utilisés comme médicaments dans le traitement et/ou la prévention de la douleur dont notamment : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans le traitement et/ou la prévention des troubles du métabolisme, de l'appétit, de l'appétence (pour les sucres, carbohydrates, drogues, alcools ou toute substance appétissante) et/ou des conduites alimentaires, notamment pour le traitement et/ou la prévention de l'obésité, de la boulimie ainsi que pour le traitement et/ou la prévention du diabète, notamment diabète de type II ou diabète non insulinodépendant, et pour le traitement et/ou la prévention des dyslipidémies, du syndrome métabolique. Ainsi les composés de formule (I) selon l'invention sont utiles dans le traitement et/ou la prévention de l'obésité et des risques associés à l'obésité, notamment les risques cardio-vasculaires.

De plus, les composés de formule (I) selon l'invention peuvent être utilisés en tant que médicaments dans le traitement et/ou la prévention des troubles gastro-intestinaux, des troubles diarrhéiques, des ulcères, des vomissements, des troubles vésicaux et urinaires, des maladies du foie d'origine alcoolique ou non telles que la cirrhose chronique, la fibrose, la stéatose hépatique, la stéatohépatite ; ainsi que des troubles d'origine endocrinienne, des troubles cardio-vasculaires, de l'hypotension, de l'athérosclérose, du choc hémorragique, du choc septique, de l'asthme, de la bronchite chronique, des maladies pulmonaires chroniques obstructives, du syndrome de Raynaud, du glaucome, des troubles de la fertilité, de l'accouchement prématuré, de l'interruption de grossesse, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement et/ou la prévention des maladies des os et de l'ostéoporose.

De plus, les composés de formule (I) selon l'invention peuvent être utilisés pour leurs effets protecteurs contre la cardiotoxicité induite par les médicaments.

Selon la présente invention, les composés de formule (1) sont tout particulièrement utiles pour la préparation de médicaments utiles pour le traitement et/ou la prévention des désordres psychiatriques, en particulier la schizophrénie, les troubles de l'attention et de la vigilance, les troubles du déficit de l'attention et de l'hyperactivité (TDAH) chez les enfants hyperkinétiques; pour le traitement et/ou la prévention des déficits mnésiques et des troubles cognitifs ; de la dépendance et du sevrage à une substance, en particulier la dépendance alcoolique, la dépendance nicotinique, le sevrage alcoolique et le sevrage tabagique ; des maladies neurodégénératives aiguës ou chroniques.

Selon la présente invention, les composés de formule (I) sont également tout particulièrement utiles pour la préparation de médicaments utiles dans le traitement et/ou la prévention des troubles de l'appétit, les troubles de l'appétence, des troubles du métabolisme, l'obésité, le diabète de type II, le syndrome métabolique, la dyslipidémie, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I) et de ses sels pharmaceutiquement acceptables pour le traitement et/ou la prévention des troubles et maladies indiqués ci-dessus.

Selon un autre de ses aspects, la présente invention concerne des compositions pharmaceutiques comprenant, en tant que principe actif, un composé de formule (I) selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Les compositions pharmaceutiques selon la présente invention peuvent contenir à côté d'un composé de formule (I) un (ou plusieurs) autre principe actif utile dans le traitement et/ou la prévention des troubles et maladies indiquées ci-dessus.

Ainsi, la présente invention a également pour objet des compositions pharmaceutiques contenant un composé de formule (I) selon la présente invention associé à un (ou plusieurs) principe actif choisi parmi l'une des classes thérapeutiques suivantes :

- un autre antagoniste ou modulateurs allosteriques des récepteurs $CB_1$ aux cannabinoïdes ;
- un modulateur des récepteurs $CB_2$ aux cannabinoïdes ;
- un antagoniste des récepteurs $AT_1$ de l'angiotensine II ;
- un inhibiteur de l'enzyme de conversion ;
- un antagoniste calcique ;
- un diurétique ;
- un béta-bloquant ;
- un antihyperlipémiant ou un antihypercholestérolémiant ;
- un antidiabétique ;
- un autre agent anti-obésité ou agissant sur les troubles du métabolisme ;
- un agoniste nicotinique, un agoniste nicotinique partiel ;
- un antidépresseur, un antipsychotique, un anxiolytique ;
- un anticancéreux ou un agent antiprolifératif ;
- un antagoniste des opioïdes ;

ainsi que :

- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Selon un autre aspect de l'invention, le composé de formule (I), un de ses sels pharmaceutiquement acceptables et l'autre principe actif associé peuvent être administrés de manière simultanée, séparée ou étalée dans le temps.

On entend par «utilisation simultanée» l'administration des composés de la composition selon l'invention compris dans une seule et même forme pharmaceutique.

On entend par « utilisation séparée » l'administration, en même temps, des deux composés de la composition selon l'invention chacun compris dans une forme pharmaceutique distincte.

On entend par « utilisation étalée dans le temps » l'administration successive, du premier composé de la composition de l'invention, compris dans une forme pharmaceutique, puis du deuxième composé de la composition selon l'invention, compris dans une forme pharmaceutique distincte. Dans ce cas, le laps de temps écoulé entre l'administration du premier composé de la composition selon l'invention et l'administration du deuxième composé de la même composition selon l'invention n'excède généralement pas 24 heures.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel éventuel, peut être administré sous forme unitaire d'administration, en mélange avec des excipients pharmaceutiques classiques, aux animaux et aux êtres humains pour la prophylaxie, le traitement et/ou la prévention des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement et/ou de prévention des pathologies ci-dessus indiquées qui comprend l'administration, à un patient, d'une dose efficace d'un composé selon l'invention, ou un de ses sels pharmaceutiquement acceptables.

**Revendications**

1. Composé de formule (I) :

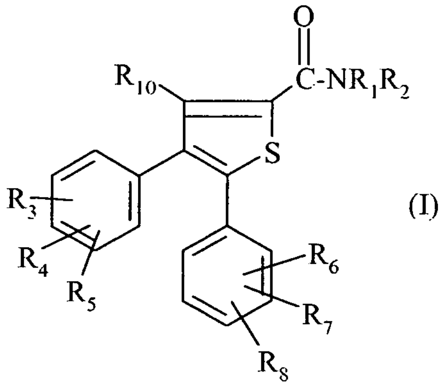

(I)

dans laquelle :

- $R_1$ et $R_2$ ensemble avec l'atome d'azote auquel ils sont liés constituent :

  o soit un radical hétérocyclique saturé de 5 à 7 atomes comprenant deux atomes d'azote, non substitué ou substitué par un groupe phényle, benzyle, benzodioxolyle, benzodioxolylméthyle, tétrahydrofuranylcarbonyle, $-COR_{11}$, et/ou $-CH_2COR_{11}$, le groupe phényle étant lui-même non substitué ou substitué une ou plusieurs fois par un substituant choisi chacun indépendamment parmi un atome d'halogène, un groupe $(C_1-C_4)$alkyle , trifluorométhyle, hydroxyle, $(C_1-C_4)$alcoxy et/ou cyano ;
  o soit un radical hétérocyclique saturé de 4 à 7 atomes comprenant un atome d'azote, non substitué ou substitué une ou deux fois par un substituant choisi chacun indépendamment parmi :

    . un groupe cyano, $-COR_{11}$, $-CH_2NHR_{12}$, $-(C_3-C_7)$cycloalkyle, $-CH_2COR_{11}$, $-NR_{12}R_{13}$, $-NHCOR_{14}$, $-SO_2R_{14}$, et/ou $-SO_2NR_{12}R_{13}$ ;
    . et/ou un groupe phényle, benzyle, pyridinyle ; lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
    . et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou $OCF_3$ ;
    . et/ou un groupe phénylamino, benzylamino lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
    . et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy, $(C_3-C_7)$cycloalkyle et/ou cyano ;
    . et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$ alkyle, $(C_1-C_4)$alcoxy et/ou cyano, ledit groupe $(C_1-C_4)$alkyle étant non substitué ou substitué une ou plusieurs fois par un atome de fluor ;

- $R_3$, $R_4$, $R_5$, $R_6$, $R_7$, $R_8$ représentent chacun indépendamment un atome d'hydrogène, un atome d'halogène,

un groupement -CN, $-S(O)_nR_{14}$, $-OSO_2R_{14}$, un groupe $(C_1-C_6)$alkyle et/ou un groupe $(C_1-C_6)$alcoxy, lesdits groupes étant non substitués ou substitués une ou plusieurs fois par un atome de fluor, à la condition que l'un des deux substituants $R_3$, $R_6$ représente un groupe Y-A-$R_9$ ;
- Y représente un atome d'oxygène, un groupe $-S(O)_{n'}-$, ou $-OSO_2$ ;
- A représente un groupe alkylène en $(C_1-C_4)$ non substitué ou substitué une ou plusieurs fois par un substituant choisi chacun indépendamment parmi un groupe $(C_1-C_3)$alkyle et/ou par un atome de fluor ;
- $R^9$ représente un groupe $-OR_{19}$, -CN, $-CH_3$, $-CF_3$, $-NR_{19}R_{20}$, $-CO_2R_{19}$,
- $CONR_{19}R_{20}$, $-NR_{15}COR_{19}$, $-CONHNH_2$, -CONHOH, $-CONHSO_2R_{21}$
- $S(O)_nR_{21}$, $-SO_2NR_{19}R_{20}$, $-NR_{18}SO_2R_{21}$, $-NR_{15}SO_2NR_{19}R_{20}$ ;
- $R_{10}$ représente un atome d'hydrogène ou un groupe $(C_1-C_4)$alkyle, et de préférence un atome d'hydrogène ;
- $R_{11}$ représente :

  o un groupe $(C_1-C_4)$alkyle, phényle, benzyle, $(C_1-C_4)$alcoxy, ou $(C_1-C_3)$alkylène-O-$(C_1-C_3)$alkyle, lesdits groupes étant non substitués ou substitués par un substituant choisi chacun indépendamment parmi un groupe $(C_1-C_4)$alcoxy et/ou un groupe hydroxy, et/ou par un ou plusieurs atomes de fluor ;
  o et/ou un groupement $-NR_{16}R_{17}$ ;

- $R_{12}$ et $R_{13}$ représentent chacun indépendamment un atome d'hydrogène ou un groupe $(C_1-C_6)$alkyle éventuellement substitué une ou plusieurs fois par un substituant choisi chacun indépendamment parmi un atome de fluor, un groupe
- OH et/ou groupe $-OR_{14}$ ;
- ou $R_{12}$ et $R_{13}$ ensemble avec l'atome d'azote auquel ils sont liés constituent un radical hétérocyclique de 4 à 7 chaînons pouvant comporter un second hétéroatome choisi parmi un atome d'azote, d'oxygène ou de soufre ;
- n représente 0, 1 ou 2 ;
- n' représente 0, 1 ou 2 ;
- $R_{14}$ représente un groupe $(C_1-C_4)$alkyle non substitué ou substitué une ou plusieurs fois par un atome de fluor ;
- $R_{15}$ représente un atome d'hydrogène ou un groupe $(C_1-C_4)$alkyle
- $R_{16}$ et $R_{17}$ représentent chacun indépendamment :

  o un atome d'hydrogène ;
  o et/ou un groupe benzyle non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$ alcoxy et/ou cyano ;
  o et/ou un groupe $(C_1-C_6)$alkyle éventuellement substitué une ou plusieurs fois par un substituant choisi chacun indépendamment parmi un atome d'halogène, un groupe -OH et/ou un groupe $-OR_{14}$ ;

- $R_{18}$ représente un atome d'hydrogène ou un groupe $(C_1-C_4)$alkyle non substitué ou substitué une ou plusieurs fois par un atome de fluor ;
- $R_{19}$ et $R_{20}$ représentent chacun indépendamment un atome d'hydrogène ou un groupe $(C_1-C_6)$alkyle éventuellement substitué une ou plusieurs fois par un substituant choisi chacun indépendamment parmi un atome de fluor, un groupe - OH et/ou un groupe $-OR_{14}$ ;
- ou $R_{19}$ et $R_{20}$ ensemble avec l'atome d'azote auquel ils sont liés constituent un radical hétérocyclique de 4 à 7 chaînons pouvant comporter un second hétéroatome choisi parmi un atome d'azote, d'oxygène ou de soufre ;
- $R_{21}$ représente un groupe $(C_1-C_4)$alkyle non substitué ou substitué une ou plusieurs fois par un atome de fluor ;

ou son sel.

**2.** Composé selon la revendication 1 dans lequel :

- $R_3$ représente un groupe Y-A-$R_9$ ;
- $R_4$, $R_5$, $R_6$, $R_7$ et $R_8$ représentent chacun indépendamment un atome d'hydrogène, un atome d'halogène, un groupement -CN et/ou un groupe $(C_1-C_6)$alkyle, ledit groupe étant non substitué ou substitué une ou plusieurs fois par un atome de fluor.
- et les autres substituants étant tels que définis pour les composés de formule (I) ; ou son sel.

**3.** Composé selon la revendication 1 dans lequel :

- $R_6$ représente un groupe Y-A-$R_9$ ;

- $R_3$, $R_4$, $R_5$, $R_7$ et $R_8$ représentent chacun indépendamment un atome d'hydrogène, un atome d'halogène, un groupement -CN et/ou un groupe ($C_1$-$C_6$)alkyle, ledit groupe étant non substitué ou substitué une ou plusieurs fois par un atome de fluor.
- et les autres substituants étant tels que définis pour les composés de formule (I) ;
ou son sel.

**4.** Composé de formule (I) selon l'une quelconque des revendications précédentes 1 à 3 dans lequel :

- A représente un groupe alkylène en ($C_1$-$C_4$) non substitué,
- et les autres substituants étant tels que définis pour les composés de formule (I) ; ou son sel.

**5.** Composé de formule (I) selon l'une quelconque des revendications précédentes 1 à 4 dans lequel :

- $R_9$ représente un groupe $-OR^{19}$, $-CH_3$, $-CF_3$, $-NR_{19}R_{20}$, $-CONR_{19}R_{20}$,
- $NR_{15}COR_{19}$, $-S(O)_nR_{21}$, ou $-NR_{18}SO_2R_{21}$ ;
- et les autres substituants étant tels que définis pour les composés de formule (I) ; ou son sel.

**6.** Composé de formule (I) selon l'une quelconque des revendications précédentes 1 à 5 dans lequel :

- Y représente un atome d'oxygène ou un atome de soufre;
- et les autres substituants étant tels que définis pour les composés de formule (I) ; ou son sel.

**7.** Composé de formule (I) selon l'une quelconque des revendications précédentes 1 à 6 dans lequel :

- $R_1$ et $R_2$ ensemble avec l'atome d'azote auquel ils sont liés constituent un radical homopipéridin-1-yle, pipéridin-1-yle, pyrrolidin-1-yle ou azétidin-1-yle, lesdit radical étant substitué une ou deux fois par un substituant choisi chacun indépendamment parmi :

o un groupe cyano, $-COR_{11}$, $-NR_{12}R_{13}$, $-NHCOR_{14}$, $-CH_2COR_{11}$, $-SO_2R_{14}$, et/ou $-SO_2NR_{12}R_{13}$ ;
o et/ou un groupe phényle, benzyle, pyridinyle ; lesdits groupes étant non substitués ou substitués une ou plusieurs fois par un substituant choisi chacun indépendamment parmi un atome d'halogène, un groupe ($C_1$-$C_4$)alkyle, trifluorométhyle, hydroxyle, ($C_1$-$C_4$)alcoxy et/ou cyano ;
o et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe ($C_1$-$C_4$)alkyle, ($C_1$-$C_4$)alcoxy, hydroxy, trifluorométhyle et/ou $OCF_3$ ;
o et/ou un groupe phénylamino, benzylamino, lesdits groupes étant non substitués ou substitués une ou plusieurs fois par un substituant choisi chacun indépendamment parmi un atome d'halogène, un groupe ($C_1$-$C_4$)alkyle, trifluorométhyle, hydroxyle, ($C_1$-$C_4$)alcoxy et/ou cyano ;
o et/ou un groupe amino ($C_1$-$C_6$)alkyle non substitué ou substitué une ou plusieurs fois par un substituant choisi chacun indépendamment parmi un atome d'halogène, un groupe hydroxyle, ($C_1$-$C_4$)alcoxy et/ou cyano ;
o et/ou un groupe amino ($C_3$-$C_7$)cycloalkyle non substitué ou substitué une ou plusieurs fois par un substituant choisi chacun indépendamment parmi un atome d'halogène, un groupe hydroxyle, ($C_1$-$C_4$)alkyle, ($C_1$-$C_4$)alcoxy et/ou cyano, ledit groupe ($C_1$-$C_4$)alkyle étant non substitué ou substitué une ou plusieurs fois par un atome de fluor ;

- et les autres substituants étant tels que définis pour les composés de formule (I) ; ou son sel.

**8.** Composé de formule (I) selon la revendication 7 dans lequel :

- $R_1$ et $R_2$ ensemble avec l'atome d'azote auquel ils sont liés constituent un radical homopipéridin-1-yle, pipéridin-1-yle, pyrrolidin-1-yle ou azétidin-1-yle, ledit radical étant gem-disubstitué :

o le premier substituant étant choisi parmi un groupe cyano, $-COR_{11}$, $-NHCOR_{14}$, ou $-SO_2R_{14}$ ;
o le second substituant étant choisi parmi :

. $NR_{12}R_{13}$;
. et/ou un groupe phényle, ledit groupe étant non substitué ou substitué une ou plusieurs fois par un

substituant choisi chacun indépendamment parmi un atome d'halogène, un groupe ($C_1$-$C_4$)alkyle, trifluorométhyle, hydroxyle, ($C_1$-$C_4$)alcoxy et/ou cyano ;
. et/ou un groupe pipéridin-1-yle, ledit groupe étant non substitué ou substitué une ou plusieurs fois par un substituant choisi chacun indépendamment parmi un atome de fluor, un groupe ($C_1$-$C_4$)alkyle, ($C_1$-$C_4$)alcoxy, hydroxy, trifluorométhyle et/ou $OCF_3$ ;
. et/ou un groupe benzylamino, ledit groupe étant non substitué ou substitué une ou plusieurs fois par un substituant choisi chacun indépendamment parmi un atome d'halogène, un groupe ($C_1$-$C_4$)alkyle, trifluorométhyle, hydroxyle, ($C_1$-$C_4$)alcoxy et/ou cyano.

- et les autres substituants étant tels que définis pour les composés de formule (I) ; ou son sel.

**9.** Composé de formule (I) selon la revendication 8 dans lequel :

- $R_1$ et $R_2$ ensemble avec l'atome d'azote auquel ils sont liés constituent un radical pipéridin-1-yle ou azétidin-1-yle , ledit radical étant gem-disubstitué:
- le premier substituant étant -$COR_{11}$ ;
- et le second substituant étant choisi parmi -$NR_{12}R_{13}$, un groupe phényle, un groupe benzylamino ou un groupe pipéridin-1-yle, ledit groupe pipéridin-1-yle étant non substitué ou substitué une ou plusieurs fois par un substituant choisi chacun indépendamment parmi un atome de fluor, un groupe ($C_1$-$C_4$)alkyle, ($C_1$-$C_4$)alcoxy, hydroxy, trifluorométhyle et/ou $OCF_3$ ;
- et les autres substituants étant tels que définis pour les composés de formule (I) ; ou son sel.

**10.** Procédé de préparation d'un composé de formule (I) **caractérisé en ce que** l'on traite l'un des composés de formule (XXX), (XXXI), (XXXII) :

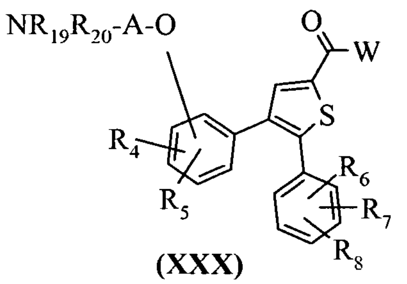
(XXX)

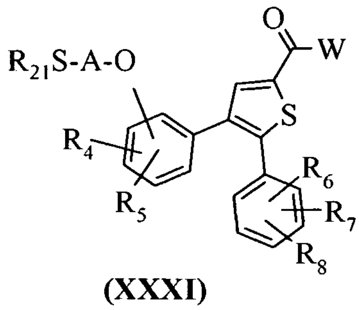
(XXXI)

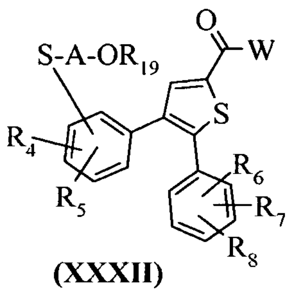
(XXXII)

dans lesquels :

- W représente un groupe ($C_1$-$C_4$)alcoxy, de préférence méthoxy,
- et les autres substituants $R_4$, $R_5$, $R_6$, $R_7$, $R_8$, $R_{19}$, $R_{20}$ et $R_{21}$ sont tels que définis dans l'une quelconque des revendications 1, 2 ou 3 ;

avec un agent de saponification puis avec une amine de formule $HNR_1R_2$ dans laquelle $R_1$ et $R_2$ sont tels que définis dans l'une quelconque des revendications 1 et 7 à 9.

**11.** Procédé de préparation d'un composé de formule (I) **caractérisé en ce que** l'on traite l'un des composés de formule (XXX), (XXXI), (XXXII) :

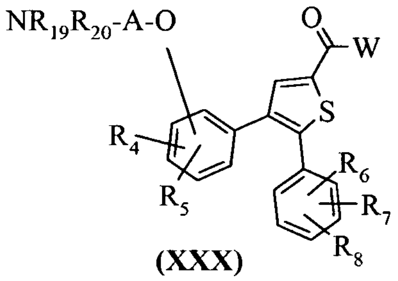

(XXX)

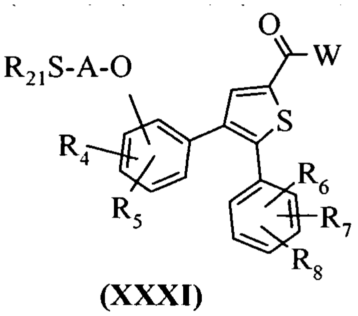

(XXXI)

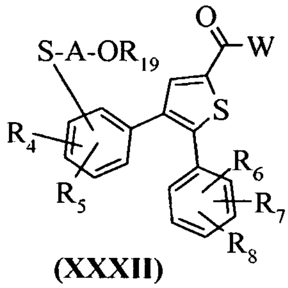

(XXXII)

dans lesquelles :

- W représente un radical hydroxyle ou un chlore,
- et les autres substituants $R_4$, $R_5$, $R_6$, $R_7$, $R_8$, $R_{19}$, $R_{20}$ et $R_{21}$ sont tels que définis dans l'une quelconque des revendications 1, 2 ou 3 ;

avec une amine de formule $HNR_1R_2$ dans laquelle $R_1$ et $R_2$ sont tels que définis dans l'une quelconque des revendications 1 et 7 à 9.

**12.** Composé de formule (XXX), (XXXI) ou (XXXII) :

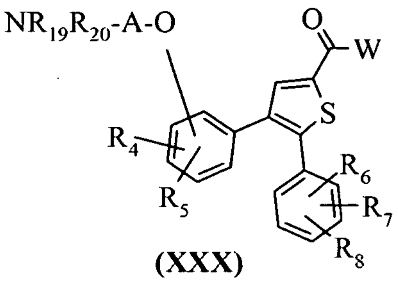

(XXX)

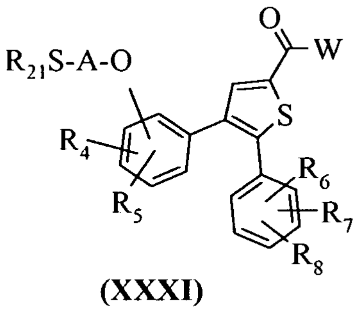

(XXXI)

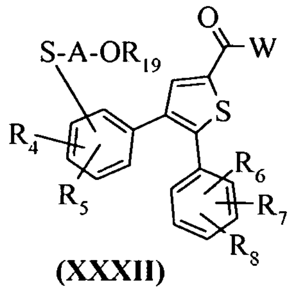

(XXXII)

dans lesquelles :

- W représente un groupe ($C_1$-$C_4$)alcoxy, de préférence méthoxy, un halogène de préférence un atome de chlore ou un radical hydroxyle,
- et les autres substituants sont tels que définis pour les composés de formule (I).

**13.** Médicament, **caractérisé en ce qu'**il comprend un composé de formule (I) selon l'une quelconque des revendications 1 à 9, ou un sel d'addition de ce composé de formule (I) à un acide ou une base pharmaceutiquement acceptable.

**14.** Composition pharmaceutique, **caractérisée en ce qu'**elle comprend un composé de formule (I) selon l'une quelconque des revendications 1 à 9 ou un sel d'addition de ce composé de formule (I) à un acide pharmaceutiquement acceptable ainsi qu'au moins un excipient pharmaceutiquement acceptable.

**15.** Utilisation d'un composé selon l'une quelconque des revendications 1 à 9 pour la préparation d'un médicament destiné au traitement et/ou prévention des désordres psychiatriques, de la dépendance et du sevrage à une substance, des troubles cognitifs, des troubles de l'attention et de la vigilance, des maladies neurodégénératives aigües et chroniques ; du métabolisme, des troubles de l'appétit, des troubles de l'appétence, de l'obésité, de la boulimie, du diabète, du syndrome métabolique, de la dyslipidémie ; de la douleur, des douleurs neuropathiques, des douleurs aiguës périphériques, des douleurs chroniques d'origine inflammatoire, des douleurs induites par un traitement anticancéreux ; des troubles gastro-intestinaux, des vomissements, des troubles diarrhéiques, des ulcères, des maladies du foie ; des phénomènes inflammatoires, des maladies du système immunitaire, de l'arthrite rhumatoïde, des maladies entrainant une démyélinisation, de la sclérose en plaque, des maladies des os et de l'ostéoporose ; des maladies et troubles du mouvement, en particulier des dyskinésies ou de la maladie de Parkinson, des démences

séniles et de la maladie d'Alzheimer.

## Patentansprüche

**1.** Verbindung der Formel (I):

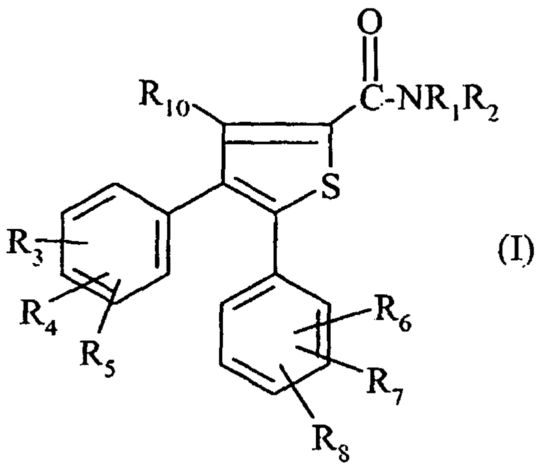

(I)

mit den folgenden Bedeutungen:

- $R_1$ und $R_2$ gemeinsam mit dem Stickstoffatom, an das sie gebunden sind, bilden:

o entweder einen gesättigten heterocyclischen Rest mit 5 bis 7 Atomen, der zwei Stickstoffatome umfasst und der unsubstituiert oder durch eine Phenyl-, Benzyl-, Benzodioxolyl-, Benzodioxolylmethyl-, Tetrahydrofuranylcarbonyl-, $-COR_{11}$-und/oder $-CH_2COR_{11}$-Gruppe substituiert ist, wobei die Phenylgruppe selbst unsubstituiert oder einfach oder mehrfach durch einen Substituenten, der jeweils unabhängig aus der Reihe Halogenatom, $(C_1-C_4)$-Alkylgruppe, Trifluormethyl, Hydroxyl, $(C_1-C_4)$-Alkoxy und/oder Cyano ausgewählt ist, substituiert ist;

o oder einen gesättigten heterocyclischen Rest mit 5 bis 7 Atomen, der ein Stickstoffatom umfasst und der unsubstituiert oder ein oder zweimal durch einen Substituenten substituiert ist, der jeweils unabhängig aus folgenden ausgewählt ist:

. einer Cyano-, $-COR_{11}$-, $-CH_2NHR_{12}$-, $(C_3-C_7)$ Cycloalkyl-, $-CH_2COR_{11}$-, $-NR_{12}R_{13}$-, $-NHCOR_{14}$-, $-SO_2R_{14}$ - und/oder - $SO_2NR_{12}R_{13}$-Gruppe;

. und/oder einer Phenyl-, Benzyl- oder Pyridinylgruppe; wobei diese Gruppen unsubstituiert oder einfach oder mehrfach durch einen Substituenten substituiert sind, der jeweils unabhängig aus der Reihe Halogenatom, $(C_1-C_4)$-Alkylgruppe, Trifluormethyl, Hydroxyl, $(C_1-C_4)$-Alkoxy und/oder Cyano ausgewählt ist;

. und/oder einer Piperidin-1-yl-, Pyrrolidin-1-yl-oder Azetidin-1-yl-Gruppe, wobei diese Gruppen unsubstituiert oder einfach oder mehrfach durch einen Substituenten substituiert sind, der jeweils unabhängig aus der Reihe Fluoratom, $(C_1-C_4)$-Alkyl-, $(C_1-C_4)$-Alkoxy-, Hydroxy-, Trifluormethyl- und/oder $OCF_3$-Gruppe ausgewählt ist;

. und/oder einer Phenylamino- oder Benzylaminogruppe, wobei diese Gruppen unsubstituiert oder einfach oder mehrfach durch einen Substituenten substituiert sind, der jeweils unabhängig aus der Reihe Halogenatom, $(C_1-C_4)$-Alkyl-, Trifluormethyl-, Hydroxyl-, $(C_1-C_4)$-Alkoxy- und/oder Cyanogruppe ausgewählt ist;

. und/oder einer Amino-$(C_1-C_6)$-alkylgruppe, die unsubstituiert oder einfach oder mehrfach durch einen Substituenten substituiert ist, der jeweils unabhängig aus der Reihe Halogenatom, Hydroxyl-, $(C_1-C_4)$-Alkoxy-, $(C_3-C_7)$ -Cycloalkyl- und/oder Cyanogruppe ausgewählt ist;

. und/oder einer Amino-$(C_3-C_7)$-cycloalkylgruppe, die unsubstituiert oder einfach oder mehrfach durch einen Substituenten substituiert ist, der jeweils unabhängig aus der Reihe Halogenatom, Hydroxyl-, $(C_1-C_4)$ -Alkyl-, $(C_1-C_4)$ -Alkoxy- und/oder Cyanogruppe ausgewählt ist, wobei die $(C_1-C_4)$-Alkylgruppe

unsubstituiert oder einfach oder mehrfach durch ein Fluoratom substituiert ist;

- $R_3$, $R_4$, $R_5$, $R_6$, $R_7$, $R_8$ bedeuten unabhängig ein Wasserstoffatom, ein Halogenatom, eine -CN-Gruppierung, $-S(O)_nR_{14}$-Gruppierung, $-OSO_2R_{14}$-Gruppierung, eine $(C_1-C_6)$-Alkylgruppe und/oder eine $(C_1-C_6)$-Alkoxygruppe, wobei diese Gruppen unsubstituiert oder einfach oder mehrfach durch ein Fluoratom substituiert sind, mit der Maßgabe, dass einer der beiden Substituenten $R_3$, $R_6$ eine Y-A-$R_9$-Gruppe bedeutet;
- Y bedeutet ein Sauerstoffatom, eine $-S(O)_{n'}$-Gruppe oder eine $-OSO_2$-Gruppe;
- A bedeutet eine $(C_1-C_4)$ -Alkylengruppe, die unsubstituiert oder einfach oder mehrfach durch einen Substituenten, der jeweils unabhängig aus einer $(C_1-C_3)$-Alkylgruppe ausgewählt ist und/oder durch ein Fluoratom substituiert ist;
- $R_9$ bedeutet eine $-OR_{19}$-, -CN-, $-CH_3$-, $-CF_3$-,
- $NR_{19}R_{20}$-, $-CO_2R_{19}$-, $-CONR_{19}R_{20}$-, $-NR_{15}COR_{19}$-,
- $CONHNH_2$-, -CONHOH-, $-CONHSO_2R_{21}$, $-S(O)_nR_{21}$-,
- $SO_2NR_{19}R_{20}$-, $-NR_{18}SO_2R_{21}$- oder $-NR_{15}SO_2NR_{19}R_{20}$-Gruppe;
- $R_{10}$ bedeutet ein Wasserstoffatom oder eine $(C_1-C_4)$-Alkylgruppe, vorzugsweise ein Wasserstoffatom;
- $R_{11}$ bedeutet:

  o eine $(C_1-C_4)$-Alkyl-, Phenyl-, Benzyl-, $(C_1-C_4)$-Alkoxy- oder $(C_1-C_3)$ -Alkylen-O- $(C_1-C_3)$ -alkylgruppe, wobei diese Gruppen unsubstituiert oder durch einen Substituenten, der jeweils aus einer $(C_1-C_4)$-Alkoxygruppe und/oder einer Hydroxygruppe ausgewählt ist und/oder durch ein oder mehrere Fluoratome ausgewählt ist, substituiert sind;
  o und/oder eine $-NR_{16}R_{17}$-Gruppierung;

- $R_{12}$ und $R_{13}$ bedeuten jeweils unabhängig ein Wasserstoffatom oder eine $(C_1-C_6)$-Alkylgruppe, die gegebenenfalls einfach oder mehrfach durch einen Substituenten, der jeweils unabhängig aus der Reihe Fluoratom, -OH-Gruppe oder $-OR_{14}$-Gruppe ausgewählt ist, substituiert ist;
- oder $R_{12}$ und $R_{13}$ gemeinsam mit dem Stickstoffatom, an das sie gebunden sind, bilden einen heterocyclischen Ring mit 4 bis 7 Ringgliedern, der ein zweites Heteroatom aus der Reihe Stickstoff-, Sauerstoff- oder Schwefelatom umfassen kann;
- n bedeutet 0, 1 oder 2;
- n' bedeutet 0, 1 oder 2;
- $R_{14}$ bedeutet eine $(C_1-C_4)$ -Alkylgruppe, die unsubstituiert oder einfach oder mehrfach durch ein Fluoratom substituiert ist;
- $R_{15}$ bedeutet ein Wasserstoffatom oder eine $(C_1-C_4)$-Alkylgruppe;
- $R_{16}$ und $R_{17}$ bedeuten jeweils unabhängig:

  o ein Wasserstoffatom;
  o und/oder eine Benzylgruppe, die unsubstituiert oder einfach oder mehrfach durch einen Substituenten substituiert ist, der jeweils unabhängig aus der Reihe Halogenatom, $(C_1-C_4)$-Alkyl-, Trifluormethyl-, Hydroxyl-, $(C_1-C_4)$-Alkoxy-und/oder Cyanogruppe ausgewählt ist;
  o und/oder eine $(C_1-C_6)$-Alkylgruppe, die gegebenenfalls einfach oder mehrfach durch einen Substituenten substituiert ist, der jeweils unabhängig aus der Reihe Halogenatom, -OH-Gruppe und/oder $-OR_{14}$-Gruppe ausgewählt ist;

- $R_{18}$ bedeutet ein Wasserstoffatom oder eine $(C_1-C_4)$-Alkylgruppe, die unsubstituiert oder einfach oder mehrfach durch ein Fluoratom substituiert ist;
- $R_{19}$ und $R_{20}$ bedeuten jeweils unabhängig ein Wasserstoffatom oder eine $(C_1-C_6)$-Alkylgruppe, die gegebenenfalls einfach oder mehrfach durch einen Substituenten substituiert ist, der jeweils unabhängig aus der Reihe Fluoratom, -OH-Gruppe und/oder $-OR_{14}$-Gruppe ausgewählt ist;
- oder $R_{19}$ und $R_{20}$ gemeinsam mit dem Stickstoffatom, an das sie gebunden sind, bilden einen heterocyclischen Rest mit 4 bis 7 Ringgliedern, der ein zweites Heteroatom ausgewählt aus einem Stickstoff-, Sauerstoff- oder Schwefelatom, umfassen kann;
- $R_{21}$ bedeutet eine $(C_1-C_4)$ -Alkylgruppe, die unsubstituiert oder einfach oder mehrfach durch ein Fluoratom substituiert ist;

oder Salz davon.

**2.** Verbindung nach Anspruch 1, in der :

- $R_3$ eine Y-A-$R_9$-Gruppe bedeutet;
- $R_4$, $R_5$, $R_6$, $R_7$ und $R_8$ jeweils unabhängig ein Wasserstoffatom, ein Halogenatom, eine -CN-Gruppierung und/oder eine ($C_1$-$C_6$)-Alkylgruppe bedeuten, wobei diese Gruppe unsubstituiert oder einfach oder mehrfach durch ein Fluoratom substituiert sein kann,
- und die restlichen Substituenten wie für die Verbindungen der Formel (I) definiert sind;

oder Salz davon.

**3.** Verbindung nach Anspruch 1, in der

- $R_6$ eine Y-A-$R_9$-Gruppe bedeutet;
- $R_3$, $R_4$, $R_5$, $R_7$ und $R_8$ jeweils unabhängig ein Wasserstoffatom, ein Halogenatom, eine -CN-Gruppierung und/oder eine ($C_1$-$C_6$)-Alkylgruppe bedeuten, wobei diese Gruppe unsubstituiert oder einfach oder mehrfach durch ein Fluoratom substituiert sein kann,
- und die restlichen Substituenten wie für die Verbindungen der Formel (I) definiert sind;

oder Salz davon.

**4.** Verbindung der Formel (I) nach einem der vorhergehenden Ansprüche 1 bis 3, in der:

- A eine unsubstituierte ($C_1$-$C_4$)-Alkylengruppe bedeutet,
- und die restlichen Substituenten wie für die Verbindungen der Formel (I) definiert sind;

oder Salz davon.

**5.** Verbindung der Formel (I) nach einem der vorhergehenden Ansprüche 1 bis 4, in der:

- $R_9$ eine -$OR_{19}$-, -$CH_3$-, -$CF_3$-, -$NR_{19}R_{20}$-, -$CONR_{19}R_{20}$,
- $NR_{15}COR_{19}$-, -$S(O)_nR_{21}$ oder -$NR_{18}SO_2R_{21}$-Gruppe bedeutet;
- und die restlichen Substituenten wie für die Verbindungen der Formel (I) definiert sind;

oder Salz davon.

**6.** Verbindung der Formel (I) nach einem der vorhergehenden Ansprüche 1 bis 5, in der:

- Y ein Sauerstoffatom oder ein Schwefelatom bedeutet;
- und die restlichen Substituenten wie für die Verbindungen der Formel (I) definiert sind;

oder Salz davon.

**7.** Verbindung der Formel (I) nach einem der vorhergehenden Ansprüche 1 bis 6, in der:

- $R_1$ und $R_2$ gemeinsam mit dem Stickstoffatom, an das sie gebunden sind, einen Homopiperidin-1-yl-, Piperidin-1-yl-, Pyrrolidin-1-yl- oder Azetidin-1-ylrest bilden, wobei diese Reste einfach oder zweifach durch einen Substituenten substituiert sind, der jeweils unabhängig aus der folgenden Reihe ausgewählt ist:

  o einer Cyano-, -$COR_{11}$-, -$NR_{12}R_{13}$-, -$NHCOR_{14}$-, -$CH_2COR_{11}$-, -$SO_2R_{14}$- und/oder -$SO_2NR_{12}R_{13}$-Gruppe;
  o und/oder einer Phenyl-, Benzyl- oder Pyridinylgruppe; wobei diese Gruppen unsubstituiert oder einfach oder mehrfach durch einen Substituenten substituiert sind, der jeweils unabhängig aus der Reihe Halogenatom, ($C_1$-$C_4$)-Alkylgruppe, Trifluormethylgruppe, Hydroxylgruppe, ($C_1$-$C_4$)-Alkoxygruppe und/oder Cyanogruppe ausgewählt ist;
  o und/oder einer Piperidin-1-yl-, Pyrrolidin-1-yl-oder Azetidin-1-ylgruppe, wobei diese Gruppen unsubstituiert oder einfach oder mehrfach durch einen Substituenten substituiert sind, der jeweils unabhängig aus der Reihe Fluoratom, ($C_1$-$C_4$)-Alkyl-, ($C_1$-$C_4$)-Alkoxy-, Hydroxyl-, Trifluormethyl-und/oder $OCF_3$-Gruppe ausgewählt ist;
  o und/oder einer Phenylamino- oder Benzylaminogruppe, wobei diese Gruppen unsubstituiert oder einfach oder mehrfach durch einen Substituenten substituiert sind, der jeweils unabhängig aus der Reihe Halogenatom, ($C_1$-$C_4$)-Alkyl-, Trifluormethyl-, Hydroxyl-, ($C_1$-$C_4$)-Alkoxy- und/oder Cyanogruppe ausgewählt ist;
  o und/oder einer Amino-($C_1$-$C_6$)-Alkylgruppe, die unsubstituiert oder einfach oder mehrfach durch einen Substituenten substituiert ist, der jeweils unabhängig aus der Gruppe Wasserstoffatom, Hydroxyl-, ($C_1$-$C_4$)-Alkoxy- und/oder Cyanogruppe ausgewählt ist;
  o und/oder einer Amino-($C_3$-$C_7$)-cycloalkylgruppe, die unsubstituiert oder einfach oder mehrfach durch einen Substituenten substituiert ist, der jeweils unabhängig aus der Reihe Halogenatom, Hydroxyl-,

($C_1$-$C_4$)-Alkyl-, ($C_1$-$C_4$)-Alkoxy- und/oder Cyanogruppe ausgewählt ist, wobei die ($C_1$-$C_4$)-Alkylgruppe unsubstituiert oder einfach oder mehrfach durch ein Fluoratom substituiert ist;

- und die restlichen Substituenten wie für die Verbindungen der Formel (I) definiert sind;
oder Salz davon.

**8.** Verbindung der Formel (I) nach Anspruch 7, in der:

- $R_1$ und $R_2$ gemeinsam mit dem Stickstoffatom, an das sie gebunden sind, einen Homopiperidin-1-yl-, Piperidin-1-yl-, Pyrrolidin-1-yl- oder Azetidin-1-ylrest bilden, wobei der Rest gem-disubstituiert ist:

o wobei der erste Substituent aus der Reihe Cyano-, -$COR_{11}$-, -$NHCOR_{14}$- oder -$SO_2R_{14}$-Gruppe ausgewählt ist;
o und wobei der zweite Substituent aus der folgenden Reihe ausgewählt ist:

. $NR_{12}R_{13}$;
. und/oder einer Phenylgruppe, wobei die Gruppe unsubstituiert oder einfach oder mehrfach durch einen Substituenten substituiert ist, der jeweils unabhängig aus der Reihe Halogenatom, ($C_1$-$C_4$) - Alkyl-, Trifluormethyl-, Hydroxyl-, ($C_1$-$C_4$)-Alkoxy-und/oder Cyanogruppe substituiert ist;
. und/oder einer Piperidin-1-ylgruppe, wobei die Gruppe unsubstituiert oder einfach oder mehrfach durch einen Substituenten substituiert ist, der jeweils unabhängig aus der Reihe Fluoratom, ($C_1$-$C_4$)-Alkyl-, ($C_1$-$C_4$)-Alkoxy-, Hydroxyl-, Trifluormethyl- und/oder $OCF_3$-Gruppe ausgewählt ist;
. und/oder einer Benzylaminogruppe, wobei die Gruppe unsubstituiert oder einfach oder mehrfach durch einen Substituenten substituiert ist, der jeweils unabhängig aus der Reihe Wasserstoffatom, ($C_1$-$C_4$)-Alkyl-, Trifluormethyl-, Hydroxyl-, ($C_1$-$C_4$)-Alkoxy- und/oder Cyanogruppe substituiert ist;
- und die restlichen Substituenten wie für die Verbindungen der Formel (I) definiert sind;
oder Salz davon.

**9.** Verbindung der Formel (I) nach Anspruch 8, in der:

- $R_1$ und $R_2$ gemeinsam mit dem Stickstoffatom, an das sie gebunden sind, einen Piperidin-1-yl- oder Azetidin-1-ylrest bilden, wobei dieser Rest gem-disubstituiert ist:
- wobei der erste Substituent -$COR_{11}$ ist;
- und wobei der zweite Substituent aus der Reihe
- $NR_{12}R_{13}$, einer Phenylgruppe, einer Benzylaminogruppe oder einer Piperidin-1-ylgruppe ausgewählt ist, wobei die Piperidin-1-ylgruppe unsubstituiert oder einfach oder mehrfach durch einen Substituenten substituiert ist, der jeweils unabhängig aus der Reihe Fluoratom, ($C_1$-$C_4$)-Alkyl-, ($C_1$-$C_4$)-Alkoxy-, Hydroxyl-, Trifluormethyl und/oder $OCF_3$-Gruppe ausgewählt ist;
- und die restlichen Substituenten wie für die Verbindungen der Formel (I) definiert sind;
oder Salz davon.

**10.** Verfahren zur Herstellung einer Verbindung der Formel (I), **dadurch gekennzeichnet, dass** man eine der Verbindungen der Formel (XXX), (XXXI), (XXXII):

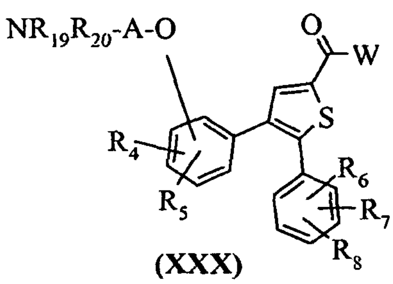
(XXX)

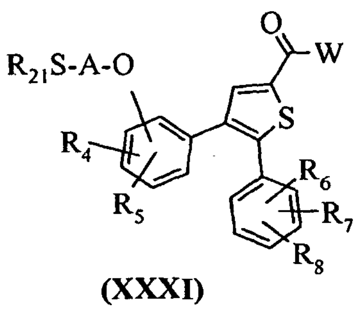
(XXXI)

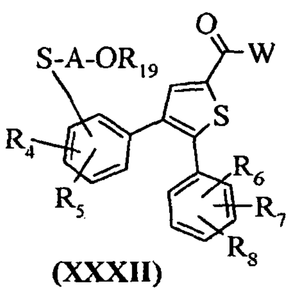
(XXXII)

in denen:

- W eine ($C_1$-$C_4$)-Alkoxygruppe, vorzugweise eine Methoxygruppe bedeutet,
- und die übrigen Substituenten $R_4$, $R_5$, $R_6$, $R_7$, $R_8$, $R_{19}$, $R_{20}$ und $R_{21}$ wie in einem der Ansprüche 1, 2 oder 3 definiert sind,

mit einem Verseifungsmittel und anschließend mit einem Amin der Formel $HNR_1R_2$, in der $R_1$ und $R_2$ wie in einem der Ansprüche 1 und 7 bis 9 definiert sind, behandelt.

**11.** Verfahren zur Herstellung einer Verbindung der Formel (I), **dadurch gekennzeichnet, dass** man eine der Verbindungen der Formel (XXX), (XXXI), (XXXII):

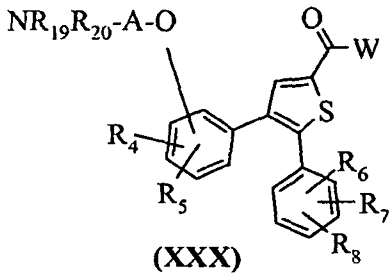
(XXX)

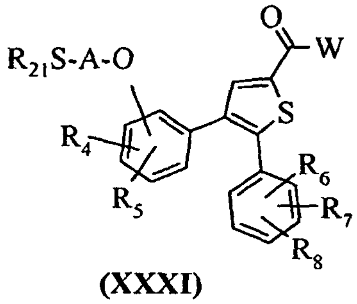
(XXXI)

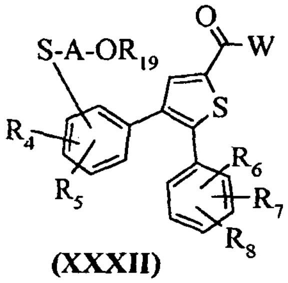
(XXXII)

in denen:

- W einen Hydroxylrest oder ein Chlor bedeutet,
- und die übrigen Substituenten $R_4$, $R_5$, $R_6$, $R_7$, $R_8$, $R_{19}$, $R_{20}$ und $R_{21}$ wie in einem der Ansprüche 1, 2 oder 3 definiert sind,

mit einem Amin der Formel $HNR_1R_2$, in der $R_1$ und $R_2$ wie in einem der Ansprüche 1 und 7 bis 9 definiert sind, behandelt.

**12.** Verbindung der Formel (XXX), (XXXI) oder (XXXII):

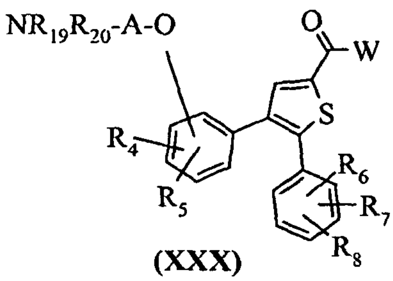
(XXX)

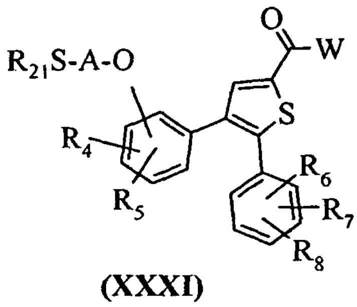
(XXXI)

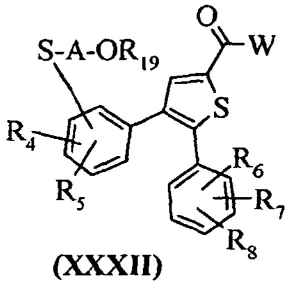
(XXXII)

in denen:

- W eine ($C_1$-$C_4$)-Alkoxygruppe, vorzugweise eine Methoxygruppe, ein Halogen, vorzugweise ein Chloratom, oder einen Hydroxylrest bedeutet,
- und die übrigen Substituenten wie für die Verbindungen der Formel (I) definiert sind.

**13.** Arzneimittel, **dadurch gekennzeichnet, dass** es eine Verbindung der Formel (I) nach einem der Ansprüche 1 bis 9 oder ein Additionssalz dieser Verbindung der Formel (I) mit einer pharmazeutisch unbedenklichen Säure oder Base umfasst.

**14.** Pharmazeutische Zusammensetzung, **dadurch gekennzeichnet, dass** sie eine Verbindung der Formel (I) nach einem der Ansprüche 1 bis 9 oder ein Additionssalz dieser Verbindung der Formel (I) mit einer pharmazeutisch unbedenklichen Säure sowie mindestens einen pharmazeutisch unbedenklichen Grundstoff umfasst.

**15.** Verwendung einer Verbindung nach einem der Ansprüche 1 bis 9 zur Herstellung eines Arzneimittels zur Behandlung und/oder Vorbeugung von psychiatrischen Erkrankungen, Substanzabhängigkeit und -entwöhnung, kognitiven Störungen, Aufmerksamkeitsstörungen und Wachsamkeitsstörungen, akuten und chronischen neurodegenerativen Erkrankungen, Erkrankungen des Metabolismus, Appetitstörungen, Appetenzstörungen, Fettleibigkeit, Bulimie, Diabetes, metabolischem Syndrom, Dyslipidämie, Schmerzen, neuropathischen Schmerzen, akuten peripheren Schmerzen, chronischen Schmerzen entzündlichen Ursprungs, durch Antikrebsbehandlung induzierten Schmerzen, Magen-Darm-Problemen, Erbrechen, Durchfallstörungen, Geschwüren, Lebererkrankungen; entzündlichen Erscheinungen, Krankheiten des Immunsystems, rheumatoider Arthritis, Krankheiten, die zu Entmyelinisierung führen, Multipler Sklerose, Knochenkrankheiten und Osteoporose; Bewegungserkrankungen und -störungen, insbesondere Dyskinesien oder Morbus Parkinson, seniler Demenz und Morbus Alzheimer.

## Claims

**1.** Compound of formula (I):

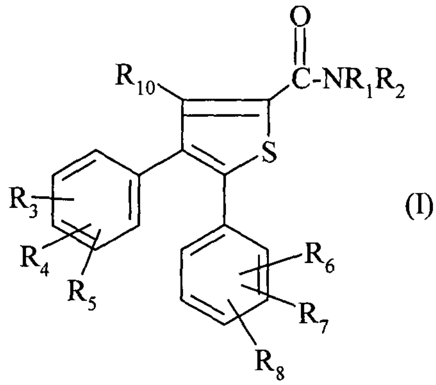

(I)

in which:

- $R_1$ and $R_2$, together with the nitrogen atom to which they are bonded, form:

  o either a saturated heterocyclic radical of 5 to 7 atoms comprising two nitrogen atoms which is unsubstituted or substituted by a phenyl, benzyl, benzodioxolyl, benzodioxolylmethyl, tetrahydrofuranylcarbonyl, $-COR_{11}$ and/or $-CH_2COR_{11}$ group, the phenyl group being itself unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;

  o or a saturated heterocyclic radical of 4 to 7 atoms comprising a nitrogen atom which is unsubstituted or substituted once or twice by a substituent each independently chosen from:

  . a cyano, $-COR_{11}$, $-CH_2NHR_{12}$, $-(C_3-C_7)$cycloalkyl, $-CH_2COR_{11}$, $-NR_{12}R_{13}$, $-NHCOR_{14}$, $-SO_2R_{14}$ and/or $-SO_2NR_{12}R_{13}$ group;

  . and/or a phenyl, benzyl or pyridinyl group; the said groups being unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;

  . and/or a piperidin-1-yl, pyrrolidin-1-yl or azetidin-1-yl group, the said groups being unsubstituted or substituted one or more times by a substituent each independently chosen from a fluorine atom or a $(C_1-C_4)$alkyl, $(C_1-C_4)$alkoxy, hydroxyl, trifluoromethyl and/or $OCF_3$ group;

  . and/or a phenylamino or benzylamino group, the said groups being unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;

  . and/or an amino$(C_1-C_6)$alkyl group which is unsubstituted or substituted one or more times by a substituent each independently chosen from a halogen atom or a hydroxyl, $(C_1-C_4)$alkoxy, $(C_3-C_7)$cycloalkyl and/or cyano group;

  . and/or an amino$(C_3-C_7)$cycloalkyl group which is unsubstituted or substituted one or more times by a substituent each independently chosen from a halogen atom or a hydroxyl, $(C_1-C_4)$alkyl, $(C_1-C_4)$

alkoxy and/or cyano group, the said $(C_1-C_4)$alkyl group being unsubstituted or substituted one or more times by a fluorine atom;

- $R_3$, $R_4$, $R_5$, $R_6$, $R_7$ and $R_8$ each independently represent a hydrogen atom, a halogen atom, a -CN, $-S(O)_nR_{14}$, $-OSO_2R_{14}$ or $(C_1-C_6)$alkyl group and/or a $(C_1-C_6)$alkoxy group, the said groups being unsubstituted or substituted one or more times by a fluorine atom, provided that one of the two substituents $R_3$ and $R_6$ represents a Y-A-$R_9$ group;
- Y represents an oxygen atom or an $-S(O)_{n'}$- or $-OSO_2$- group;
- A represents a $(C_1-C_4)$alkylene group which is unsubstituted or substituted one or more times by a substituent each independently chosen from a $(C_1-C_3)$alkyl group and/or by a fluorine atom;
- $R_9$ represents an $-OR^{19}$, -CN, $-CH_3$, $-CF_3$, $-NR_{19}R_{20}$, $-CO_2R_{19}$,
- $CONR_{19}R_{20}$, $-NR_{15}COR_{19}$, $-CONHNH_2$, -CONHOH, $-CONHSO_2R_{21}$,
- $S(O)_nR_{21}$, $-SO_2NR_{19}R_{20}$, $-NR_{18}SO_2R_{21}$ or $-NR_{15}SO_2NR_{19}R_{20}$ group;
- $R_{10}$ represents a hydrogen atom or a $(C_1-C_4)$alkyl group and preferably a hydrogen atom;
- $R_{11}$ represents:

  o a $(C_1-C_4)$alkyl, phenyl, benzyl, $(C_1-C_4)$alkoxy or $(C_1-C_3)$alkylene-O-$(C_1-C_3)$alkyl group, the said groups being unsubstituted or substituted by a substituent each independently chosen from a $(C_1-C_4)$alkoxy group and/or a hydroxyl group and/or by one or more fluorine atoms;
  o and/or an $-NR_{16}R_{17}$ group;

- $R_{12}$ and $R_{13}$ each independently represent a hydrogen atom or a $(C_1-C_6)$alkyl group optionally substituted one or more times by a substituent each independently chosen from a fluorine atom, an -OH group and/or an
- $OR_{14}$ group;
- or $R_{12}$ and $R_{13}$, together with the nitrogen atom to which they are bonded, form a 4- to 7-membered heterocyclic radical which can comprise a second heteroatom chosen from a nitrogen, oxygen or sulphur atom;
- n represents 0, 1 or 2;
- n' represents 0, 1 or 2;
- $R_{14}$ represents a $(C_1-C_4)$alkyl group which is unsubstituted or substituted one or more times by a fluorine atom;
- $R_{15}$ represents a hydrogen atom or a $(C_1-C_4)$alkyl group;
- $R_{16}$ and $R_{17}$ each independently represent:

  o a hydrogen atom;
  o and/or a benzyl group which is unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;
  o and/or a $(C_1-C_6)$alkyl group optionally substituted one or more times by a substituent each independently chosen from a halogen atom or an -OH and/or an $-OR_{14}$ group;

- $R_{18}$ represents a hydrogen atom or a $(C_1-C_4)$alkyl group which is unsubstituted or substituted one or more times by a fluorine atom;
- $R_{19}$ and $R_{20}$ each independently represent a hydrogen atom or a $(C_1-C_6)$alkyl group optionally substituted one or more times by a substituent each independently chosen from a fluorine atom, an -OH group and/or an
- $OR_{14}$ group;
- or $R_{19}$ and $R_{20}$, together with the nitrogen atom to which they are bonded, form a 4- to 7-membered heterocyclic radical which can comprise a second heteroatom chosen from a nitrogen, oxygen or sulphur atom;
- $R_{21}$ represents a $(C_1-C_4)$alkyl group which is unsubstituted or substituted one or more times by a fluorine atom;

or its salt.

**2.** Compound according to Claim 1, in which:

- $R_3$ represents a Y-A-$R_9$ group;
- $R_4$, $R_5$, $R_6$, $R_7$ and $R_8$ each independently represent a hydrogen atom, a halogen atom, a -CN group and/or a $(C_1-C_6)$alkyl group, the said group being unsubstituted or substituted one or more times by a fluorine atom;
- and the other substituents are as defined for the compounds of formula (I); or its salt.

**3.** Compound according to Claim 1, in which:

- $R_6$ represents a Y-A-$R_9$ group;
- $R_3$, $R_4$, $R_5$, $R_7$ and $R_8$ each independently represent a hydrogen atom, a halogen atom, a -CN group and/or a $(C_1-C_6)$alkyl group, the said group being unsubstituted or substituted one or more times by a fluorine atom;
- and the other substituents are as defined for the compounds of formula (I); or its salt.

**4.** Compond of formula (I) according to any one of the preceding Claims 1 to 3, in which:

- A represents an unsubstituted $(C_1-C_4)$alkylene group;
- and the other substituents are as defined for the compounds of formula (I); or its salt.

**5.** Compound of formula (I) according to any one of the preceding Claims 1 to 4, in which:

- $R_9$ represents an $-OR^{19}$, $-CH_3$, $-CF_3$, $-NR_{19}R_{20}$, $-CONR_{19}R_{20}$,
- $NR_{15}COR_{19}$, $-S(O)_nR_{21}$ or $-NR_{18}SO_2R_{21}$ group;
- and the other substituents are as defined for the compounds of formula (I); or its salt.

**6.** Compound of formula (I) according to any one of the preceding Claims 1 to 5, in which:

- Y represents an oxygen atom or a sulphur atom;
- and the other substituents are as defined for the compounds of formula (I); or its salt.

**7.** Compound of formula (I) according to any one of the preceding Claims 1 to 6, in which:

- $R_1$ and $R_2$, together with the nitrogen atom to which they are bonded, form a homopiperidin-1-yl, piperidin-1-yl, pyrrolidin-1-yl or azetidin-1-yl radical, the said radical being substituted once or twice by a substituent each independently chosen from:

  o a cyano, $-COR_{11}$, $-NR_{12}R_{13}$, $-NHCOR_{14}$, $-CH_2COR_{11}$, $-SO_2R_{14}$ and/or $-SO_2NR_{12}R_{13}$ group;
  o and/or a phenyl, benzyl or pyridinyl group; the said groups being unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;
  o and/or a piperidin-1-yl, pyrrolidin-1-yl or azetidin-1-yl group, the said groups being unsubstituted or substituted one or more times by a substituent each independently chosen from a fluorine atom or a $(C_1-C_4)$ alkyl, $(C_1-C_4)$alkoxy, hydroxyl, trifluoromethyl and/or $OCF_3$ group;
  o and/or a phenylamino or benzylamino group, the said groups being unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;
  o and/or an amino$(C_1-C_6)$alkyl group which is unsubstituted or substituted one or more times by a substituent each independently chosen from a halogen atom or a hydroxyl, $(C_1-C_4)$alkoxy and/or cyano group;
  o and/or an amino$(C_3-C_7)$cycloalkyl group which is unsubstituted or substituted one or more times by a substituent each independently chosen from a halogen atom or a hydroxyl, $(C_1-C_4)$alkyl, $(C_1-C_4)$alkoxy and/or cyano group, the said $(C_1-C_4)$alkyl group being unsubstituted or substituted one or more times by a fluorine atom;

- and the other substituents are as defined for the compounds of formula (I); or its salt.

**8.** Compound of formula (I) according to Claim 7, in which:

$R_1$ and $R_2$, together with the nitrogen atom to which they are bonded, form a homopiperidin-1-yl, piperidin-1-yl, pyrrolidin-1-yl or azetidin-1-yl radical, the said radical being gem-disubstituted:

  o the first substituent being chosen from a cyano, $-COR_{11}$, $-NHCOR_{14}$ or $-SO_2R_{14}$ group;
  o the second substituent being chosen from:

    . $NR_{12}R_{13}$;
    . and/or a phenyl group, the said group being unsubstituted or substituted one or more times by a substituent each independently chosen from a halogen atom or a $(C_1-C_4)$alkyl, trifluoromethyl, hydroxyl, $(C_1-C_4)$alkoxy and/or cyano group;

. and/or a piperidin-1-yl group, the said group being unsubstituted or substituted one or more times by a substituent each independently chosen from a fluorine atom or a $(C_1\text{-}C_4)$alkyl, $(C_1\text{-}C_4)$alkoxy, hydroxyl, trifluoromethyl and/or $OCF_3$ group;

. and/or a benzylamino group, the said group being unsubstituted or substituted one or more times by a substituent each independently chosen from a halogen atom or a $(C_1\text{-}C_4)$alkyl, trifluoromethyl, hydroxyl, $(C_1\text{-}C_4)$alkoxy and/or cyano group;

- and the other substituents are as defined for the compounds of formula (I); or its salt.

**9.** Compound of formula (I) according to Claim 8, in which:

- $R_1$ and $R_2$, together with the nitrogen atom to which they are bonded, form a piperidin-1-yl or azetidin-1-yl radical, the said radical being gem-disubstituted:
- the first substituent being $-COR_{11}$;
- and the second substituent being chosen from $-NR_{12}R_{13}$, a phenyl group, a benzylamino group or a piperidin-1-yl group, the said piperidin-1-yl group being unsubstituted or substituted one or more times by a substituent each independently chosen from a fluorine atom or a $(C_1\text{-}C_4)$alkyl, $(C_1\text{-}C_4)$alkoxy, hydroxyl, trifluoromethyl and/or $OCF_3$ group;
- and the other substituents are as defined for the compounds of formula (I); or its salt.

**10.** Process for the preparation of a compound of formula (I), **characterized in that** one of the compounds of formula (XXX), (XXXI) or (XXXII):

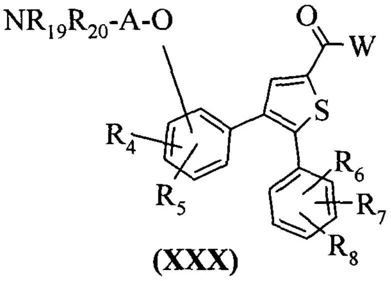
(XXX)
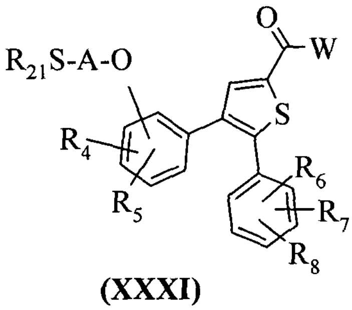
(XXXI)
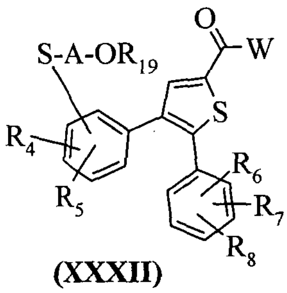
(XXXII)

in which:

- W represents a $(C_1\text{-}C_4)$alkoxy group, preferably a methoxy group,
- and the other substituents $R_4$, $R_5$, $R_6$, $R_7$, $R_8$, $R_{19}$, $R_{20}$ and $R_{21}$ are as defined in any one of Claims 1, 2 or 3,

is treated with a saponification agent and then with $HNR_1R_2$ in which $R_1$ and $R_2$ are as defined in any one of Claims 1 and 7 to 9.

**11.** Process for the preparation of a compound of formula (I), **characterized in that** one of the compounds of formula (XXX), (XXXI) or (XXXII):

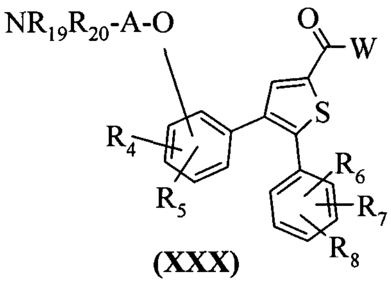
(XXX)
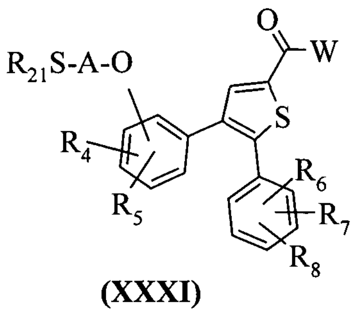
(XXXI)
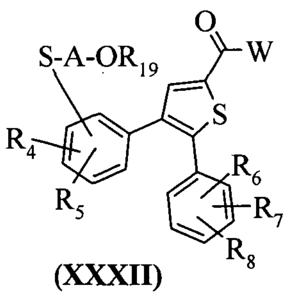
(XXXII)

in which:

- W represents a hydroxyl radical or a chlorine,
- and the other substituents $R_4$, $R_5$, $R_6$, $R_7$, $R_8$, $R_{19}$, $R_{20}$ and $R_{21}$ are as defined in any one of Claims 1, 2 or 3,

is treated with an amine of formula $HNR_1R_2$ in which $R_1$ and $R_2$ are as defined in any one of Claims 1 and 7 to 9.

**12.** Compound of formula (XXX), (XXXI) or (XXXII):

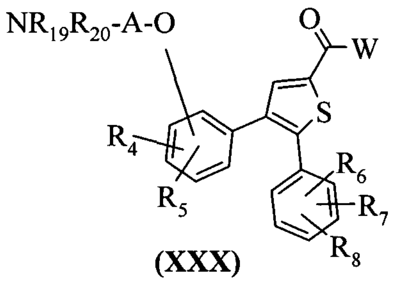
(XXX)

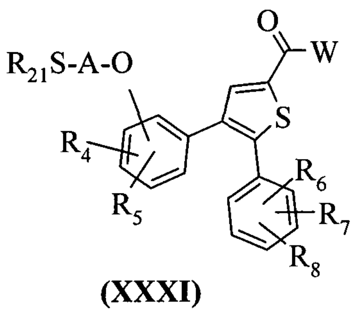
(XXXI)

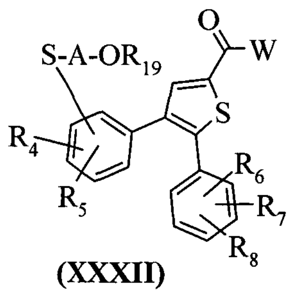
(XXXII)

in which:

- W represents a $(C_1-C_4)$alkoxy group, preferably a methoxy group, a halogen, preferably a chlorine atom, or a hydroxyl radical,
- and the other substituents are as defined for the compounds of formula (I).

**13.** Medicament, **characterized in that** it comprises a compound of formula (I) according to any one of Claims 1 to 9 or an addition salt of this compound of formula (I) with a pharmaceutically acceptable acid or base.

**14.** Pharmaceutical composition, **characterized in that** it comprises a compound of formula (I) according to any one of Claims 1 to 9 or an addition salt of this compound of formula (I) with a pharmaceutically acceptable acid and at least one pharmaceutically acceptable excipient.

**15.** Use of a compound according to any one of Claims 1 to 9 in the preparation of a medicament intended for the treatment and/or prevention of psychiatric disorders, dependence on and withdrawal from a substance, cognitive disorders, disorders of attention and of vigilance, or acute and chronic neurodegenerative diseases; metabolic disorders, disorders of appetite, disorders of appetency, obesity, bulimia, diabetes, metabolic syndrome or dyslipidaemia; pain, neuropathic pain, acute peripheral pain, chronic pain of inflammatory origin or pain brought about by an anticancer treatment; gastrointestinal disorders, vomiting, diarrhoea, ulcers or liver diseases; inflammatory phenomena, diseases of the immune system, rheumatoid arthritis, diseases which bring about demyelination, multiple sclerosis, bone diseases and osteoporosis; or movement diseases and disorders, in particular dyskinesias or Parkinson's disease, senile dementia and Alzheimer's disease.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5624941 A
- EP 0576357 A
- EP 0656354 A
- EP 1150961 A
- WO 03051850 A
- WO 03027076 A
- WO 9119708 A
- WO 200503548 A
- FR 2860792
- WO 2005035488 A
- WO 2008068423 A


### Littérature non-brevet citée dans la description


- **GREEN et al.** Protective Group in Organic Synthesis. John Wiley & Sons, Inc, 2002
- *Chem. Comm.,* 1988, 475-477
- *J. Med. Chem.,* 1964, vol. 7, 619-622
- **M. RINALDI-CARMONA et al.** *FEBS Letters,* 1994, vol. 350, 240-244
- **M. BOUABOULA et al.** *J. Biol. Chem.,* 1995, vol. 270, 13973-13980
- **M. RINALDI-CARMONA et al.** *J. Pharmacol. Exp. Ther.,* 1996, vol. 278, 871-878
- **M. BOUABOULA et al.** *J. Biol. Chem.,* 1997, vol. 272, 22330-22339
- **M. RINALDI-CARMONA et al.** *Life Sciences,* 1995, vol. 56, 1941-1947
- **M. RINALDI-CARMONA et al.** *J. Pharmacol. Exp. Ther.,* 2004, vol. 310, 905-914
- **RINALDI-CARMONA M. et al.** *JPET,* 2004, vol. 310, 905-914